# EUROPEAN PATENT APPLICATION

(11) **EP 4 361 175 A1**
(43) Date of publication of application: **01.05.2024**
(21) Application number: 22828434.5
(22) Date of filing: 21.06.2022
(51) Int. Cl.: C07K 16/28, C07K 16/46, C12N 15/13

(54) **RECEPTOR TYROSINE KINASE AGONIST, COMPOSITION FOR CELL CULTURE MEDIUM, AND UNDIFFERENTIATED MAINTENANCE COMPOSITION**

(30) Priority: 21.06.2021 JP 2021102762
(71) Applicant: SEKISUI CHEMICAL CO., LTD., Osaka-shi Osaka 530-8565 (JP); Epsilon Molecular Engineering Inc., Saitama City, Saitama 338-8570 (JP)
(72) Inventor: KASHIWAGI Kenji, Tsukuba-shi, Ibaraki 300-4292 (JP); YANAGISAWA Teruhiko, Tsukuba-shi, Ibaraki 300-4292 (JP); YONEHARA Ryo, Saitama City, Saitama 338-8570 (JP); KUMACHI Shigefumi, Saitama City, Saitama 338-8570 (JP); NAKAO Kanako, Saitama City, Saitama 338-8570 (JP); TSUCHIYA Masayuki, Saitama City, Saitama 338-8570 (JP)
(74) Representative: Ter Meer Steinmeister & Partner
(86) International application number: PCT/JP2022/024784
(87) International publication number: WO 2022/270518

(57) **Abstract**

A receptor tyrosine kinase agonist which is a single-domain antibody having a cell growth activity, and is at least one receptor tyrosine kinase agonist selected from the group consisting of a human fibroblast growth factor receptor 1 (FGFR1), a human fibroblast growth factor receptor 2 (FGFR2), a human fibroblast growth factor receptor 3 (FGFR3), and a human fibroblast growth factor receptor 4 (FGFR4).

## Description

### [Technical Field]

The present invention relates to a receptor tyrosine kinase agonist, a medium composition for cell culture, and a composition for maintaining an undifferentiated state.

Priority is claimed on Japanese Patent Application No. 2021-102762, filed Jun 21, 2021, the content of which is incorporated herein by reference.

### [Background Art]

Many extracellular signaling proteins utilize receptor tyrosine kinases (hereinafter also referred to as "RTKs"). It has been reported that there are about 60 types of human RTKs, that the RTKs have structures called an extracellular domain, a transmembrane domain, and an intracellular domain, and that the RTKs are classified into about 20 types of sub-families depending on the structures (Non Patent Document 1). As a signaling protein family that acts via the RTK, an epidermal growth factor (EGF) that acts in responses such as promotion of survival, growth, proliferation, and differentiation of various cells; an insulin-like growth factor (IGF1) that acts in the survival or growth of various cells; a fibroblast growth factor (hereinafter also simply referred to as "FGF") that acts on, for example, growth acceleration of various cells, inhibition of differentiation of some progenitor cells, and development-inducing signals; and the like are known, and as these receptor families, an EGF receptor, an IGF 1 receptor, a fibroblast growth factor receptor (FGFR; hereinafter also simply referred to as "FGFR"), and the like are known.

The FGF family is formed of 23 types of members, and among FGF 1 to FGF 10 that bind to FGFR, the oldest known ones are FGF1 (also sometimes referred to as acidic FGF or aFGF) and FGF2 (also sometimes referred to as basic FGF or bFGF). FGF2 is a protein consisting of 154 amino acids having a molecular weight of 18,000.

Moreover, FGF is known to be involved in angiogenesis, wound healing, embryonic development, and the like as described above, and to play an important role in processes of growth, differentiation, and the like of cells. From the viewpoint of these various functions, attempts have been made to use FGF in a regenerative medicine field and a medical field.

It has been reported that a conditioned medium from a mouse embryonic fibroblast (MEFCM, hereinafter sometimes referred to as a "conditioned medium") and a matrix component are required for long-term growth of undifferentiated human embryonic stem cells (hESCs). It has been reported that the hESCs expresses a receptor of a growth factor (a receptor of a growth factor including bFGF, a stem cell factor (SCF), a fetal liver tyrosine kinase-3 ligand (Flt3L), and the like), and in a case where the hESCs are cultured in a culture solution including only bFGF or in a culture solution including bFGF in combination with other factors, it exhibits characteristics similar to those of control culture of MEFCM not only in terms of morphology but also in terms of a physiological activity, differentiation, or the like (see Non Patent Document 1, which is hereinafter referred to as "Related Art 1").

Moreover, it has been reported that in many cases, ES cells can be cultured without using a conditioned medium in a case where hESCs are cultured in the presence of FGF2 or on a feeder layer of fibroblasts, or cultured in a conditioned medium for fibroblasts, and the concentration of FGF2 is high. Furthermore, in an unconditioned medium added with 4 ng to 250 ng/mL of FGF2, the number of passages of ES cells can be increased under a low density. However, under stringent conditions, in a medium added with 4 to 40 ng/mL of FGF2, the number of passages is 3 or less, but in a case where a medium added with 100 ng/mL of FGF2 is used, there is an effect comparable to that in a case where a conditioned medium is used (see Non Patent Document 2, which is hereinafter referred to as "Related Art 2"). In addition, it has also been reported that a decomposition rate of FGF2 in an unconditioned medium is higher than that in a conditioned medium.

On the other hand, it has been reported that a VHH-type single-domain antibody (also referred to as a single-domain antibody) produced in advance by an immunized llama was rapidly isolated instead of an antibody, and a substance specifically binding to a human FGFR1 could be obtained. Furthermore, it has been reported that a large-scale library was obtained (see Non Patent Document 3, which is hereinafter referred to as "Related Art 3").

### [Citation List]

### [Non Patent Documents]

[Non Patent Document 1]
   Xu, Chunhui et al., Basic Fibroblast Growth Factor Supports Undifferentiated Human Embryonic Stem Cell Growth Without Conditioned Medium, 2005, Stem Cells, vol. 23, No. 3, p. 315-323
[Non Patent Document 2]
   Levenstein, Mark E. et al., Basic Fibroblast Growth Factor Support of Human Embryonic Stem Cell Self-Renewal, 2006, Stem Cells, vol. 24, p. 568-574
[Non Patent Document 3]
   Monegal, Ana et. al., ImMunological applications of single-domain llama recombinant antibodies isolated from a naive library, Protein Engineering, Design & Selection, 2009, vol. 22, No. 4, p. 273-280

### [Summary of Invention]

### [Technical Problem]

Related Art 1 is an invention which was excellent in terms that it was found that hESCs can be cultured in a medium including only bFGF at a sufficiently high concentration without a conditioned medium, or using an unconditioned medium in a culture solution including a combination with other factors. This is because it is not necessary to culture fibroblasts. However, there was a problem that autocrine and paracrine factors produced by hESCs are not suitable for long-term growth of hESCs.

Related Art 2 is an invention which is excellent in terms that it was found that hESCs cells can be cultured even in an unconditioned medium in a case where the medium is added with FGF2; an amount of FGF2 to be added is increased in a case where the culture conditions are stringent; and a decomposition rate of FGF2 is higher in an unconditioned medium than in a conditioned medium.

However, since FGF2 has a short half-life in the medium, there has been a problem in that it is not possible to maintain an effective concentration in a culture product simply by addition of FGF2. That is, there has been a problem that in a case where an unconditioned medium including no conditioned medium is used, it is necessary to add fresh FGF2 to the unconditioned medium every day in order to maintain the concentration of FGF2 in a culture product.

Here, the addition of FGF2 to the medium every day means that it is necessary to continuously produce a FGF2 brought into a state where FGF2 can be purified and added. Furthermore, extremely complicated operations and time are required in order to produce and purify proteins such as FGF2. This had a problem that there is a great concern from the viewpoint of production cost in a large-scale culture required in a case of using the stem cell as described above as a therapeutic agent. In addition, there has been also a problem that a risk of contamination and the like increases in a case where an operation of adding FGF2 almost every day during the culture is performed.

Due to this, there has been a strong social demand for a peptide which is available without performing complicated operations such as production and purification, and has an activity equivalent to that of FGF2.

Related Art 3 is an invention which is excellent in terms that a VHH having the same function as an antibody can be obtained with a simpler operation than purification of an antibody in the related art. However, in Related Art 3, a phage display method is used, infection with a TG1 cell (an Escherichia coli strain for phage display) is performed to express VHH, and screening is performed. This means that even though phage particles are formed, the expression is not done in a case where a construct including a DNA sequence defining a peptide that cannot express TG1 is formed. That is, since a peptide that can express Escherichia coli is restricted, there is a problem that such a peptide cannot be screened from a library.

Due to this, there has been a strong social demand for producing a peptide obtained from a library in a cell-free system.

### [Solution to Problem]

The present invention has been completed to solve such problems.

That is, one aspect of the present invention is a receptor tyrosine kinase agonist which consists of a single-domain antibody having a cell growth activity, and is at least one receptor tyrosine kinase agonist selected from the group consisting of a human fibroblast growth factor receptor 1 (FGFR1), a human fibroblast growth factor receptor 2 (FGFR2), a human fibroblast growth factor receptor 3 (FGFR3), and a human fibroblast growth factor receptor 4 (FGFR4).

Here, the EC₅₀ value of the cell growth activity is preferably 10 µg/mL or less. In addition, the receptor tyrosine kinase agonist is preferably a dimer in which the single-domain antibodies having the same amino acid sequence are linked by a linker.

Moreover, the linker is preferably an oligopeptide linker or a chemical linker. The denaturation temperature (Td) is preferably 65°C or higher, and more preferably 70°C or higher. A value of the equilibrium dissociation constant (KD) is preferably 5 × 10⁻⁹ M or less.

The amino acid sequence of a complementarity determining region (CDR) of the single-domain antibody preferably includes an amino acid sequence represented by SEQ ID NO: 29 or an amino acid sequence represented by SEQ ID NO: 30.

The receptor tyrosine kinase agonist is preferably one in which the amino acid sequence of the single-domain antibody has characteristics of any one of the following (1) to (5).
(1) The 37^{th} (according to Kabat numbering) amino acid is any amino acid selected from the group consisting of tyrosine (Y), phenylalanine (F), and valine (V).
(2) The 41^{st} amino acid is proline (P).
(3) The 44^{th} amino acid is any amino acid selected from the group consisting of glutamine (Q), glycine (G), glutamic acid (E), and lysine (K).
(4) The 45^{th} amino acid is arginine (R) or leucine (L).
(5) The 47^{th} amino acid is any amino acid selected from the group consisting of leucine (L), alanine (A), tryptophan (W), glycine (G), and phenylalanine (F).

The receptor tyrosine kinase agonist is preferably derived from a gene of a heavy-chain antibody of an animal of the family Camelidae or cartilaginous fish. In addition, it is preferable that the animal of the family Camelidae is an alpaca.

Another aspect of the present invention is a medium composition for cell culture, containing 0.1 ng/mL to 10 µg/mL of a receptor tyrosine kinase agonist which consists of a single-domain antibody having a cell growth activity, and is at least one receptor tyrosine kinase agonist selected from the group consisting of a human fibroblast growth factor receptor 1 (FGFR1), a human fibroblast growth factor receptor 2 (FGFR2), a human fibroblast growth factor receptor 3 (FGFR3), and a human fibroblast growth factor receptor 4 (FGFR4). Here, a cell grown by the cell growth activity is preferably at least one selected from the group consisting of a fibroblast, a mesenchymal stem cell, and an iPS cell.

Still another aspect of the present invention is a method for maintaining an undifferentiated state of a stem cell, the method including a step of culturing a stem cell in a medium composition for cell culture, containing 0.1 ng/mL to 10 µg/mL of a receptor tyrosine kinase agonist, at about 36°C to about 38°C, in which the receptor tyrosine kinase agonist includes a single-domain antibody having a cell growth activity, and is at least one receptor tyrosine kinase agonist selected from the group consisting of a human fibroblast growth factor receptor 1 (FGFR1), a human fibroblast growth factor receptor 2 (FGFR2), a human fibroblast growth factor receptor 3 (FGFR3), and a human fibroblast growth factor receptor 4 (FGFR4). Here, in the method for maintaining an undifferentiated state of a stem cell, the stem cell is a mesenchymal stem cell or an iPS cell.

A yet another aspect of the present invention is a use of a medium composition containing 0.1 ng/mL to 10 µg/mL of a receptor tyrosine kinase agonist for cell culture, in which the receptor tyrosine kinase agonist consists of a single-domain antibody having a cell growth activity, and is at least one receptor tyrosine kinase agonist selected from the group consisting of a human fibroblast growth factor receptor 1 (FGFR1), a human fibroblast growth factor receptor 2 (FGFR2), a human fibroblast growth factor receptor 3 (FGFR3), and a human fibroblast growth factor receptor 4 (FGFR4). Here, a cell grown by the cell growth activity is preferably at least one selected from the group consisting of a fibroblast, a mesenchymal stem cell, and an iPS cell.

### [Advantageous Effects of Invention]

According to the present invention, it is possible to provide a receptor tyrosine kinase agonist having excellent stability. In addition, according to the present invention, it is possible to provide a medium composition including the receptor tyrosine kinase agonist. Furthermore, it is possible to provide a method for maintaining an undifferentiated state of a stem cell, the method including a step of culturing the stem cell in the medium composition for cell culture.

### [Brief Description of Drawings]

FIG. 1 is a view showing a scheme of hit compound screening by a cDNA display method.
FIG. 2 is a view showing an elution method for each screening round.
FIG. 3 is a view showing a phage display scheme that is divided into a case where the scheme after panning of an initial library (phage library) is performed using an ELISA plate and a case where the scheme is performed using an immunotube.
FIG. 4 is a view showing CDR regions 1 to 3 of VHH Clone #1 and VHH Clone #2.
FIG. 5 is an electrophoresis image showing results of SDS-PAGE carried out after purification of VHH.
FIG. 6 is a graph showing measurement results of the affinity of VHH Clone #1 for a target molecule FGFR1.
FIG. 7 is a graph showing measurement results of the affinity of VHH Clone #2 for a target molecule FGFR1. The vertical axis indicates a response and the horizontal axis indicates a time (seconds).
FIG. 8A is a graph showing measurement results of the affinity of VHH Clone #1 for a target molecule FGFR2. The vertical axis and the horizontal axis are each the same as those in FIG. 7.
FIG. 8B is a graph showing measurement results of the affinity of VHH Clone #1 for a target molecule FGFR3. The vertical axis and the horizontal axis are each the same as those in FIG. 7.
FIG. 8C is a graph showing measurement results of the affinity of VHH Clone #1 for a target molecule FGFR4. The vertical axis and the horizontal axis are each the same as those in FIG. 7.
FIG. 9 is a graph showing the expression level of phospho-p44/42 MAPK (Erk1/2) by FGF2 or VHH (monomer) stimulation. The vertical axis indicates a relative expression level in a case where the expression level in the absence of a stimulus was set to 1.
FIG. 10 is an electrophoresis image showing results of SDS-PAGE on a homodimer (VHH).
FIG. 11 is a graph showing the expression level of phospho-p44/42 MAPK (Erk1/2) by FGF2 or VHH (homodimer) stimulation. The vertical axis indicates a relative expression level in a case where the expression level in the absence of a stimulus was set to 1.
FIG. 12 is a graph showing measurement results of the EC₅₀ of a VHH monomer of Clone #2 (indicated by "Clone #2 (mono)" in the drawing).
FIG. 13A is a graph showing measurement results of the EC₅₀ of a VHH homodimer of Clone #1 (indicated by "#1 5aa" and "#1 L3" in the drawing). The vertical axis indicates a relative luminescence intensity (RLU) and the horizontal axis indicates a peptide concentration.
FIG. 13B is a graph showing measurement results of the EC₅₀ of a VHH homodimer of Clone #2 (indicated by "#1 5aa" and "#1 L3" in the drawing). The vertical axis indicates a relative luminescence intensity (RLU) and the horizontal axis indicates a peptide concentration.
FIG. 14 is a graph showing results of an inhibition experiment on the growth factor activity of a VHH homodimer by an FGFR antagonist (a case where BGJ398 which is an FGFR inhibitor was not added (indicated by "(-)" in the drawing) and a case of being added in 20 nM). The vertical axis indicates a relative luminescence intensity (RLU) and the horizontal axis indicates a type and a concentration of a peptide added.
FIG. 15 is a graph showing results of a cell growth assay performed on a VHH monomer. The vertical axis indicates a relative luminescence intensity in a case where none was added (indicated as (-) in the drawing) and a case where Clone #1, Clone #2, or FGF2 was added at the displayed concentration.
FIG. 16 is a graph showing measurement results of the thermostability of VHH (evaluation of the heat resistance of Clone #1) by a protein thermal shift assay method. The vertical axis indicates a fluorescence intensity.
FIG. 17 is a graph showing measurement results of the thermostability of VHH (evaluation of the heat resistance of Clone #1L4) by a protein thermal shift assay method. The vertical axis indicates a fluorescence intensity.
FIG. 18 is an alignment of VHH Clone #1 (FGFR #1), VHH Clone #2 (FGFR #2), a humanized VHH, a human VH reference, a universal VHH, CAMDR, LAMGL, and VICPA.
FIG. 19 is a gel electrophoresis image in a case where a VHH clone (coiled coil body, hereinafter sometimes referred to as "VHH-C") expressed using Corynebacterium was purified.
FIG. 20 is a gel electrophoresis image obtained in a case where VHH (Fc body, hereinafter sometimes referred to as a "VHH-Fc") expressed using AAVpro293T cells was purified.
FIG. 21 is a gel electrophoresis image of an FGFR1 extracellular domain (hereinafter sometimes abbreviated as "FGFR1-DIIDIII", "FGFR1-DII", or "FGFR1-DIII") expressed using AAVpro293T cells.
FIG. 22 is a sensorgram showing measurement results of the binding activity of an FGFR1 extracellular domain to VHH Clone #1 by a biolayer interference method. (A) of FIG. 22 shows the measurement results of the binding activity of FGFR1-DIIDIII, (B) of FIG. 22 shows the measurement results of the binding activity of FGFR1-DII, and (C) of FIG. 22 shows the measurement results of the binding activity of FGFR1-DIII.
FIG. 23 is a gel electrophoresis image showing results of examining agonist activities of VHH-C (indicated by "#1-C" in the drawing) and VHH-Fc (indicated by "#1-Fc" in the drawing) using NIH3T3 cells.
FIG. 24 is a graph showing results of examining the cell growth activities of VHH-C and VHH-Fc on NIH3T3 cells. The horizontal axis indicates a concentration of a peptide added and the vertical axis indicates a relative luminescence amount.
FIG. 25 is a graph showing a comparison of the growths of cultured iPS cells in a case where bFGF or a homodimer #1-L4-#1 of VHH was used.
FIG. 26 is an optical microphotograph on Day 5 in a third passage of iPS cell culture in a case where bFGF or a homodimer #1-L4-#1 of VHH was used.
FIG. 27 is a photograph visualized by immunostaining the expressions of OCT4 and SSEA4 which are undifferentiation markers in an iPS cell (2 passages, Day 6 (Culture day 20)) cultured using bFGF or a homodimer VHH.
FIG. 28 is an optical microphotograph showing the morphology (4 passages, Day 1) of an MSC in a case where bFGF or a homodimer VHH was added to perform culture in a 10% or 20% FBS-containing medium.
FIG. 29 is a graph showing a comparison of the growths of cultured MSCs in a case where a basic FGF or a homodimer #1-L4-#1 of VHH was added to a 10% or 20% FBS-containing medium.
FIG. 30 is a graph showing a comparison of expressions of MSC markers in MSCs (fourth passage) cultured with the addition of bFGF or a homodimer #1-L4-#1 of VHH to a 10% FBS-containing medium, using flow cytometry.
FIG. 31 is a graph showing a comparison of expressions of MSC markers in MSCs (fourth passage) cultured with the addition of bFGF or a homodimer #1-L4-#1 of VHH to a 20% FBS-containing medium, using flow cytometry.
FIG. 32 is an optical microphotograph of an MSC (3-passage subculture) after being cultured for 14 days in a medium for growth or a medium for fat cell differentiation induction.
FIG. 33 is a graph showing quantitative evaluation of the absorbance of Oil Red O extracted with Isopanol after Oil Red O staining of an MSC which has been induced to differentiate into a fat cell. The bFGF, a VHH, and a FBS below the graph indicate medium conditions of the MSC.
FIG. 34 is an optical microphotograph of an MSC (3-passage subculture) after being cultured in a medium for growth or a bone differentiation induction medium for 14 days.
FIG. 35 is a graph showing quantitative evaluation of the absorbance of Alizarin Red obtained by staining an MSC which has been induced to differentiate into the bone with Alizarin Red, and then extracting the Alizarin Red with a calcified knot lysate. The bFGF, a VHH, and a FBS below the graph indicate medium conditions of the MSC.
FIG. 36 is a graph showing EC₅₀'s of the growth of cultured fibroblasts in a case where bFGF or a homodimer #1-L4-#1 of VHH was added to a culture medium.
FIG. 37 is a graph showing results of examining the inhibition of a growth factor activity of bFGF and a homodimer #1-L4-#1 of VHH by FGFR kinase inhibitor, using fibroblasts. The vertical axis indicates a relative luminescence intensity with the background as a control.
FIG. 38 is a graph showing a cell growth activity for a fibroblast of a homodimer #1-L4-#1 of VHH after incubation at 37°C in D-PBS for one week or bFGF immediately after solutionizing a freeze-dried product in D-PBS.

### [Description of Embodiments]

An aspect of the present invention is a novel receptor tyrosine kinase agonist, in which the receptor tyrosine kinase agonist is: (s1) at least one selected from the group consisting of a human fibroblast growth factor receptor 1 (FGFR1), a human fibroblast growth factor receptor 2 (FGFR2), a human fibroblast growth factor receptor 3 (FGFR3), and a human fibroblast growth factor receptor 4 (FGFR4); (s2) consists of at least one single-domain antibody; and (s3) has a cell growth activity.

Here, the receptor tyrosine kinase (RTK) is a cell surface receptor having a high affinity for many polypeptide-type growth factors, cytokines, and hormones. In the human genome, 90 types of tyrosine kinase genes have been identified, of which 58 types encode receptor tyrosine kinases. The receptor tyrosine kinases have been shown not only to be important regulators of normal cell functions but also to play a significant role in the development and progression of many types of cancers.

The receptor tyrosine kinases are known as an enzyme-linked receptor together with protein kinases such as a tyrosine kinase-linked receptor, a receptor-like tyrosine phosphatase, a receptor serine/threonine kinase, a receptor guanyl cyclase, and a histidine kinase-linked receptor. Here, the "enzyme-linked receptor" refers to an enzyme itself or a receptor that directly activates a linking enzyme. Usually, the enzyme-linked receptor has a single-pass transmembrane structure, and an enzyme portion of the receptor is held in the cell.

The fibroblast growth factor (hereinafter sometimes referred to as "FGF") is a family composed of 23 members known as proteins involved in angiogenesis, wound healing, embryogenesis, and the like. This family binds to heparin and transmits signals through four receptor tyrosine kinases referred to as FGFR1, FGFR2, FGFR3, and FGFR4.

The FGF receptor (hereinafter sometimes referred to as "FGFR"), which is a receptor of FGF, is a family composed of four members of FGFR1 to FGFR4, and has a common structure. Specifically, FGFR1 to FGFR4 all have three immunoglobulin loops (I, II, and III) in the extracellular domain, and have a tyrosine kinase in the cell.

The binding counterpart of the receptor tyrosine kinase agonist of the present invention is any of the four FGFRs, and is preferably FGFR1 or FGFR2, and more preferably FGFR2. FGF1 to which FGFR1 binds is also referred to as FGF-acidic or aFGF, and is a non-glycosylated heparin-bound growth factor expressed in the brain, kidney, retina, smooth muscle cells, bone matrix, osteoblasts, astrocytes, or endothelial cell. This is because FGF1 is involved in the growth, differentiation, survival, and the like of various tissues and cells. In addition, FGF2 to which FGFR2 binds is also known as bFGF or FGF-basic 154, and is an important component for maintaining an undifferentiated state of an ES cell in the cell culture system.

Here, the "agonist" is a substance that acts on a receptor molecule in the living body to exhibit the same function as a neurotransmitter, a hormone, or the like, and is a substance other than the substance that actually acts in the living body.

In the present specification, the term "antibody" is used in the broadest sense, and is not particularly limited as long as it has a desired binding activity to an antigen. Specifically, the antibody includes both a naturally occurring immunoglobulin (Ig) and a substance including an artificially synthesized structure in a part thereof (hereinafter sometimes referred to as an "artificial antibody"). In addition, the antibody includes not only the entire immunoglobulin, but also a fragment thereof as long as it exhibits a desired antigen-binding activity. A bispecific antibody and other specific antibodies are also included in the artificial antibody.

In addition, the antibody may be produced as a monoclonal antibody or may be produced as a polyclonal antibody.

A general antibody molecule (IgG: immunoglobulin molecule) derived from a human, a mouse, or the like is a glycoprotein composed of a plurality of units, and each unit includes four polypeptide chains. The four polypeptide chains may be composed of two identical heavy chains (hereinafter sometimes referred to as "H chains") and two identical light chains (hereinafter sometimes referred to as "L chains"). The H chain and the L chain are linked by one disulfide crosslinkage to constitute one unit (heterodimer), and two heterodimers are linked by two disulfide crosslinkages to form IgG having a "Y" letter shape.

Since the amino terminal of a polypeptide chain constituting the IgG has a large change in the amino acid sequence, it is referred to as a constant region (hereinafter sometimes referred to as a "C region") having a relatively small change in the amino acid sequence, which is distinguished from a variable region (hereinafter sometimes referred to as a "V region"). Furthermore, each L chain is composed of one variable domain VL and one constant domain CL. The H chain is composed of one variable domain VH and three constant domains CH1, CH2, and CH3. Since the molecular weight of each L chain is about 25,000, whereas each H chain has about twice the number of amino acids of the L chain, it also has a molecular weight of about 50,000. As a result, the total molecular weight of immunoglobulin molecule monomers is about 150,000.

Since the H chain and the L chain are held together by a combination of a noncovalent interaction and a covalent disulfide crosslinkage between the chains, the H chain and the L chain form a bilaterally symmetrical structure. The V regions of the H chain and the L chain may each be represented by VH and VL. Since each of the V regions includes an antigen-binding site of an immunoglobulin molecule, the Ig monomer includes two antigen-binding sites, and is thus said to be divalent. The H chain region between the first and second C region domains is referred to as a hinge region, and is held by a disulfide crosslinkage. The hinge region is flexible and can allow a distance between the two antigen-binding sites to be changed. The hinge region is found in IgG, IgA, and IgD, but is not found in IgM and IgE.

In addition, from the viewpoint of the bindability, the immunoglobulin molecule can be divided into two regions of Fab and Fc. Fab is obtained by treating the immunoglobulin molecule with papain which is a proteolytic enzyme. The region where Fab is removed from the immunoglobulin molecule is Fc. Then, the "Fc region" is used to define a C-terminal region of the immunoglobulin heavy chain which includes at least a part of the constant region. This term includes a natural sequence Fc region and a variant Fc region. In the present specification, the Fc region of the H chain of human IgG includes a region from Cys226 or Pro230 to the carboxyl terminal of the H chain. However, the C-terminal lysine (Lys447) of the Fc region is not present in some cases.

With respect to the above-described general immunoglobulin molecules, an antibody, which lacks an L chain and is composed only of an H chain, found in an animal of the family Camelidae and cartilaginous fish, is referred to as a "heavy-chain antibody" or a "single heavy-chain antibody".

Here, the single heavy chain means consisting of only a heavy chain and not forming a double chain of a heavy chain and a light chain as in the immunoglobulin. It is a single chain rather than a double chain even in a case where two heavy chains form a dimer by, for example, a disulfide bond.

In addition, a variable region antibody of a monomer of a heavy-chain antibody obtained from an animal of the family Camelidae is referred to as a "single-domain antibody (sdAb)" or a "variable domain of a heavy chain" (hereinafter sometimes referred to as "VHH"). This is a type of an antibody fragment which consists of a single variable molecule variable domain, and lacks an L chain and a CH domain of an H chain of an Fab region of an antibody molecule in the related art. However, the antibody can correspond to an antigen-binding domain in which a normal antibody is composed of an H chain and a L chain. VHH exhibits the same selectivity and specificity to an antigen as a normal antibody, but since VHH has a low molecular weight, it has high resistance to high-temperature heat, a surfactant, a high-concentration urea, or the like.

In the present specification, VHH means the smallest unit (domain) of an antibody structure having a single immunoglobulin fold structure and 2 or 3 complementarity determining regions (hereinafter sometimes referred to as "CDR") inside the structure. The immunoglobulin fold exhibits a structure (β-barrel structure) in which 2 β-sheets are combined like a sandwich, where one β-sheet consists of 3 to 5 antiparallel β-strands and the other β-sheet consists of 4 antiparallel β-strands. Each β-strand is arranged in order to move between two sheets.

Human- and mouse-derived VH regions, and camel-derived VHH have 3 CDRs. On the other hand, a shark-derived variable domain of a new antigen receptor (VNAR) has 2 CDRs (Wang, Yongzhong and 8 others, Nanobody-derived Nanobiotechnology tool kits for diverse biomedical and biotechnology applications, International Journal of Nanomedicine, 2016, Vol. 11, 3287-3303).

A method for generating a single-domain antibody in the present invention will be described later.

In the present specification, the "cell growth activity" refers to an activity that indicates whether or not the agonist of the present invention can cause cells to grow for the specific cells, and in a case where a concentration of the agonist at the time of reaching the number of cells of 50% relative to the number of cells at the time of reaching 100% of growth is defined as EC₅₀, the lower the value of EC₅₀, the higher the growth activity. The lower limit of EC₅₀ is not particularly limited, but is, for example, 0.1 µg/mL or more. In the agonist, the EC₅₀ value of the cell growth activity is preferably 10 µg/mL or less because of the suppression of culture cost, suppression of aggregate formation, and the like, and the EC₅₀ value is more preferably 8 µg/mL or less, and still more preferably 5 µg/mL or less.

In addition, the "agonist" is a substance that acts on a receptor molecule in the living body to exhibit the same function as a neurotransmitter, a hormone, or the like, and is referred to as a substance other than the substance that actually acts in the living body. For reasons such as acceleration of dimerization of a receptor tyrosine kinase, it is preferable that the single-domain antibody and a dimer thereof according to the present embodiment have an agonist activity of a receptor tyrosine kinase.

The single-domain antibody and a dimer thereof according to the present invention preferably induce multimerization of a receptor tyrosine kinase on the surface of a cell. This is because the receptor tyrosine kinase efficiently transmits a signal by forming a dimer as follows. That is, the receptor tyrosine kinase, which is a single-pass transmembrane glycoprotein consisting of three domain structures having an extracellular domain that binds to a ligand, a transmembrane domain, and an intracellular domain having a kinase activity, generally forms a dimer by binding to a ligand. As a result, the kinase domains are physically brought close to each other, and the tyrosine residues in the kinase domains are mutually phosphorylated to be activated. Then, an effector molecule binds to the activated kinase domain, and a tyrosine residue of the effector molecule is phosphorylated to be activated. In this manner, various proteins in the cell are activated one after another.

In the present embodiment, the single-domain antibody and a dimer thereof preferably have one or more VHH domains, and more preferably have two VHH domains. This is because the larger the number of VHH domains, which are heavy chain variable regions, the more the improvement of the affinity for an antigen can be expected.

In a case where the single-domain antibody and a dimer thereof according to the present embodiment have two VHH domains, it is preferable that the two VHH domains are linked by a linker. Examples of the linker include an oligopeptide linker and a multimerized peptide linker.

Here, the oligopeptide linker is a linker consisting of an oligopeptide, and is usually composed of 2 to 20 amino acid residues.

The multimerized peptide linker may be any linker through a peptide bond, and examples thereof include GSGGG, (GSGGG)2, (GSGGG)4, GSAGSAAGSGEF, and GSEGKSSGSGSESKST. In addition, as an example of a case of utilizing a self-assembling property of the peptide, there is a method through a peptide chain having a coiled-coil structure rich in hydrophobic amino acids, and specifically, there is a method in which peptides are dimerized by using a Leucine-Zipper sequence (Vinson, Charles and 5 others, Molecular and Cellular Biology, 2002, Vol. 22, No. 18, p. 6321-6335). Examples of the multimerization domain protein linker include an antibody Fc domain in the dimerization.

As one of the indices for evaluating the performance of the receptor tyrosine kinase agonist of the present embodiment, there is a structural stability thereof. The high structural stability leads to high practicality in terms of mass production by a recombinant microorganism or the like, preservation stability, stability during use, and the like. As an index of the structural stability of the protein, there is heat resistance, which can be expressed as a denaturation midpoint (Td: also referred to as a denaturation temperature) value by a thermal shift assay method, that is, by examination of the temperature dependence of structural changes.

The denaturation temperature (Td) of the receptor tyrosine kinase agonist of the present embodiment is not particularly limited, but is preferably 65°C or higher, and more preferably 70°C or higher.

The Td value can be measured by, for example, differential scanning calorimetry (DSC) or a SYPRO Orange addition method (Huynh, Kathy, Partch, Carrie L., Analysis of protein stability and ligand interactions by thermal shift assay, Current Protocols in Protein Science, 2015, Vol. 79, p. 28.9.1-28.9.14). Even by any of methods, it is possible to monitor a thermal change or the like that occurs in a case where a protein structure in the cell is transferred or denatured at the time of an increase (decrease) in temperature at a constant rate.

The Td is evaluated by a Td value at the time of raising the temperature from 25°C to 95°C (temperature rising rate: 1°C/min) under the solution conditions of a protein concentration (0.5 mg/mL) in a D-PBS solution (including 0.2 g of KH₂PO₄, 1.15 g of Na₂HPO₄, 0.2 g of KCl, and 8 g of NaCl per liter of the aqueous solution, pH 7.4).

An equilibrium dissociation constant (KD) of the receptor tyrosine kinase agonist of the present embodiment is not particularly limited, but is preferably 5 × 10⁻⁹ M or less, more preferably 1 × 10⁻⁹ M or less, and still more preferably 5 × 10⁻¹⁰ M or less. This is because the lower the value of KD, the higher the affinity. The lower limit of KD is not particularly limited, but is, for example, 1 × 10⁻¹² M or more. Here, KD is a value obtained by an affinity measurement test by a surface plasmon resonance (SPR) method. A detailed measurement method for KD is as described in Examples which will be described later.

The amino acid sequence of a complementarity determining region (CDR) of the single-domain antibody preferably includes an amino acid sequence of SEQ ID NO: 29 or an amino acid sequence of SEQ ID NO: 30, more preferably includes an amino acid sequence having 60% or more homology to the amino acid sequence of SEQ ID NO: 29 or the amino acid sequence of SEQ ID NO: 30, and still more preferably includes an amino acid sequence having 70% or more homology to the amino acid sequence of SEQ ID NO: 29 or the amino acid sequence of SEQ ID NO: 30.

The homology (identity) between the amino acid sequences is calculated based on an "alignment" in which two sequences are aligned to obtain the highest matching. The homology between the two aligned sequences is expressed by a percentage of amino acids having matching or similar physical properties. That is, the amino acid sequence is represented by a proportion (%) of the "number of amino acids having matching or matching physical properties" in one amino acid sequence serving as a reference sequence with respect to the "total number of amino acid residues" in the other amino acid sequence. Among the properties of the amino acid, there are physical properties such as hydrophilicity, hydrophobicity, acidity, basicity, and neutrality. Examples of a preferred computer program method for determining the alignment and the homology include, but are not limited to, a method in a GCS program package (Devereux, John and 2 others, A comprehensive set of sequence analysis programs for the VAX, Nucleic Acid Research, 1984, Vol. 12, No. 1, p. 387-395); BLAST/FASTA (Altschul, Stephen F. and 4 others, Basic local alignment search tool, Journal of Molecular Biology, 1990, Vol. 215, No. 3, p. 403-410); Clustal Omega (Sievers, Fabian, Higgins, Desmond G., Clustal Omega for making accurate alignments of many protein sequences, Protein Science, 2018, Vol. 27, No. 1, p. 135-145); and the like.

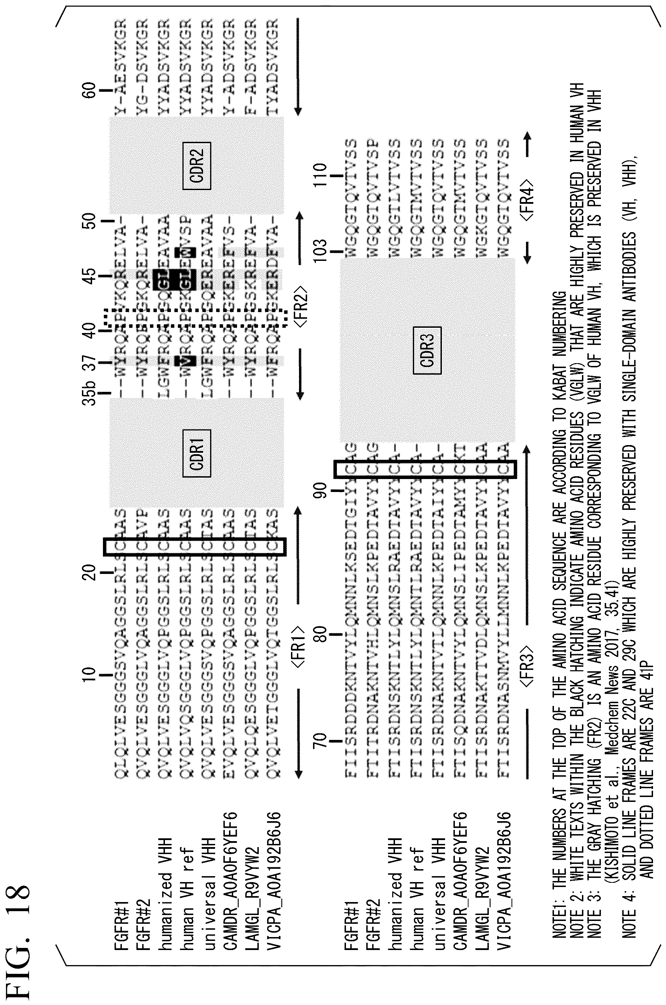

The amino acid sequence of a framework (non-CDR region) of the single-domain antibody is considered to be important for the stability and the solubility of a structure thereof, and the immunogenicity or the stability in blood in a case where the single-domain antibody is used as a therapeutic agent. An alignment of VHH Clone #1 (SEQ ID NO: 17), VHH Clone #2 (SEQ ID NO: 18), humanized VHH (SEQ ID NOs: 31 to 34; excluding CDR), a human VH reference (SEQ ID NOs: 35 to 38; excluding CDR), universal VHH (SEQ ID NOs: 39 to 42; excluding CDR), CAMDR (accession number: A0A0F6YEF6; SEQ ID NOs: 43 to 46; excluding CDR), LAMGL (accession number: R9VYW2; SEQ ID NOs: 47 to 50; excluding CDR), and VICPA (accession number: A0A192B6J6; SEQ ID NOs: 51 to 54; excluding CDR) was created with a computer software GENETYX veR13 (Genetyx Corporation) (FIG. 18).

The amino acid numbers in the alignment are determined in accordance with "Kabat numbering" (Dondelinger, Mathieu and 6 others, Understanding the Significance and Implications of Antibody Numbering and Antigen-Binding Surface/Residue Definition, Frontiers in Immunology, 2018, Vol. 9, p. 2278) (hereinafter sometimes simply referred to as "Kabat No." or "Kabat number"). In addition, the homology between Clone #1 and a framework sequence (non-CDR region) of each of the other single-domain antibodies was calculated by the homology analysis of GENETYX veR13.

Humanized VHH (Vincke, Cecile and 5 others, General Strategy to Humanize a Camelid Single-domain Antibody and Identification of a Universal Humanized Nanobody Scaffold, Journal of Biological Chemistry, 2009, Vol. 284, No. 5, p. 3273-3284; hereinafter sometimes referred to as "Vincke's literature") is VHH that has been humanized. Human VH reference (Chen, Weizao and 4 others, Construction of a Large Phage-Displayed Human Antibody Domain Library with a Scaffold Based On a Newly Identified Highly Soluble, Stable Heavy Chain Variable Domain, Journal of Molecular Biology, 2008, Vol. 382, No. 3, p. 779-789) is a representative example of human VH.

Universal VHH (Saerens, Dirk and 8 others, Identification of a Universal VHH Framework to Graft Non-canonical Antigen-binding LoopsoFcamel Single-domain Antibodies, Journal of Molecular Biology, 2005, Vol. 352, No. 3, p.597-607) has a framework sequence that serves as an acceptor suitable for transplantation of a CDR sequence of another antibody. CAMDR, LAMGL, and VICPA are each a representative example of VHH of the genus Lama, the genus Camelidae, and the genus Vicuna.

As a result of examining homology, it was found that the homology values between VHH Clone #1, and Clone #2, humanized VHH, human VH reference, universal VHH, CAMDR, LAMGL, and VICPA were each 78%, 72%, 72%, 77%, 78%, 75%, and 75%. Based on the above, it can be determined that the single-domain antibody in the present invention preferably has a framework sequence of 70% or more homology to the framework sequence of SEQ ID NO: 17.

As a characteristic of the amino acid sequence of the framework 2 (FR2) of a human VH (hVH) antibody (see FIG. 18), 37V, 44G, 45L, and 47W which are four hydrophobic amino acids originally important for binding to an L-chain variable region (VL) are highly conserved in hVH (the numbers are Kabat No.). The amino acids of VHH corresponding to these four sites in hVH are each 37F/Y (F/Y represents F or Y; the same applies hereinafter), 44E, 45R/C, and 47F/G (Satoshi Kishimoto, Yuji Ito, Application of Antigen-Binding Domain VHH Derived from Heavy-Chain Antibody of Animal of Family Camelidae to Medical Technology, Medchem News, 2017, Vol. 27, No. 1, p. 35-41). In addition, with reference to the alignment in FIG. 18, 44Q/K and 47L/A are also used in Clone #1 and Clone #2 of the present invention. As an example of humanization of a VHH antibody, substitutions of amino acids at the positions 44 and 45 of VHH with G and L have been proposed (see the document of Vincke et al.).

As shown in FIG. 18 as a characteristic of other single-domain antibodies hVH and VHH, cysteine at the 41^{st} proline (FR2), the 22^{nd} (FR1), and the 92^{nd} (FR3) in FR2 are highly conserved. These 22C and 92C form a disulfide bond, and are considered to be important for the stability of a single-domain antibody structure (Ban, Bhupal and 2 others, Optimization of Methods for the Production and Refolding of Biologically Active Disulfide Bond-Rich Antibody Fragments in Microbial Hosts, Antibodies, 2020, vol. 9, p. 39).

On the other hand, there are also methods for substituting an amino acid in FR2 or the like with an amino acid used in VHH for the purpose of improving the physical properties of hVH (Liu, Jianbin and 2 others, Construction and characterization of a camelized, human, VH-based peptide vaccine against CD20 antigen, Immunotherapy, 2013, Vol. 5, No. 2, p. 135-141; and Soler, Miguel A. and 7 others, Effect of Humanizing Mutations on the Stability of the Llama Single-Domain Variable Region, Biomolecules, 2021, Vol. 11, p.163). The single-domain antibody in the present invention also includes at least amino acid substitution made to modify the characteristics and the functions of the framework amino acid sequences of the VHH and the VH.

The amino acid sequence of the receptor tyrosine kinase agonist preferably includes a framework sequence having 70% or more homology to a non-CDR amino acid sequence (framework sequence) of SEQ ID NO: 17.

The amino acid sequence of the receptor tyrosine kinase agonist preferably has characteristics of any one of the following (1) to (5).
(1) The 37^{th} (according to Kabat numbering) amino acid is any one amino acid selected from the group consisting of tyrosine (Y), phenylalanine (F), and valine (V).
(2) The 41^{st} amino acid is proline (P).
(3) The 44^{th} amino acid is any one amino acid selected from the group consisting of glutamine (Q), glycine (G), glutamic acid (E), and lysine (K).
(4) The 45^{th} amino acid is arginine (R) or leucine (L).
(5) The 47^{th} amino acid is any one amino acid selected from the group consisting of leucine (L), alanine (A), tryptophan (W), glycine (G), and phenylalanine (F).

The single-domain antibody and a polymer thereof according to the present embodiment are preferably derived from a gene of a natural heavy-chain antibody. The gene of the natural heavy-chain antibody is preferably derived from an animal of the family Camelida or a cartilaginous fish, and more preferably derived from the animal of the family Camelida. Examples of the animal of the family Camelid include animals of the genus Camelid, including dromedary and Bactrian camel, animals of the genus Vicuna, including vicuna and alpaca, and animals of the genus LLama, including guanaco and llama, and among these, the alpaca is preferable.

According to another aspect of the present invention, in the medium composition for cell culture and a use of the medium composition for cell culture in the present embodiment the medium composition is a medium composition containing the above-described receptor tyrosine kinase agonist at 0.1 ng/mL to 10 µg/mL.

In the medium composition for cell culture and the use of the medium composition for culturing cells of the present embodiment, the medium composition can include substantially the same components as those of a medium composition used in culturing cells in the related art, as components other than the above-described receptor tyrosine kinase agonist and water.

Here, a cell grown by the cell growth activity of the receptor tyrosine kinase agonist is preferably at least one selected from the group consisting of a fibroblast, a mesenchymal stem cell, and an iPS cell.

The mesenchymal stem cell, which is one of stem cells that are present in adult bodies, is a cell having an ability to differentiate into bones or cartilages, blood vessels, and cardiomyocytes, which are mesoderm-derived tissues, and is also referred to as a "mesenchymal stromal cell" since 1980's (Mizukami, Amada, Swiech, Kamilla, Stem Cells International, Volume 2018, Article ID 4083921, https://doi.org/10.1155/2018/4083921). In recent years, it has been reported that they can be differentiated into ectoderm-derived nerve cells or glial cells (with functions such as supporting nerve cells) and endoderm-derived hepatocytes. In the present technical field, the "mesenchymal stem cell" is abbreviated as "MSC".

The fibroblast is one of the representative cells constituting the connective tissue, and exhibits basophilicity. In the tissue, the cell is a flat or spindle-shaped cell which is present along the long shaft of the collagen fiber bundle and retains a large number of cytoplasmic neurites. The cell has an oval nucleus, has abundant Golgi apparatus and rough endoplasmic reticulum, and is particularly well-developed in areas where extracellular matrix synthesis is active (such as a wound healing area). The cell in the active phase and the cell in the resting phase are sometimes distinguished by being referred to as a fibroblast and a fibrocyte, respectively. The fibroblast is a relatively undifferentiated cell that is differentiated from a mesenchymal stem cell, and is considered to have a differentiation potency into a fat cell, a cartilaginous blast, or an osteoblast. In addition, the fibroblast hardly divides in adults, but divides as necessary (wound healing and the like).

The iPS cell is an induced pluripotent stem cell, which has differentiation pluripotency that the cell can differentiate into a very large number of cells such as ES cells (embryonic stem cells) by introducing four kinds of genes into somatic cells, and a self-renewal ability that the cell can maintain the characteristics even after undergoing division and growth.

Still another aspect of the present invention is a method for maintaining an undifferentiated state of the present embodiment, the method including a step of culturing a stem cell at about 36°C to about 38°C in a medium composition for cell culture, containing 0.1 ng/mL to 10 µg/mL of the above-described receptor tyrosine kinase agonist. In this method, the above-described medium composition for cell culture is used.

The stem cell is a non-specialized cell having a self-renewal ability and a differentiation potency. The self-renewal is an ability to maintain an undifferentiated state (that is, to generate the same female cell as a mother cell) even after the stem cell undergoes a plurality of cycles of cell division, and the differentiation potency is an ability of a stem cell to differentiate into specialized cell types found in the body, such as a neuron, a hepatocyte, or a muscle cell. The stem cells are roughly classified into two types, that is, adult stem cells and embryonic stem cells. The adult stem cells are considered to be in an undifferentiated state and are present in the differentiated cells of a tissue. Major roles thereof are maintenance and repair of tissues in which adult stem cells are present. The adult stem cells are pluripotent, but their differentiation is limited to differentiation into various cell types within the tissue from which they are derived. The embryonic stem cells (ESCs) are separated from early embryos before implantation. Unlike adult stem cells, the ESCs can divide over longer-term culture, and have an ability to differentiate into any cell types that are present in the human body, and are thus referred to as pluripotent stem cells (PSCs). As other types of pluripotent stem cells, there is an induced pluripotent stem cell (iPSC or an iPS cell) that can be manufactured by reprogramming a somatic cell such as a fibroblast.

The method for maintaining an undifferentiated state of a stem cell of the present embodiment is substantially the same as a method for maintaining an undifferentiated state of a stem cell in the related art, except that the above-described medium composition including a receptor tyrosine kinase agonist, is used.

In the method for maintaining an undifferentiated state of a stem cell of the present embodiment, the cell grown by the cell growth activity of the receptor tyrosine kinase agonist is preferably at least one selected from the group consisting of a fibroblast, a mesenchymal stem cell, and an iPS cell. These cells are as described above.

The receptor tyrosine kinase agonist of the present invention can be prepared by a cDNA display method. The outline will be described below.

A detailed description will be given below of a general selection cycle using the cDNA display method. Here, a method for acquiring a peptide that is bound to an anti-FLAG antibody will be described as an example.

### (1) Preparation of mRNA

A case of using a library DNA having a desired sequence and a DNA encoding a FLAG sequence will be described as an example. For example, the DNAs are mixed at a molar ratio of 25,000 to 100,000:1 to prepare a DNA mixture, 250 to 1,000 ng is taken from the DNA mixture, and transcription is performed at a scale of 10 to 20 µL using a kit such as RiboMAX Large Scale RNA Production Systems-T7.

The DNA mixture is incubated, for example, at about 37°C for 3 to 5 hours, and then a desired amount of a DNase is added thereto. For example, the DNA mixture is incubated at about 37°C for about 4 hours, about 0.5 µL of a DNase (for example, RQ1 Dnase, manufactured by Promega Corporation) attached to the kit is added thereto, and incubation is further performed at about 37°C for about 10 minutes to obtain an mRNA. The obtained mRNA can be purified using, for example, After Tri-Reagent RNA CleanUp Kit (manufactured by Favorgen Biotech Corp.).

### (2) Photocrosslinking

Next, the mRNA obtained as described above and the linker are linked to each other by irradiation with long-wave UV (for example, at about 350 to about 370 nm) for 0.5 to 5 minutes. The obtained linker-mRNA conjugate is subjected to cell-free translation at a desired scale in the same manner as described above. For example, 10 to 20 pmol of the mRNA-linker conjugate is translated at a scale of 100 to 150 µL at about 30°C for about 15 minutes, using a cell-free translation system such as a rabbit reticulocyte erythrocyte lysate. Thereafter, MgCl₂ and KCl are added thereto, for example, so that the concentrations are each about 70 to 80 mM and about 850 to 950 mM, and the mixture is incubated at about 37°C for about 1 hour to obtain a translation reaction solution including the mRNA-peptide conjugate. This translation reaction solution includes IW in which a peptide is further linked to the mRNA-linker.

### (3) Preparation of cDNA Display

About 0.25 to about 0.75 M of EDTA (pH about 7.8 to about 8.2) is added to the translation reaction solution obtained as described above so that the final concentration is about 80 to 85 mM, and incubation is performed at room temperature for about 3 to 7 minutes. Subsequently, for example, an equal amount of 2 × a binding buffer for SA (including about 20 mM Tris-HCl (pH about 7.5), about 2 M NaCl, about 2 mM EDTA, and about 0.2% Tween-20) is added thereto and mixed with 100 to 200 µL of magnetic beads such as, for example, Dynabeads MyOne C1 Streptavidin, which has been prewashed with 1 × a binding buffer for SA, and the mixture is stirred at about 20°C to 30°C for about 20 to 40 minutes.

Next, the magnetic beads are washed 2 to 4 times, for example, with about 200 µL of 1 × a binding buffer for SA, and for example, according to a protocol attached to ReverTra Ase (registered trademark), about 100 µL of a reverse transcription reaction solution is put thereinto and the mixture is stirred at about 42°C for about 15 minutes to perform reverse transcription, thereby preparing an mRNA/cDNA-peptide conjugate (hereinafter sometimes referred to as a "cDNA display").

### (3-1-4) Purification of mRNA/cDNA-Peptide Conjugate

Next, for example, Dynabeads MyOne C1 Streptavidin is washed with about 150 µL of 1 × NE Buffer 4, then about 75 µL of 1 × NE Buffer 4 including about 200 U of RNase T1 is added thereto, and the mixture is stirred at about 37°C for about 1 hour. Then, about 75 µL of 2 × a His-tag washing buffer (about 40 mM sodium phosphate (pH about 7.4) including about 1 M NaCl and about 0.1% Tween-20) is added thereto, and the supernatant is recovered.

Next, for example, about 150 µL of the recovered supernatant and about 20 µL of His Mag Sepharose Ni (manufactured by GE Healthcare, washed with 1 × a His-tag washing buffer) are mixed, and the mixture is stirred at room temperature for about 1 hour using a mixer such as Intelli-Mixer RM-2M (manufactured by Toho K. K.).

Thereafter, the mixture is washed 1 to 3 times with about 100 µL of 1 × a His-tag washing buffer, about 30 µL of a selection buffer with an increased EDTA concentration (about 50 mM Tris-HCl buffer (pH about 7.4) including about 1 M NaCl, about 10 mM imidazole, about 5 mM EDTA, and about 0.1% Tween-20) is added thereto, the mixture is stirred at room temperature for about 10 minutes using the mixer, and then the supernatant is recovered.

Subsequently, for example, an appropriate column such as MicroSpin Empty Columns (manufactured by GE Healthcare) is filled with about 25 to 100 µL of an anti-FLAG M2 affinity gel (about 40% to 60% suspension), and washed twice to four times with about 150 to 250 µL of a selection buffer. Thereafter, about 50 to 150 µL of the supernatant is taken therefrom, loaded onto a column, and stirred with a rotator at room temperature for about 1 hour. The column is washed 3 to 5 times with about 150 to 250 µL of the selection buffer, then about 50 to 150 µL of 3xFLAG Peptide (manufactured by Sigma-Aldrich Japan) at about 50 to 150 ng/µL is added thereto, and the mixture is stirred at room temperature for about 15 minutes, using the rotator, whereby an mRNA/cDNA-peptide conjugate bound to the anti-FLAG M2 affinity gel can be competitively eluted.

### (3-1-5) PCR

The eluate in the column is recovered by a centrifugal operation, subjected to ethanol-precipitation, and then dissolved in a desired amount of Nuclease-free water. The ethanol-precipitation product is added to about 150 to 250 µL of a PCR reaction solution to perform PCR. For example, the eluate in the column recovered by a centrifugal operation is ethanol-precipitated using Quick-Precip Plus Solution or the like, and dissolved in about 15 µL of Nuclease-free water. The ethanol-precipitation product is diluted with about 150 to 250 µL of a PCR reaction solution (1 × PrimeSTAR Buffer (containing Mg²⁺) including about 0.2 mM dNTPs, about 0.4 µM T7Qnew, about 4 µM NewYtag for cnvK, and about 0.02 U/µL of PrimeSTAR HS DNA Polymerase) to perform PCR as follows. As the primer used herein, for example, T7Qnew and NewYtag for cnvK (SEQ ID NOs: 3 and 4 in the sequence listing) can be used. The sequences of these primers are shown below.
[SEQ ID NO: 3] 
5’-
GATCCCGCGAAATTAATACGACTCACTATAGGGGAAGTATTTTTACAACAATTA
CCAACA-3’
[SEQ ID NO: 4]
   5'-TTTCCACGCCGCCCCCCGTCCTGCTTCCGCCGTGATGAT-3'

Next, PCR can be performed, for example, (a1) at 98°C for 1 minute, (b1) at 98°C for 15 seconds, (c1) at 68°C for 30 seconds, and (d1) at 68°C for 1 minute, with (b1) and (c1) set to be run 25 cycles. The obtained PCR product is subjected to SDS gel electrophoresis to cut out and purify a full construct DNA according to an ordinary method. In a case where the purified full construct DNA is added to a desired amount of a PCR reaction solution and the solution is dispensed at a desired amount to perform PCR again, a double-stranded DNA can be obtained.

More specifically, a PCR product obtained by performing PCR as described above is electrophoresed on, for example, an 8 M urea-modified 6% PAGE, and a full construct DNA (260- to 300-mer) is cut out and purified according to an ordinary method. The purified full construct DNA is added to about 150 to 250 µL of a PCR reaction solution (1 × PrimeSTAR Buffer (containing Mg²⁺) including about 0.2 mM dNTPs, about 0.4 µM Newleft, about 0.4 µM NewYtag for cnvK, and about 0.02 U/µL of PrimeSTAR HS DNA Polymerase), and the solution is dispensed at 25 to 75 µL to perform about 5 cycles of PCR, whereby a double-stranded DNA can be obtained. The sequence of the Newleft (SEQ ID NO: 5 in the sequence listing) exemplified as the primer used herein is shown below.

[SEQ ID NO: 5]
GATCCCGCGAAATTAATACGACTCACTATAGGG

PCR can be performed, for example, (a2) at 98°C for 1 minute, (b2) at 98°C for 10 seconds, (c2) at 68°C for 30 seconds, and (d2) at 68°C for 1 minute, with (b2) and (c2) set to be run 5 cycles. The PCR reaction solution after performing PCR as described above is collected, column-purified, and used in the next round.

The procedure, from transcription of library DNA to affinity selection, is performed as desired rounds to obtain a complex molecule (display molecule) in which a DNA sequence encoding a target binding peptide (in this case, a FLAG peptide) and a peptide are integrated. The above is a selection cycle using the cDNA display method used in the present invention.

As described above, a peptide that binds to a desired antigen, for example, FGF 1 or FGF2 can be obtained.

### [Examples]

Hereinafter, the present invention will be described in more detail with reference to Examples, but the present invention is not limited to Examples which will be described below and various modifications can be made without departing from the gist of the present invention.

### (Example 1)

### [Method] Screening of Hit Compound

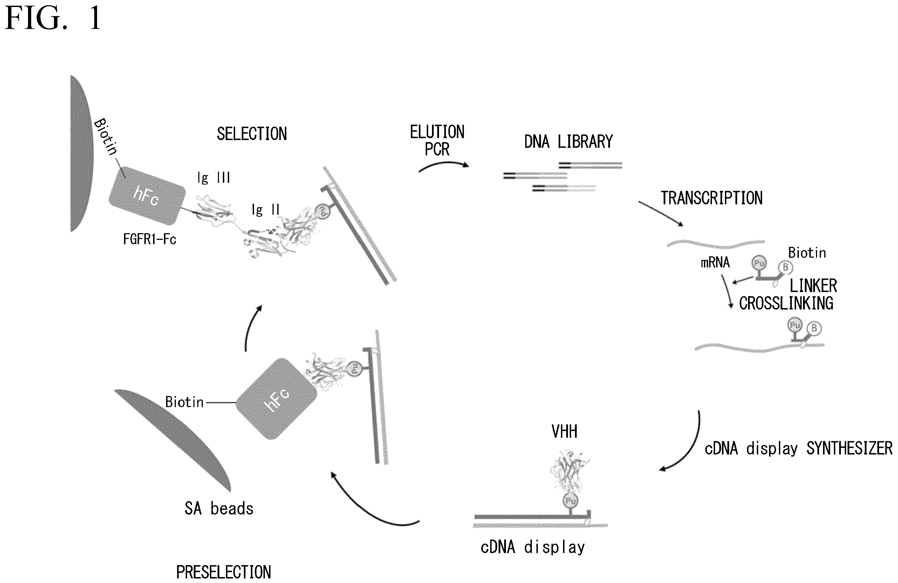

Screening of a hit compound was carried out from a library of VHH-displaying cDNA display molecules, using a cDNA display method. FIG. 1 shows the outline of a scheme of hit compound screening by a cDNA display method.

### 1. Immobilization of Target Molecule.

As a target molecule used for screening, a human fibroblast growth factor receptor 1 (FGFR1) was used.

In addition, as proteins used in the screening test, a protein A and a protein B, each consisting of an extracellular region of FGFR1, and a protein C used as a negative control for the protein B were used.

Protein A: Recombinant human FGFR1 protein (Active), purchased from Abcam plc (Cat. no. ab168696; hereinafter referred to as "FGFR-His")

Protein B: FGF ReceptoR1β (IIIc)/Fc Chimera Human Recombinant Carrier-free, purchased from R & D Systems (Cat. no. 661-FR; hereinafter referred to as "FGFR-Fc").

Protein C: IgG1 (Fc) Human Recombinant Carrier-free, purchased from R & D Systems (Cat. no. 110-HG; hereinafter referred to as "hFc")

The target molecule and the like were biotinylated by the following procedure. 20 equivalents of a biotinylation reagent (EZ-link sulfo NHSSS Biotin; manufactured by Thermo Fisher Scientific Inc.) was added to each of FGFR1-Fc (protein B) and hFc (protein C), and the mixture was reacted at 25°C for 30 minutes. Thereafter, the unreacted biotinylation reagent was removed with a desalting column (Zeba Spin Desalting Columns; manufactured by Thermo Fisher Scientific Inc.).

### 2. Synthesis of cDNA Display

### (1) Manufacture of DNA Library Encoding Single Variable Region Fragment of Full-Length Alpaca-Derived Antibody (Single Variable Domain of the Heavy Chain of a Heavy-Chain Antibody (VHH))

Amplification by PCR was performed with an S hinge VHH-specific primer (SEQ ID NO: 1, SEQ ID NO: 2) and an L hinge VHH-specific primer (SEQ ID NO: 1 and SEQ ID NO: 3), using an S hinge and an L hinge, which are two kinds of alpaca-derived naive VHH library genes provided by RePHAGEN Co., Ltd., as templates. Then, extension PCR was performed using a primer for overlap PCR (SEQ ID NO: 4, SEQ ID NO: 5) (Table 1 below) in order to prepare a DNA fragment consisting of a T7 promoter, an omega (ω) enhancer, a Kozak consensus sequence, a VHH gene, a His tag, and a linker hybridization region (Y tag), thereby manufacturing a full-length VHH-encoded DNA library.

**[Table 1]**

| Name of primer | Base sequence (5' → 3') | Sequence length (b) | SEQ ID NO: |
|---|---|---|---|
| VHH-specific primer | CAT TTT ACA TTC TAC AAC TAC AAG CCA CCA TGG CTG AGT TGC AGC TCG TGG | 51 | 1 |
| | GCC GCC GTG ATG ATG ATG ATG ATG GCT ACC ACC TCC CGA TTG TGG TTT TGG TGT CTT GGG TTC TGA GGA G | 70 | 2 |
| | GCC GCC GTG ATG ATG ATG ATG ATG GCT ACC ACC TCC CGA TTG TGG TTT TGG TGT CTT GGG | 60 | 3 |
| Primer for overlap PCR | GAT CCC GCG AAA TTA ATA CGA CTC ACT ATA GGG GAA GTA TTT TTA CAA CAA TTA CCA ACAACA ACA ACA AAC AAC AAC AAC ATT ACA TTT TAC ATT CTA CAA CTA CAA GCC ACC ATG | 110 | 4 |
| | TTT CCA CGC CGC CCC CCG TCC TGC TTC CGC CGT GAT GAT GAT GAT GAT GGC TGC CTC CCC C | 61 | 5 |

### (2) Preparation of ^{cnv}K rG Linker

The ^{cnv}K rG Linker has a main chain and a side chain, and the base sequence of a biotin fragment serving as the main chain is 5'-AAgAATTTCCAKGCCGCCCCCCGVCCT-3' (SEQ ID NO: 55). Here, BioTEG is bound to the 5' terminal of the main chain. In the base sequence, g represents guanosine, V represents Amino C6-dT, and K represents 3-cyanovinylcarbazole.

In addition, the puromycin segment that serves as a side chain of the ^{cnv}K rG Linker has a structure of 5'-(5S)TCTFZZCCP. P, which serves as a free terminal, in the side chain sequence represents puromycin as a protein binding site. Furthermore, (5S) represents 5' Thiol C6, F represents FITC-dT, and Z represents SpaceR18.

The chemical synthesis of the main chain and the side chain was deputed to TSUKUBA OLIGO SERVICE CO., LTD. (Ushiku, Ibaraki Prefecture).

First, 15 nmol of a biotin fragment (final concentration: 150 µM) and EMCS (manufactured by Dojin Chemical Laboratories, final concentration: 16.7 mM) were added to a 0.2 M sodium phosphate buffer (pH 7.2), and incubation was performed at 37°C for 30 minutes. Thereafter, ethanol-precipitation was performed using Quick-Precip Plus Solution (manufactured by Edge Biosystems Inc.).

Next, the puromycin segment corresponding to 37.5 nmol was dissolved in a 1 M aqueous disodium hydrogen phosphate solution including 50 mM DTT so that the final concentration was 417 µM, and the solution was stirred at room temperature for 1 hour using a shaker. Then, the buffer was exchanged with a 0.02 M sodium phosphate buffer (pH 7.0) including 0.03 M of NaCl using NAP 5 Column (manufactured by GE Healthcare Bioscience, Inc.).

The reduced puromycin segment solution which had been subjected to pre-buffer exchange was mixed with the ethanol-precipitation product of the EMCS-modified biotin fragment, and the mixture was left at 4°C overnight. Subsequently, DTT was added to the reaction solution so that the final concentration of 50 mM, and the mixture was stirred at room temperature for 30 minutes. Thereafter, ethanol-precipitation was performed using Quick-Precip Plus Solution (manufactured by Edge Biosystems Inc.). The ethanol-precipitation product was dissolved in 100 µL of Nuclease-Free water (manufactured by Nacalai Tesque, Inc.).

The dissolution product was separated by 12% polyacrylamide gel electrophoresis, and the ^{cnv}K rG Linker fraction was cut out. The cut-out gel was crushed using a BioMasher II set (Nippi, Inc.), 500 µL of Nuclease-Free water was added thereto, and the mixture was stirred overnight at 4°C to extract ^{cnv}K rG Linker. The stirred solution was transferred to a Costar (registered trademark) Spin-X (registered trademark) centrifugal tube filter and 0.22 µm cellulose acetate (Corning), and then centrifuged at 16,000 × g for 15 minutes to separate the gel and the extracted liquid. Thereafter, ethanol-precipitation was performed using Quick-Precip Plus Solution to obtain desired ^{cnv}K poly A Linker. The obtained ^{cnv}K poly A linker was dissolved in Nuclease-Free water and preserved at -20°C.

### (Example 2) Manufacture of cDNA Display

Each cDNA display was prepared as follows. The buffers to be used are described in Table 2 below.

**[Table 2]**

| Name of buffer | Composition |
|---|---|
| 2 × binding | 20 mM Tris-HCl, 2 mM EDTA, 2 M NaCl, 0.2% Tween 20 (pH 7.5) |
| Binding (selection) | 50 mM Tris-HCl, 0.5 M NaCl, 1 mM EDTA, 0.05% Tween 20 (pH 7.4) |
| His Tag binding/washing | 20 mM Phosphate, 0.5 M NaCl, 20 mM imidazole (pH 7.4) |
| His Tag elution | 20 mM Phosphate, 0.5 M NaCl, 250 mM imidazole (pH 7.4) |
| Selection | PBS, 0.05% Tween 20, 0.1% BSA |

### (1) Transcription

Using T7 RiboMAX ExpreSS Large Scale RNA Production System (manufactured by Promega Corporation), transcription of the DNA for synthesizing the cDNA display encoding VHH manufactured in "2. Synthesis of cDNA Display" of Example 1 was performed according to the attached manual. The amount of the DNA used was 6.6 µg for the 1 st round and 0.1 to 1 µg for the 2nd round and thereafter. The obtained transcripts were purified using RNaClean XP (manufactured by Beckman Coulter, Inc.) according to the attached manual. The concentration of the purified product was quantified by NanoPad DS-11FX (DeNovix Inc.).

### (2) Ligation

NaCl (final concentration: 0.2 M) and a Tris-HCl buffer (pH 7.5, final concentration: 0.05 M) were added to 20 pmol of the purified mRNA and 20 pmol of ^{cnv}K rG Linker manufactured in "2. Synthesis of cDNA Display" in Example 1, and incubation was performed at 90°C for 1 minute. Thereafter, the temperature was lowered to 70°C at a rate of 0.1°C/sec and incubation was performed at 70°C for 1 minute. Then, the temperature was lowered to 25°C at a rate of 0.1°C/sec and the temperature was lowered to 10°C at a rate of 2°C/sec to hybridize ^{cnv}K rG Linker on the 3'-terminal side of the mRNA.

Thereafter, irradiation with UV at 365 nm was performed for 5 minutes, using Handheld UV Lamp, 6 W, UVGL-56, 254/365 nm, and 100 V (Analytik jena US, An EndreSS + Hauser Company), to photocrosslink ^{cnv}K rG Linker and mRNA, thereby obtaining an mRNA-linker.

### (3) Preparation of mRNA Display

6 pmol of the mRNA-linker was incubated at 30°C for 30 minutes using a 50 µL-scale cell-free translation system (Rabbit reticulocyte Lysate (nuclease-treated), manufactured by Promega Corporation). Next, MgCl₂ and KCl were added thereto so that the final concentrations were each 75 mM and 900 mM, and the cells were incubated at 37°C for 1 hour to display the peptides corresponding to mRNAs in the puromycin on the mRNA-linker. Next, EDTA (pH 8.0) was added thereto so that the final concentration was 70 mM, and the mixture was incubated at 4°C for 5 minutes to remove ribosomes bound to the mRNA-linker, thereby forming an mRNA display.

### (4) Preparation of cDNA Display

60 µL of Dynabeads Myone streptavidin C1 (manufactured by Thermo Fisher Scientific Inc.) was placed in Protein Lobind Tube, and washed with 200 µL of a binding buffer, then the mRNA display prepared by the methods of (1) to (3) above was added thereto, and the mixture was stirred at 25°C for 30 minutes. After washing the cells with 200 µL of a binding buffer, incubation was performed in a reaction solution having the composition shown in Table 3 at 42°C for 30 minutes to perform a reverse transcription reaction, thereby preparing an mRNA/cDNA-VHH conjugate. After washing with 200 µL of a binding buffer, 39 µL of a His tag binding/washing buffer, and 1 µL of 1,000 U/µL of RNase T1 were added thereto, the mixture was stirred at 37°C for 15 minutes, and the mRNA/cDNA-VHH conjugate (cDNA display) was eluted from Dynabeads MyOne streptavidin C1.

**[Table 3]**

| Composition | Amount (µL) |
|---|---|
| 25 mM dNTP Mix (NIPPON GENE) | 2 |
| GeneAce Reverse Transcriptase (200 U/µL) (NIPPON GENE) | 1 |
| 5 × RT Buffer (NIPPON GENE) | 10 |
| Nuclease-free water | 37 |

### (5) Purification of cDNA Display Molecule

30 µL of His Mag Sepharose Ni Beads (GE Health Care) was put into a 1.5 /mL microtube and washed with 200 µL of a His tag binding/washing buffer, then the cDNA display molecule prepared by the methods in the sections (1) to (4) was were added thereto, and the mixture was stirred at 25°C for 30 minutes. After washing with 200 µL of a His tag binding/washing buffer, 30 µL of a His tag elution buffer was added thereto, and the mixture was stirred at 37°C for 15 minutes to elute the cDNA display molecules.

### (Example 3) Selection of VHH

### 1. Selection of Target VHH That Binds to Ligand

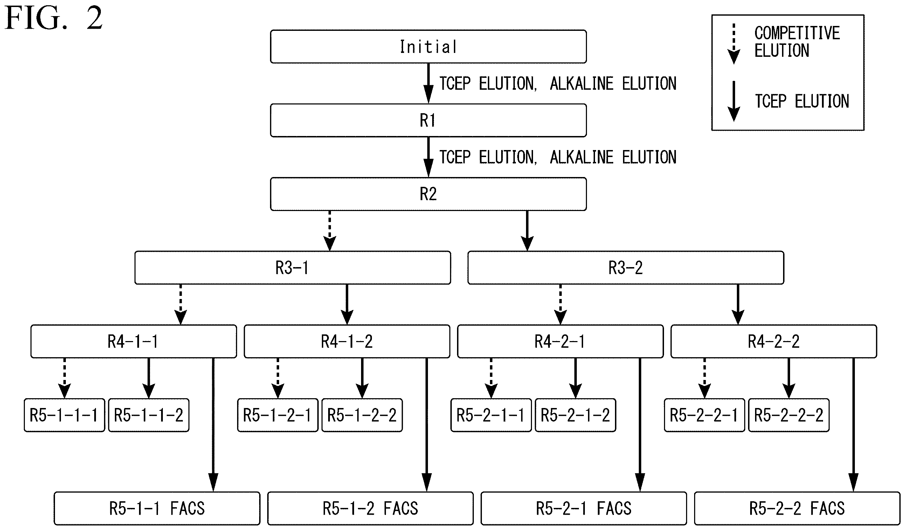

(1) to (5) of Example 2 were performed as one round, the library obtained at the end of the first round was defined as R1, and the library obtained at the end of the second round was defined as R2. The libraries used in the selection performed in the present Example and synthesis scales thereof are shown in Table 4. In addition, in the present Example, the cDNA display molecules obtained in each round of Example 2 were immobilized on beads, and these were eluted with TCEP for Initial and R1, and then eluted with alkali. In R2 to R4, the obtained eluates were divided equally, one of the eluates was subjected to competitive elution, and the other thereof was subjected to TCEP elution. A screening test flowchart showing case classification by the elution method is shown in FIG. 2. With regard to the eluate of R4, the elution fraction obtained by elution TCEP was analyzed by FACS.

**[Table 4]**

| VHH-displaying cDNA display library scale for each selection round | |
|---|---|
| Selection round | Amount of mRNA-linker conjugate |
| Round 1 (initial library) | 192 pmol |
| Round 2 | 12 pmol |
| Round 3 | 6 pmol |
| Round 4 | 3 pmol |
| Round 5 | 3 pmol |

### 1-1. Screening Procedure of Selection Cycle 1 (Round 1 (R1))

The biotinylated FGFR1-Fc prepared in "1. Immobilization of Target Molecule" of Example 1 was added to a low protein binding tube charged with 1 mg of Dynabeads MyOne streptavidin C1, and mixed by inversion at 25°C for 30 minutes, and beads on which a 100 pmol fraction of FGFR-Fc had been immobilized were taken in a new tube in which FGFR-Fc-immobilized beads had been manufactured. The cDNA display prepared in (1) to (5) of Example 2 was added thereto and mixed by inversion at 25°C for 30 minutes, the mixture was washed four times with a selection buffer, and subsequently, a TCEP elution operation was performed.

That is, 40 µL of 10 mM tris(2-carboxyethyl)phosphine (TCEP) was added into the tube, which was left to stand at 25°C for 15 minutes to cleave and elute an SS bond of the biotinylation reagent. 10 µL of 10 mM NaOH was further added into the tube, and the mixture was left to stand at 25°C for 10 minutes and subjected to additional alkaline elution. This alkali extraction operation was performed twice continuously. After the alkaline elution, 4 µL of a 1 M Tris-HCl buffer (pH 8.0) was added to a tube charged with the obtained extract to neutralize the extract. 16 µL of a selection buffer was added to a tube charged with Dynabeads MyOne streptavidin C1 after the alkaline elution, the mixture was left to stand at room temperature for 1 minute, and the supernatant was taken and mixed with the alkaline eluate.

The cDNA included in the cDNA display molecule of R1 obtained as described above was amplified by PCR as follows. A PCR product was obtained by performing PCR under the conditions of (a2) at 98°C for 1 minute, (b2) at 98°C for 10 seconds, (c2) at 68°C for 30 seconds, and (d2) at 68°C for 1 minute, with the steps (b2) and (c2) set to be run 25 cycles, using ^{cnv}K NewYtag for poly A (SEQ ID NO: 6) and T7 omeganew (SEQ ID NO: 7) as primers. The obtained PCR product was subjected to 4%-modified PAGE at 200 V for 25 minutes, and it was confirmed that the cDNA included in the cDNA display molecule was amplified.

A PCR product obtained as described above was purified using Agencourt AMpure XP (manufactured by Beckman Coulter, Inc.) according to the attached manual, and then the obtained purified product R1 was subjected to the second round (R2) of selection as a library.

**[Table 5]**

| Name of sequence | Base sequence (5' → 3') | Sequence length (b) | SEQ ID NO: |
|---|---|---|---|
| ^{cnv}K NewYtag for poly A | TTT CCA CGC CCC CCG TCC T | 22 | 6 |
| T7 omeganew | GAT CCC GCG AAA TTA ATA CGA CTC ACT ATA GGG GAA GTA TTT TTA CAA TTA CCA ACA | 60 | 7 |

### 1-2. Screening Procedure of Selection Cycle 2 (R2)

### (1) Manufacture of hFc-Immobilized Beads

hFc-Immobilized beads were manufactured in the same manner as in 1-1., except that the biotinylated FGFR1-Fc was replaced with a biotinylated protein C (hFc).

### (2) Preparation of R2 Library

Beads on which 10 pmol of hFc had been immobilized and the cDNA display library prepared from the DNA obtained in Example 2(1) were added to 100 µL of a selection buffer and mixed by inversion at 25°C for 60 minutes, and then the supernatant was recovered. The supernatant was equally divided and added to a tube charged with a 10 pmol fraction of FGFR1-Fc-immobilized beads or the same amount of hFc-immobilized beads, and 100 pmol of hFc was added thereto and mixed by inversion at 25°C for 60 minutes.

200 µL of a selection buffer was added to a tube charged with each of the beads, and washed four times, and after the washing, 40 µL of 10 mM TCEP was added to each tube, and the mixture was left to stand at room temperature for 15 minutes for elution. After removing the eluate, an operation of further adding 10 µL of 10 mM NaOH to each tube and eluting at 25°C for 10 minutes was repeated twice, and then 16 µL of a selection buffer was added to each tube and eluted at room temperature for 1 minute. After the elution, a buffer (hereinafter sometimes simply referred to as an "eluate") including an eluate obtained by immediately adding 4 µL of a 1 M Tris-HCl buffer (pH 8.0) to each tube was neutralized. Each of the eluates was subjected to PCR for amplification under the conditions of (a2) at 98°C for 1 minute, (b2) at 98°C for 10 seconds, (c2) at 68°C for 30 seconds, and (d2) at 68°C for 1 minute, with the steps (b2) and (c2) set to be run 25 cycles, using ^{cnv}K NewYtag for poly A (SEQ ID NO: 6) and T7 omeganew (SEQ ID NO: 7) as primers. Next, the obtained two kinds of PCR products were purified using Agencourt AMpure XP according to the attached manual, and then mixed in equal amounts to obtain an R2 library.

### 1-3. Screening Procedure of Selection Cycle 3 (R3)

For R3, screening was performed by dividing into two fractions, which were a TCEP elution fraction and a competitive elution fraction. A 10 pmol fraction of the hFc-immobilized beads and the cDNA display library shown in Table 4 were taken into a tube, and 100 µL of a selection buffer was added thereto and mixed by inversion at 25°C for 60 minutes. Subsequently, the obtained supernatant was recovered and divided in half, a 10 pmol fraction of FGFR1-Fc or hFc was added to each of the immobilized beads, and 50 pmol of hFc, 14 pmol of streptavidin, and 100 ng/µL of salmon sperm DNAs were added thereto and mixed by inversion at 25°C for 60 minutes.

### (1) Competitive Elution

After the completion of the mixing operation, a competitive elution operation was performed according to the following procedure. 200 µL of a selection buffer was added to a tube charged with each of the beads and washed three times, and then 40 µL of a selection buffer was added thereto and washed at 4°C for 30 minutes by mixing by inversion. Then, a 100 pmol fraction of FGFR1-His was added to each tube and mixed by inversion at 25°C for 30 minutes, and competitive elution of the bound cDNA display molecule was performed to obtain an eluate. The eluate was subjected to PCR for amplification under the conditions of (a2) at 98°C for 1 minute, (b2) at 98°C for 10 seconds, (c2) at 68°C for 30 seconds, and (d2) at 68°C for 1 minute, with the steps (b2) and (c2) set to be run 25 cycles, using ^{cnv}K NewYtag for poly A (SEQ ID NO: 6) and T7 omeganew (SEQ ID NO: 7) as primers, and the obtained PCR product was purified according to the attached manual using Agencourt AMpure XP to obtain a library of R3-1.

### (2) TCEP Elution

In the TCEP elution, the same operation as that in the section 1-2 was performed to carry out elution. Each eluate was separately subjected to a PCR reaction and purified using Agencourt AMpure XP according to the attached manual to obtain a library of R3-2.

### 1-4. Screening Procedure of Selection Cycle 4 (R4)

In the competitive elution of R4, each of cDNA display libraries separately prepared from the DNA library obtained in the section 1-3 was taken into a separate tube, and beads on which 1 pmol of FGFR1-Fc had been immobilized, 1,000 µL of a selection buffer, 50 pmol of hFc, 140 pmol of streptavidin, and 100 ng/µL of salmon sperm DNAs were added thereto and mixed by inversion at 4°C for 3 hours. The beads in each tube were washed 3 times with 200 µL of a selection buffer and then divided into two portions, a phosphate buffered saline including 20 µL of Tween (registered trademark)-20 (hereinafter sometimes referred to as "PBS-T") was added thereto and mixed by inversion at 4°C for 16 hours under the condition of either the addition or absence of 10 pmol of FGFR1-His to perform competitive elution. Thereafter, 40 µL of 10 mM TCEP was added to each tube and the mixture was left to stand at room temperature for 15 minutes to perform TCEP elution.

Among the elution operations above, the eluate obtained by the competitive elution was subjected to PCR for amplification under the conditions of (a2) at 98°C for 1 minute, (b2) at 98°C for 10 seconds, (c2) at 68°C for 30 seconds, and (d2) at 68°C for 1 minute, with the steps (b2) and (c2) set to be run 25 cycles, using ^{cnv}K NewYtag for poly A (SEQ ID NO: 6) and T7 omeganew (SEQ ID NO: 7) as primers, thereby obtaining R4-1-1 and R4-2-1 DNA libraries.

TCEP elution was performed in the same manner as in 1-2, the obtained competitive eluate was used as an R4-1-2 library, and the TCEP eluate was used as an R4-2-2 library.

### 1-5. Screening Procedure of Selection Cycle 5 (R5)

In the competitive elution of R5, each of FGFR1-Fc-immobilized beads or hFc-immobilized beads and a cDNA display library separately prepared from the DNA library obtained in 1-3 were used.

Beads on which 1 pmol of FGFR1-Fc had been immobilized or beads on which hFc had been immobilized were separately taken in a tube, and a cDNA display library each prepared separately from the DNA library obtained in the section 1-3 was added thereto and bound under the same conditions as in the section 1-3. 20 µL of PBS-T and 10 pmol of FGFR1-His were added to each of the tubes, the mixture was left to stand at room temperature, and after 20 hours, the supernatant was recovered from each of the tubes (E1). Subsequently, the same amount of PBS-T and FGFR1-His was added thereto, the mixture was left to stand at room temperature, and after 40 hours, the solution was recovered again (E2). Thereafter, TCEP was added to each tube to elute the cDNA display molecules (E3).

The obtained competitively eluted solutions were separately subjected to PCR for amplification under the conditions of (a2) at 98°C for 1 minute, (b2) at 98°C for 10 seconds, (c2) at 68°C for 30 seconds, and (d2) at 68°C for 1 minute, with the steps (b2) and (c2) set to be run 25 cycles, using ^{cnv}K NewYtag for poly A (SEQ ID NO: 6) and T7 omeganew (SEQ ID NO: 7) as primers, and mixed in an equal amount to obtain R5-1-1-1, R5-2-1-1, R5-1-2-1, and R5-2-2-1 DNA libraries. TCEP elution was performed in the same manner as in the section 1-2 to obtain R5-1-1-2, R5-2-1-2, R5-1-2-2, and R5-2-2-2 DNA libraries.

In the selection cycle 5 (R5), selection was carried out using a separate fluorescence-activated cell sorting (FACS).

First, 100 µL of Biotin-Fluorescein in an amount of 10 µg/mL dissolved in PBST was added to beads on which a 70 pmol fraction of hFc had been immobilized, stirred at room temperature for 60 minutes, and then left to stand on a magnetic plate for 1 minute to remove the supernatant. 200 µL of PBST was added thereto, pipetting was performed, and then the mixture was left to stand on a magnetic plate for 1 minute to remove the supernatant. After repeating this operation three times, 70 µL of PBST was added thereto to manufacture hFc-immobilized fluorescent beads.

A 2 pmol fraction of hFc-immobilized fluorescent beads and a 0.5 pmol fraction of FGFR1-Fc-immobilized beads were mixed in a low protein binding tube, and then an mRNA-linker conjugate separately prepared from the DNA obtained in the section 1-3, a 1 pmol fraction of a cDNA display library, 50 µL of a selection buffer, 50 pmol of streptavidin, and 500 ng/µL of salmon sperm DNAs were added into the tube to undergo a reaction at 4°C for 1 hour. The supernatant was removed from this tube, 200 µL of a selection buffer was added thereto to wash the beads in the tube three times, and then 500 µL of a selection buffer was added thereto to obtain a solution for sorting. The obtained solution for sorting was set in FACS (Cell Sorter SH800, manufactured by SONY Corporation), a sample flow path and droplet formation conditions at the time of fractionation were automatically set according to the system, and sorting was carried out.

In the FACS analysis, a region in which magnetic beads were present was identified based on particle diameters, and the fluorescence intensity at a time of irradiating the regions with a laser at a wavelength of 488 nm was measured. From the rapid results, a region in which the hFc-immobilized fluorescent beads and the FGFR1-Fc-immobilized beads were present was identified, sorting was performed, and 500,000 particles were each recovered in different low protein binding tubes. Thereafter, centrifugation was performed at 13,000 × g for 10 minutes. After the centrifugation, the mixture was left to stand on a magnetic plate for 10 minutes, then the supernatant was removed, and 20 µL of RNase free water was added thereto for suspension. The suspension was subjected to PCR for amplification under the conditions of (a2) at 98°C for 1 minute, (b2) at 98°C for 10 seconds, (c2) at 68°C for 30 seconds, and (d2) at 68°C for 1 minute, with the steps (b2) and (c2) set to be run 30 cycles, using ^{cnv}K NewYtag for poly A (SEQ ID NO: 6) and T7 omeganew (SEQ ID NO: 7) as primers to obtain R5-1-1-FACS, R5-2-1-FACS, R5-1-2-FACS, and R5-2-2-FACS DNA libraries.

### 2. NGS Analysis

Sequence analysis using a next generation sequencer (NGS, MiSeq system, catalog number SY-410-1003, manufactured by Illumina, Inc.) was performed in order to confirm a degree of convergence of the DNA library in detail by an in vitro selection cycle. A sequence sample was prepared with reference to a 2-step PCR Amplicon Library Preparation method provided by Illumina, Inc. First, Amplicon PCR was performed using a PCR product obtained by each selection as a template, and using the NGS Fw 1st PCR primer (SEQ ID NO: 8) and the NGS Rv 1st PCR primer (SEQ ID NO: 9) as primers. The PCR conditions are as follows: (s1) at 98°C for 1 minute, then (s2) at 98°C for 10 seconds, at 62°C for 5 seconds, and at 72°C for 35 seconds, and (s3) at 72°C for 1 minute, (s2) set to be run 15 cycles.

The obtained PCR product was purified using Agencourt AMpure XP (Beckman Coulter, Inc.) according to the instructions attached to this kit. The obtained purified PCR product was then subjected to Index PCR according to the instructions of Illumina, Inc. to manufacture a library for NGS analysis. The obtained Index PCR product was purified using Agencourt AMpure XP according to the attached instructions, and the concentration of the obtained purified product was quantified using NanoPad DS-11FX (DeNovix).

Next, sequence analysis was carried out using MiSeq (manufactured by Illumina, Inc.) and a MiSeq Reagent Nano kit v2 (500 cycles) according to the instructions of Illumina, Inc. The obtained DNA sequence was translated into the VHH amino acid sequence and positioned according to the frequency of appearance, and it was confirmed that the obtained elution fraction included more concentrated candidate compounds, as compared with the library used.

**[Table 6]**

| Name of primer | Base sequence (5' → 3') | Sequence length (b) | SEQ ID NO: |
|---|---|---|---|
| NGS Fw 1st PCR primer | TCG GCA GCG TCA GAT GTG TAT AAG AGA CAG NNN NAT GGC TGA GGT GCA GCT CGT G | 58 | 8 |
| NGS Rv lst PCR primer | GTC TCG TGG GCT CGG AGA TGT GTA TAA GAG ACA GNN NNT GAT GGC TAC CAC CTC CCG | 63 | 9 |

### 3. Screening for Hit compound Using Phage Display Method

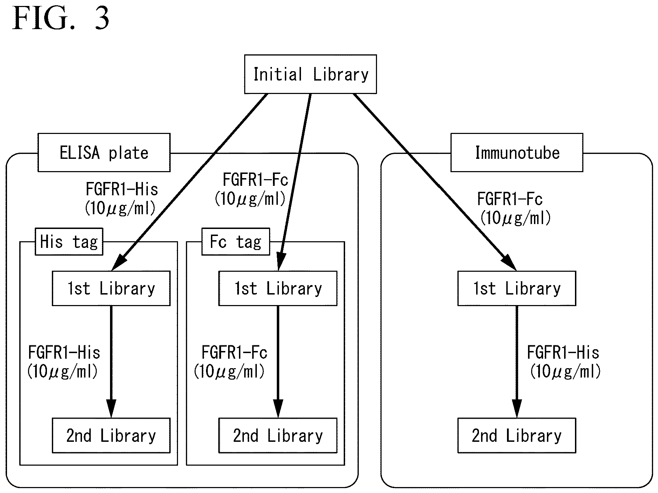

As described above, the DNA library concentrated by the cDNA display method is subjected to a phage display method to further increase the concentration rate. Therefore, the acquisition of a target compound (hereinafter sometimes referred to as a "hit compound") was carried out. Differences due to panning in the schemes of phage display are shown in FIG. 3.

### 3-1. Restriction Enzyme Treatment of Phagemid and Each DNA Library

A mixture of each DNA library obtained in the section 1-3. or 1-5. was used as a template. Alp-to-pPK4-Nco-Sfi-VHH-F (SEQ ID NO: 10) and Alp-GGGS-HisTag-to-pPK4-R (SEQ ID NO: 11) were used as primers. PCR was performed under the conditions of an annealing temperature of 55°C and an extension reaction of 60 seconds, a restriction enzyme recognition sequence for ligation with a phagemid vector was added to the obtained PCR product, and purification was performed with Agencourt AMpure XP (manufactured by Beckman Coulter Inc.). The phagemid and each DNA library were sequentially treated with two kinds of restriction enzymes. First, FastDigest BamHI (manufactured by Thermo Fisher Scientific Inc.) was added thereto to perform a treatment at 37°C for 1 hour, and then FastDigest SfiI (manufactured by Thermo Fisher Scientific Inc.) was added thereto to perform a treatment at 50°C for 1 hour. Thereafter, each DNA library was purified using Agencourt AMpure XP according to the attached manual.

**[Table 7]**

| Name of primer | Base sequence (5' → 3') | Sequence length (b) | SEQ ID NO: |
|---|---|---|---|
| Alp-to-pPK4-Nco-Sfi-VHH-F | CCG GCC ATG GCC ACT GCG GCC SAG KTG CAG CTC GTG GAG TC | 41 | 10 |
| Alp-GGGS-HisTag-to-pPK4-R | GCC TGC GGC CGC GTG ATG GGA TCC CCC CG | 44 | 11 |

The phagemid vector treated with the restriction enzyme as described above was subjected to gel electrophoresis at 100 V for 30 minutes using a 1% agarose gel including 1 × Gel green (manufactured by Fujifilm Wako Pure Chemical Corporation), extraction was then performed using FastGene Gel/PCR Extraction Kit (manufactured by Nippon Genetics Co., Ltd.), according to the attached manual to this kit, and purification was carried out. FastAP Thermosensitive Alkaline Phosphatase (manufactured by Thermo Fisher Scientific Inc.) was added to the obtained purified phagemid vector, and the mixture was reacted at 37°C for 60 minutes to perform dephosphorylation. Thereafter, a treatment was performed at 75°C for 5 minutes to deactivate the enzyme.

The VHH antibody gene fragment manufactured in the section 3-1. in the phagemid vector obtained as described above was mixed at a molar ratio of 1:5 to 10, Ligation high VeR2 (manufactured by Toyobo Co., Ltd.) was added thereto, and the mixture was reacted at 16°C overnight. The obtained phagemid vector was ethanol-precipitated and concentrated, and then the phagemid vector was electroporated to transform Escherichia coli TG-1 (competent cell for a phage display, manufactured by Lucigen). The transformed TG-1 was treated according to an ordinary method, plated on an LB agar medium, and cultured at 30°C overnight. All of the colonies appearing on the agar medium were recovered in a tube charged with a 2YTAG liquid medium, and cultured in the tube at 30°C until the O.D. 600 reached 0.5 to 1.

Thereafter, to Escherichia coli charged into the tube was added a helper phage in the amount which was 20 times that of the Escherichia coli, thereby infecting the Escherichia coli with the phage, and culture was performed at 30°C overnight. The tube that included the culture solution including the cultured Escherichia coli was centrifuged at 4,000 × g for 30 minutes at 4°C, and the supernatant was recovered in another tube. A 20% polyethylene glycol (PEG) solution containing 2.5 M of NaCl was added into the tube charged with the recovered supernatant, mixed by inversion, and then cooled on ice for 1 hour. Thereafter, the tube was centrifuged to remove the supernatant. PBS including 10% glycerol was added to the precipitate in the tube to dissolve the precipitate, thereby obtaining a phage displaying VHH.

### 3-2. Biopanning Using ELISA Plate

FGFR1-Fc or FGFR1-His was diluted with PBS to 10 µg/mL to prepare an FGFR1-immobilized solution. 100 µL of the FGFR1-immobilized solution was added to each well of a 96-well ELISA plate (Immuno Clear Standard Modules_C8_Maxisorp: cat # 445101, manufactured by Thermo Fisher Scientific Inc.) and immobilized at 4°C overnight. The same operation was performed using PBS as a negative control. After the completion of the immobilization, each well of the 96-well plate was washed three times with PBS, thereafter, 200 µL of 3% skim milk-containing PBS (hereinafter sometimes referred to as a "blocking solution") was added thereto, and the mixture was left to stand at room temperature for 1 hour to perform blocking. Next, each well was washed 3 times with PBS. 1 mL of 3% skim milk- and 5% BSA (bovine serum albumin)-containing PBS and 50 µL of the phage display solution manufactured in the section 3-1. were mixed, and then 100 µL of the mixture was added to each well and reacted at room temperature for 1 hour.

After completion of the reaction, 200 µL of PBS-T was added to each well to perform washing four times, 200 µL of PBS-T was further added thereto in the same manner, and shaking was performed for 5 minutes to wash each well. The washing operation above was repeated again. 100 µL of a 100 mM trimethylamine solution was added to each well for recovery, the same amount of a 100 mM trimethylamine solution was further added to each well, and the mixture was left to stand at room temperature for 10 minutes to elute binding phages. The eluate was recovered and immediately neutralized with 100 µL of a 0.5 M Tris-HCl buffer (pH 6.8). The neutralized eluate was recovered in a tube and mixed with 1,200 µL of Escherichia coli TG-1, and the mixture was left to stand at 30°C for 1 hour. 10 µL of the mixture solution was taken and spread on a 2YTAG agar plate (10 cm dish). The rest of the mixture solution was centrifuged at 1,450 × g for 10 minutes at 4°C and the obtained precipitate (Escherichia coli TG-1) was plated on a 2YTAG agar plate (15 cm dish).

Each of the agar plates was cultured at 30°C overnight. All of the colonies that appeared on each agar plate were recovered in a 500/mL Erlenmeyer flask including a 2YTAG liquid medium, and the flask was cultured at 30°C until the O.D. 600 reached 0.5 to 1. Thereafter, in a case where the above-described helper phage in the amount which was 20 times that of Escherichia coli was added to infect the Escherichia coli, the cells were cultured at 30°C overnight. The culture solution including the Escherichia coli after the culturing was centrifuged at 4,000 × g for 30 minutes at 4°C, and the supernatant was recovered in another tube. A 20% PEG solution containing 2.5 M NaCl in a tube including this supernatant was added thereto and mixed by inversion. Thereafter, the mixture was cooled on ice for 1 hour and then the tube was centrifuged at 4,000 × g for 30 minutes at 4°C to remove the supernatant. PBS including 10% glycerol was added to the obtained precipitate to dissolve the Escherichia coli, thereby creating a phage display, and biopanning was performed again.

### 3-3. Biopanning Using Immunotube

FGFR1-Fc was diluted with PBS to 10 µg/mL to prepare an FGFR1-immobilized solution, 1 mL of the solution was added into an immunotube (MAXISORP NUNC-IMMUNO TUBE 5.0 mL: cat # 444202, manufactured by Thermo Fisher Scientific Inc.), and the mixture was immobilized overnight at 4°C. The tube was washed 3 times with PBS, and then 5 mL of the blocking solution was added into the tube, which was left to stand at room temperature for 1 hour to perform blocking. Thereafter, the tube was washed 5 times with PBS. 100 µL of the phage display solution manufactured in the section 3-1 was mixed with PBS including 3/mL of PBS, 3% skim milk, 5% BSA, and 5% human serum albumin (HSA), and then the mixture was added into the immunotube and reacted at room temperature for 1 hour.

5 mL of PBS-T was added to the mixture to perform washing 10 times, and 5/mL of PBS-T was further added thereto to perform washing by shaking at room temperature for 30 minutes. Thereafter, 5 mL of PBS-T was further added thereto to perform washing 10 times. 1/mL of a 100 mM trimethylamine solution was added into the immunotube for recovery, 1/mL of a 100 mM trimethylamine solution was further added into the immunotube, and the mixture was left to stand at room temperature for 10 minutes to elute bound phages for recovery. The recovered eluate was immediately neutralized with 1/mL of a 0.5 M Tris-HCl buffer (pH 6.8). The neutralized eluate was mixed with 10 mL of Escherichia coli TG-1 and the mixture was left to stand at 30°C for 1 hour. Thereafter, this mixture solution was centrifuged at 1,450 × g for 10 minutes at 4°C, and the precipitate (Escherichia coli TG-1) was plated on a 2YTAG agar plate (15 cm dish). Each agar plate was cultured at 30°C overnight.

All of the colonies that appeared on the agar plate were recovered in a liquid medium and cultured at 30°C until the O.D. 600 reached 0.5 to 1. Thereafter, in a case where a helper phage in the amount which was 20 times that of Escherichia coli was added to infect the Escherichia coli, the cells were cultured at 30°C overnight. The culture solution including the cultured Escherichia coli was centrifuged at 4,000 × g for 30 minutes at 4°C, and the supernatant was recovered in a new tube. A 20% PEG solution containing 2.5 M of NaCl was added to the recovered supernatant, mixed by inversion, and then cooled on ice for 1 hour. Thereafter, the tube was centrifuged at 4,000 × g for 30 minutes at 4°C to remove the supernatant. PBS including 10% glycerol was added to the obtained precipitate to dissolve the precipitate, thereby preparing a phage display solution, and biopanning was performed again. The second panning was performed in the same manner as in the first panning, except that FGFR1-His was immobilized on the immunotube instead of FGFR1-Fc.

### 3-4. Phage ELISA

In order to confirm whether or not the library was concentrated for VHH bound to FGFR1, phage ELISA was performed in a polyclonal state.

FGFR1-Fc and FGFR1-His were diluted with PBS to 10 µg/mL to prepare an FGFR1-immobilized solution. 100 µL of the FGFR1-immobilized solution was added to each well of a 96-well ELISA plate and immobilized at 4°C overnight. Each well was washed with PBS three times, then 200 µL of the blocking solution was added thereto, and the mixture was left to stand at room temperature for 1 hour to perform blocking. Thereafter, each well of the plate was washed three times with PBS. 160 µL of each round of the phage display was mixed with 160 µL of 10% BSA-containing PBS, and 50 µL of this mixture solution was added to each well and reacted at room temperature for 1 hour. Thereafter, each well was washed 5 times with 200 µL of PBS-T. Anti-M13-mAb-HRP (manufactured by Sino Biological, Inc.) was diluted 3,000-fold with PBS-T, 50 µL of this diluted solution was added to each well, and the reaction was performed at room temperature for 1 hour.

Subsequently, each well was washed 5 times with 200 µL of PBS-T. Thereafter, an OPD tablet (cat # 155-02161, manufactured by Fujifilm Wako Pure Chemical Corporation) was dissolved in 10 mL of a 0.1 M aqueous NaH₂PO₄ solution to prepare an OPD solution, 100 µL of each of the OPD solution was added to each well, and incubation was performed for 15 minutes. Then, 100 µL of 1 M sulfuric acid was added thereto, and immediately the absorbance in each well was measured at a measurement wavelength of 490 nm using Microplate Reader Infinite 200Pro M PLEX (manufactured by Tecan Group, Ltd.). The VHH-displaying phage that had been subjected to biopanning in the section 3-2 was taken into a new tube and mixed with Escherichia coli TG-1, and the mixture was left to stand at 30°C for 1 hour.

The tube including the mixture solution was centrifuged at 4,000 × g for 10 minutes at 4°C, and the precipitate (Escherichia coli TG-1) was dissolved in a 2YTAG liquid medium, which was then seeded on a 2YTAG agar plate (15 cm dish). This agar plate was cultured at 30°C overnight, appearing colonies were subjected to single picking according to an ordinary method and cloned to recover each phage display. Phage ELISA was performed in the same manner, but a solution in which FGFR1-His was diluted with PBS to 2 µg/mL was used for an ELISA plate.

Thereafter, for clones from which a response was obtained, a phagemid was extracted from Escherichia coli, and DNA sequence analysis of a portion corresponding to the displayed VHH was performed using a Sanger method. The DNA sequence analysis was outsourced to Eurofin Genomics Co., Ltd.

### (Example 4) Production of VHH (Monomer)

VHH Clone #1 and VHH Clone #2 identified in the section 3-4. of Example 3 were subjected to expression and purification of VHH (monomer) using Corynebacterium glutamicum. The experimental procedure was as described below.

### 1. Plasmid Construction for Hit VHH (Monomer) Expression

Using the phagemids of VHH Clone #1 and VHH Clone #2 as templates and using a primer specific to each VHH, PCR was performed under the conditions of an annealing temperature of 55°C and an extension reaction of 5 seconds, and purification was performed according to a manual attached to this kit, using Gel/PCR Extraction Kit (manufactured by NIPPON Genetics Co, Ltd.).

The above-described purified DNA, a plasmid for expressing C. glutamicum, and an Escherichia coli extraction SLiCE reaction solution were added into a tube charged with a SLiCE buffer (50 mM Tris-HCl buffer (pH 7.5), 10 mM MgCl₂, 1 mM ATP, 1 mM DTT (1,4-dithiothreitol)), in vitro homologous recombination was carried out at 37°C for 15 minutes, and Competent Cell JM109 (manufactured by Takara Bio Inc.) was transformed to obtain a transformant. A plasmid was extracted from the obtained transformant, and according to the DNA sequence analysis, it was confirmed that the genes of VHH Clone #1 and VHH Clone #2 were each incorporated.

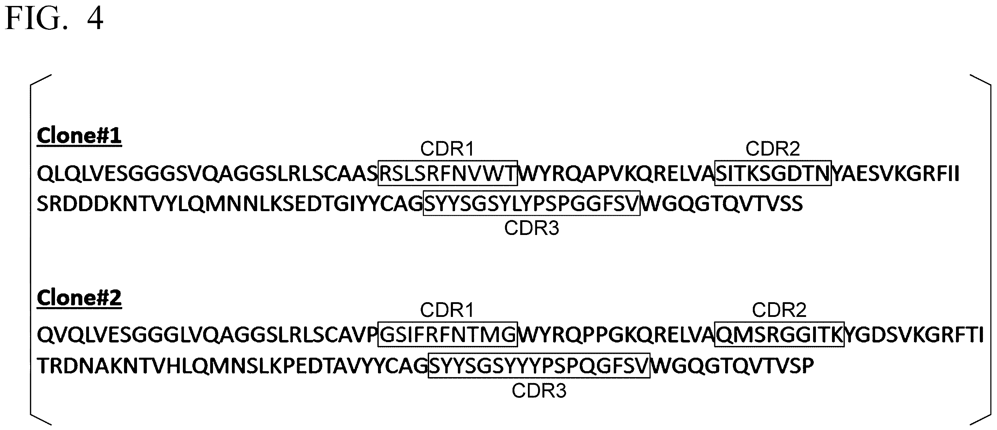

The amino acid sequences of CDR regions of VHH Clone #1 (Clone #1) and VHH Clone #2 (Clone #2) are shown in FIG. 4.

### 2. Expression of VHH (Monomer) Using C. glutamicum

Each of the plasmids obtained in the section 1. was introduced into C. glutamicum by electroporation, and transformation was performed to obtain a transformant. The obtained transformant was inoculated into a CM2G medium and pre-cultured at 30°C overnight. Thereafter, the pre-culture solution was passaged to a PM1S medium in a 96-deep well plate and cultured at 25°C for 72 hours, and VHH (monomer) was secreted into the culture supernatant. After completion of the culture, the 96-deep well plate was centrifuged at 4,000 × g for 30 minutes at 20°C. After the centrifugation was completed, the centrifugal supernatant was recovered and passed through a 0.22 µm filter to remove bacterial cells in the supernatant. The culture supernatant after removal of the bacterial cells was freeze-preserved at -80°C, appropriately thawed as necessary, and used for purification.

### 3. Purification of VHH (Monomer)

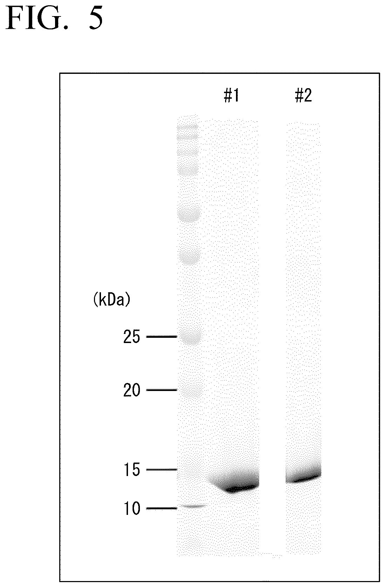

After imidazole was added to the thawed culture supernatant such that the final concentration was 10 mM, and 100 µL of Ni Sepharose 6 Fast Flow (manufactured by Cytiva, hereinafter referred to as a "carrier") was mixed therewith and mixed by inversion at 4°C for 1 hour. Centrifugation was performed at 500 × g for 1 minute at 4°C to precipitate the carrier, and the supernatant was removed such that the final solution amount was approximately 700 µL to obtain a suspension. This suspension was transferred to a spin column (EconoSpin; manufactured by Ajinomoto Bio-Pharma) and centrifuged at 100 × g for 30 seconds at 4°C. 600 µL of a washing buffer (50 mM Tris-HCl buffer (pH 7.5) including 300 mM NaCl and 30 mM imidazole) was added thereto to perform centrifugation twice under the same conditions as above. Thereafter, 700 µL of an elution buffer (a 50 mM Tris-HCl buffer (pH 7.5) including 300 mM NaCl and 500 mM imidazole) was added thereto to perform VHH elution. The purity of the eluted clone VHHs (monomer) #1 and #2 was confirmed by SDS-PAGE (FIG. 5). In FIG. 5, the marker represents the molecular weight. Each of the clone VHHs (monomers) #1 and #2 was detected near 15 kDa.

In order to remove a high concentration of imidazole, the buffer was exchanged with PBS using a desalting column (Zeba Spin Desalting Columns; manufactured by Thermo Fisher Scientific Inc.). The concentration of VHH (monomer) was quantified by a BCA method using Pierce BCA Protein Assay Kit (manufactured by Thermo Fisher Scientific Inc.) using bovine serum albumin (manufactured by Fujifilm Wako Pure Chemical Corporation) as a standard protein.

### (Example 5) Affinity Measurement of VHH (Monomer)

Affinity measurement of VHH (monomer) was performed by a surface plasmon resonance (SPR) method.

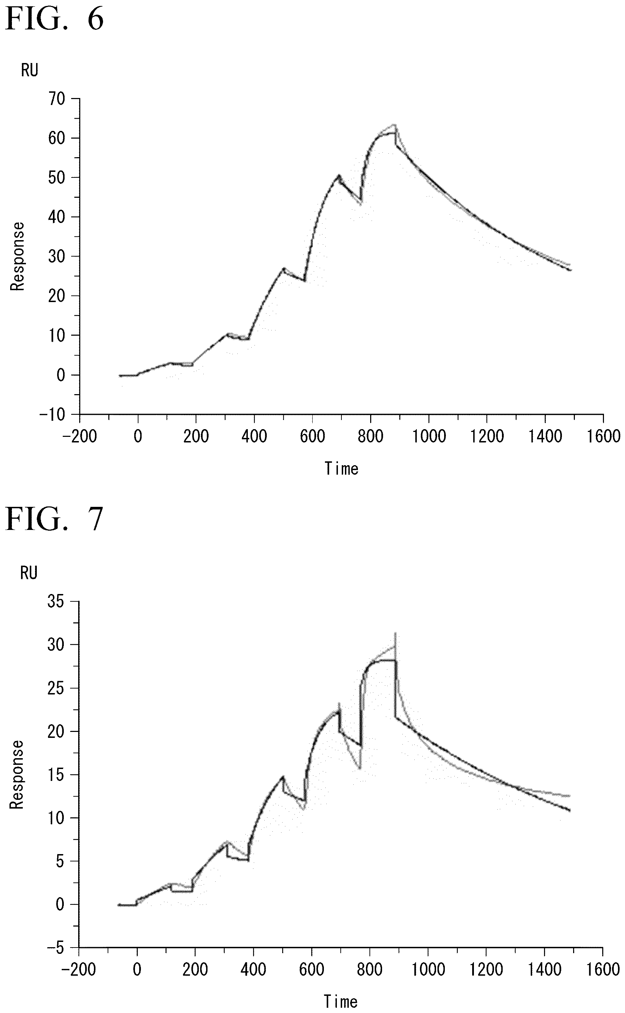

The binding activity of VHH Clone #1 or VHH Clone #2 immobilized on Series S Sensor Chip CAP (manufactured by Cytiva) with respect to FGFR1-Fc was measured using Biacore (registered trademark) T200 (manufactured by Cytiva). Measurement was performed by CAP Single-cycle Kinetics. The temperature was set to 25°C. As a running buffer, 10 mM HEPES (pH 7.4) (Cytiva) including HBS-EP + (150 mM NaCl, 0.5 mM EDTA, and 0.05% surfactant P20) was used. The measurement order for each run is as follows.
1) Immobilization of biotinylated FGFR1-Fc: The flow rate was set to 2 µL/mL, Biotin CAPture Reagent (Cytiva) was added thereto for 300 seconds, then the flow rate was set to 10 µL/mL,and an FGFR1-Fc solution diluted with a running buffer was added thereto for 120 seconds to immobilize 140 RU. Here, the "resonance unit (RU)" is a unit used in the above-described Biacore, and a change of an SPR angle of 0.1° is defined as 1,000 RU.
2) Measurement of binding activity: The flow rate was set to 30 µL/mL,and VHH Clone #1 or VHH Clone #2 each diluted to 1.85 nM, 5.56 nM, 16.67 nM, or 50 nM, using a running buffer, was allowed to have an association time set to 120 seconds for binding and a dissociation time set to 600 seconds for interaction.
3) Regeneration of sensor chip surface: The flow rate was set to 10 µL/mL,a solution in which Reaneration stock 1 and Regeneration stock 2 (manufactured by Cytiva) were mixed at the ratio of 3:1 was added thereto for 120 seconds to extract the immobilized VHH. Next, analysis was performed with a 1:1 binding model using Biacore T200 Evaluation (software version 2.0, manufactured by Cytiva), and the binding activity was calculated. The results are shown in FIG. 6 and FIG. 7.

As a result, the equilibrium dissociation constant (KD) of VHH Clone #1 for FGFR1-Fc was 1.74 × 10⁻⁹ M (ka = 8.19 × 10⁶ (1/Ms), kd = 1.42 × 10⁻³ (1/s)). In addition, the equilibrium dissociation constant (KD) of VHH Clone #2 with respect to FGFR1 was 1.02 × 10⁻⁹ M (ka = 1.11 × 10⁶ (1/Ms), kd = 1.14 × 10⁻³ (1/s)).

### (Example 6) Measurement of Affinity of VHH (Monomer) for Each Receptor

The binding activity of VHH Clone #1 to FGFR2-Fc, FGFR3-Fc, or FGFR4-Fc was measured in the same manner as in Example 5.

As a result of the analysis, it was found that the equilibrium dissociation constants (KD) of VHH Clone #1 for FGFR2-Fc, FGFR3-Fc, and FGFR4-Fc were each 1.63 × 10⁻⁶ M (ka = 4.84 × 10⁵ (1/Ms) and kd = 7.76 × 10⁻¹ (1/s)), 1.01 × 10⁻⁶ M (ka = 2.32 × 10⁵ (1/Ms), kd = 2.35 × 10⁻¹ (1/s)), 2.22 × 10⁻⁸ M (ka = 2.37 × 10⁶ (1/Ms), kd = 5.27 × 10⁻² (1/s)).

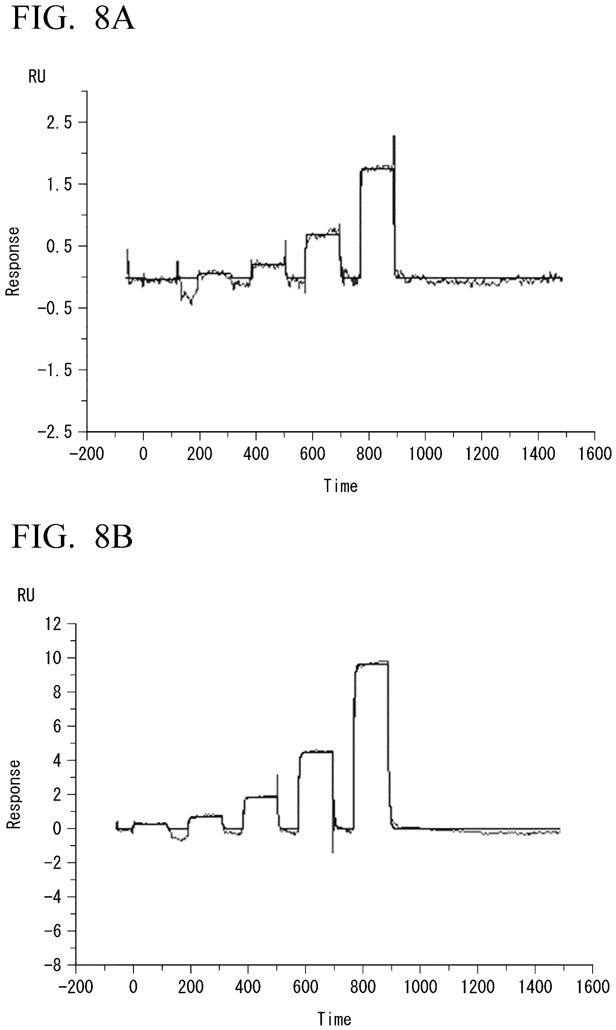

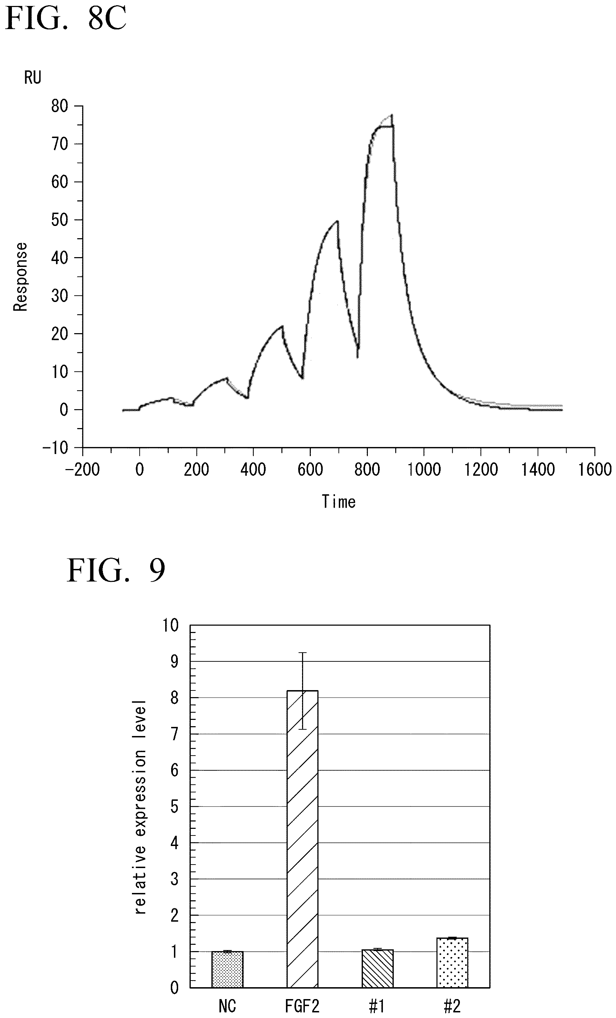

The measurement results of the affinity of VHH Clone #1 for FGFR2-Fc, FGFR3-Fc, and FGFR4-Fc are shown in FIGS. 8A to 8C in the order of vs. FGFR2-Fc (FIG. 8A), vs. FGFR3-Fc (FIG. 8B), and vs. FGFR4-Fc (FIG. 8C). The vertical axis of the graph indicates an affinity (response) and the horizontal axis indicates a time (seconds).

### (Example 7) Measurement of Agonist Activity of VHH (Monomer)

### 1. Sample and Others

In order to verify the agonist activity of the purified VHH (monomer), the expression level of phospho-p44/42 MAPK (Erk1/2) was changed by using an In Cell ELISA method. VHH (monomer) was allowed to act on the cells such that the final dilution ratio was 100-fold. In addition, as a positive control, FGF2 (fibroblast growth factor (basic) (FGF-basic/bFGF/FGF2), human, recombinant, animal-derived substance-free (154aa); product code 068-05384; manufactured by Fujifilm Wako Pure Chemical Corporation; final concentration: 100 ng/mL) was used. The experimental procedure was as described below.

### 2. Stimulation of NIH3T3 Cells by FGF2 or VHH (Monomer)

A D-MEM (D6429, manufactured by Sigma-Aldrich Co., LLC) medium including a 10% fetal bovine serum (S0400, manufactured by BWT) and 1% Penicillin-Streptomycin Mixed Solution, 26253-84, manufactured by Nacalai Tesque, Inc.) was prepared, NIH3T3 (CRL1658, ATCC) was suspended therein, and the suspension was seeded on a 96-well plate for cell culture at 1 × 10⁴ cells/well and cultured overnight at 37°C with light shielded in a 5% CO₂ incubator. The medium in each well was removed and the inside of each well was washed with a serum-free medium. Thereafter, a serum-free medium obtained by removing the serum from the medium was prepared and allowed to act on the cells so that the final dilution ratio of VHH (monomer) was 100-fold uniformly, and NIH3T3 cells were stimulated by performing normal culture at 37°C for 30 minutes with light shielded in a 5% CO₂ incubator. Furthermore, FGF2 (final concentration: 100 ng/mL) was used as a positive control.

### 3. In Cell ELISA

The medium was removed from each well, the cells stimulated with FGF2 or VHH (monomer) obtained in the section 2. were washed twice with PBS, then a 4% paraformaldehyde-PBS solution (hereinafter sometimes referred to as a "fixation solution") was added to each well at 100 µL/well, and the cells were fixed at room temperature for 15 minutes. After fixing the cells, the fixation solution was removed from each well, and PBS was added to each well and washed three times. Next, PBS containing 0.1% Triton X-100 was added to each well at 100 µL/well and a cell permeation treatment was performed at room temperature for 15 minutes. After this treatment, each well was washed with PBS again, 1% H₂O₂-containing PBS was added to each well at 100 µL/well, and the mixture was left to stand at room temperature for 20 minutes to perform an inactivation treatment. Subsequently, 1% of H₂O₂-containing PBS was removed from each well, each well was washed with PBS, then 1% of BSA-containing TBS was added to each well at 100 µL/well, the mixture was left to stand at room temperature for 1 hour, and blocking was performed.

An Anti-Phospho-p44/42 MAPK (Erk1/2) rabbit monoclonal antibody (#4370, manufactured by CST) was diluted 1,000-fold with 1% BSA-containing TBS to obtain an antibody diluted solution 1, and the solution was added to each well of the plate after blocking at 100 µL/well and reacted at 4°C overnight. The liquid in the well was removed, and an operation of adding 0.05% Tween20-containing TBS (hereinafter sometimes referred to as a "TBS-T") to each well at 100 µL/well and washing the inside of the well was repeated three times.

Anti-rabbit IgG, HRP-linked Antibody (manufactured by CST, #7074) was diluted 1,000-fold with 1% BSA-containing TBS to prepare an antibody diluted solution 2, and the solution was added to each well at 100 µL/well and reacted at room temperature for 1 hour with light shielded. As in the case of using the antibody diluted solution 1, TBS-T was added to each well at 100 µL/well to wash three times, and then an OPD detection reagent mix was added to each well at 100 µL/well. The OPD detection reagent mix is a solution in which 10PD tablet (Fujifilm Wako Pure Chemical Corporation, #154-06173) is dissolved in 12 mL of a 0.1 M NaHPO₄ (pH 5.5) solution, and H₂O₂ is added thereto such that the final concentration was 0.025%. Next, the reaction was performed with light shielded at room temperature for 15 minutes, then 1 M sulfuric acid was added to each well at 100 µL/well to stop the reaction, and the absorbance of each well was measured with a plate reader (measurement wavelength: 490 nm, ref: 630 nm).

### 4. Janus Green Staining

Cell staining with Janus Green was performed in order to correct the number of cells. The absorbance was measured in the section 3., then each well of the plate was washed three times with ultrapure water, 50 µL of a Janus Green solution (ab 111622, manufactured by Abcam) was added to each well, and the cells were left at room temperature for 5 minutes to stain the cells. Thereafter, each well was washed 5 or 6 times with ultrapure water, 0.1 M hydrochloric acid was added thereto at 100 µL/well to lyse the cells, and the cells were reacted at room temperature for 10 minutes. Thereafter, the absorbance at 595 nm was measured with a plate reader.

A graph showing the expression level of phospho-p44/42 MAPK (Erk1/2) by FGF2 or VHH (monomer) stimulation is shown in FIG. 9. The vertical axis in FIG. 9 indicates relative expression levels of FGF2, VHH Clone #1, and VHH Clone #2 in a case where the expression level of a negative control (without stimulation by FGF2 or VHH (monomer)) was set to 1. From FIG. 9, it has been revealed that none of the VHHs (monomers) have an agonist activity.

### (Example 8) Production of VHH (Homodimer)

### 1. Construction of Plasmid for Expressing VHH (Homodimer)

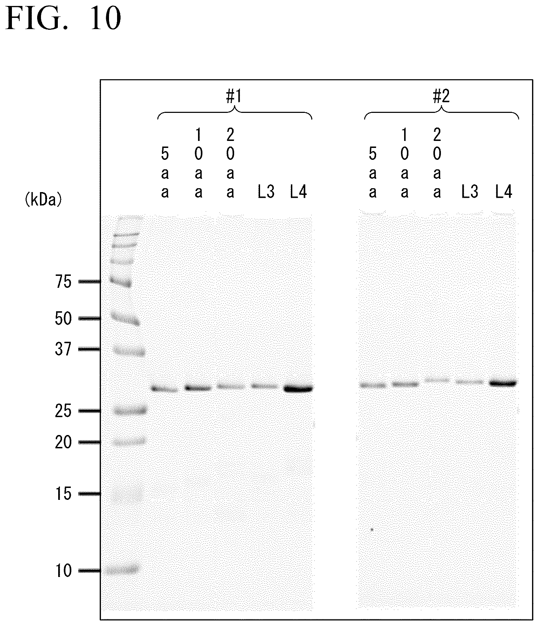

In Example 7, it was confirmed that VHH (monomer) has no agonist activity by measuring the agonist activity of VHH (monomer). Therefore, in order to add the agonist activity to VHH Clone #1 and VHH Clone #2, the homodimerization of VHH was examined using various linkers. Details of the amino acid sequence of each of the linkers examined in the present Examples are shown in Table 8. In FIG. 10, the linkers used were described under names in Table 8.

**[Table 8]**

| Amino acid sequence of linker used for VHH multimerization | | |
|---|---|---|
| Name | Amino acid sequence (N-terminal → C-terminal) | SEQ ID NO: |
| 5aa | GSGGG | 12 |
| 10aa | GSGGGGSGGG | 13 |
| 20aa | GSGGGGSGGGGSGGGGSGGG | 14 |
| L3 | GSAGSAAGSGEF | 15 |
| L4 | GSEGKSSGSGSESKST | 16 |

PCR was performed using the plasmid obtained in the section 1. of Example 4 as a template and using a primer specific to each VHH under the conditions of an annealing temperature of 55°C and an extension reaction of 5 seconds, and a linker sequence and a restriction enzyme recognition sequence of each clone were added to the N-terminal side of each clone. The obtained PCR product was purified using a Gel/PCR Extraction Kit (manufactured by Nippon Genetics Co., Ltd.) according to the attached manual to obtain a purified PCR product.

The plasmid obtained in Example 4 was subjected to a restriction enzyme treatment using FastDigest BamHI and ApaI (Thermo Fisher Scientific Inc.), and purified using a Gel/PCR Extraction Kit in the same manner as described above to obtain a purified DNA. FastDigest BglII and ApaI (manufactured by Thermo Fisher Scientific Inc.) were added to this purified DNA, the mixture was reacted at 37°C for 1 hour, and then each DNA was purified using a Gel/PCR Extraction Kit in the same manner as described above to obtain a restriction enzyme-treated plasmid.

The restriction enzyme-treated plasmid was subjected to electrophoresis at 100 V for 30 minutes using a 1% agarose gel containing 1 × Gel green, and then purification was performed using FastGene Gel/PCR Extraction Kit according to the attached manual to obtain purified DNAs.

Ligation high VeR2 was added to each of the purified DNAs obtained by the above-described procedure, and the DNAs were ligated at 16°C for 30 minutes to transform Competent Cell JM109. Using FastGene Plasmid Mini Kit (Nippon Genetics Co., Ltd.), plasmids were extracted and purified from the obtained transformant according to the manual attached to this kit. According to DNA sequence analysis of the obtained plasmid, it was confirmed that a gene of a homodimer using each linker was incorporated.

### 2. Introduction of Vector into C. glutamicum and Purification of VHH

### (Homodimer)

Each of the plasmids manufactured as above was introduced into C. glutamicum by electroporation, the expression of VHH (homodimer) was confirmed, and purification was performed. The experimental procedure was the same as that in "2. Expression of VHH (Monomer) Using C. glutamicum" and "3. Purification of VHH (Monomer)" in Example 4.

An electrophoresis image of SDS-PAGE of VHH (homodimer) is shown in FIG. 10. In FIG. 10, #1 and #2 represent each of the VHH clones, and 5aa, 10aa, and 20aa, L3, and L4 indicate the linkers used herein by the names of the linkers described in Table 8. The numbers 10 to 75 represent molecular weights and markers.

### (Example 9) Measurement of Agonist Activity of VHH (Homodimer)

The agonist activity of the purified VHH (homodimer) was verified using an In Cell ELISA method, based on a change in expression level of phospho-p44/42 MAPK (Erk1/2). The homodimer (VHH) was allowed to act on the cells such that the final dilution ratio was 100-fold uniformly. Furthermore, FGF2 (final concentration: 100 ng/mL) was used as a positive control. The experimental procedure was the same as in the section 3. in Example 7.

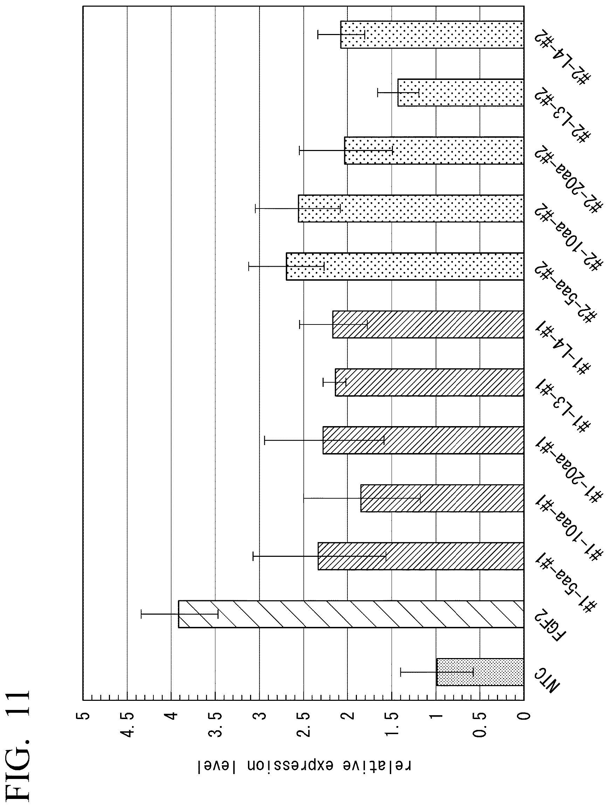

The expression level of phospho-p44/42 MAPK (Erk1/2) stimulated by FGF2 or the homodimer (VHH) is shown in FIG. 11, assuming that the relative expression level in a case of using unstimulated cells is 1. In FIG. 11, NTC indicates cells without stimulation (negative control). A combination of a homodimer linked with a linker and the linker are indicated by #1-5aa-#1 in a case where the linked clone is VHH Clone #1 and the linker is 5aa. The relative expression level of homodimer in which Clone VHH #1 or Clone #2 was linked with the linker shown in Table 8 is shown in Table 11. From the above, it was revealed that the VHH (homodimer) has an agonist activity.

### (Example 10) Measurement of Cell growth Activity of VHH (Monomer, Homodimer)

### 1. Measurement of Cell growth Activity by NIH3T3 (Mouse Fibroblast Cell Line)

As a cell for measuring a cell growth activity, NIH3T3 (mouse fibroblast cell line) was obtained from ECACC through a domestic agent. The cell which was subjected to extended culture according to an ordinary method, and then freeze-preserved at -80°C using CellBankeR1 plus (manufactured by Xenoac Co., Ltd.) was used. For the maintenance culture, D-MEM (high glucose: manufactured by Fujifilm Wako Pure Chemical Corporation, hereinafter simply referred to as "DMEM") containing a 10% bovine serum (New Zealand origin: manufactured by Thermo Fisher Scientific, hereinafter referred to as "CS") and 100U penicillin-streptomycin (manufactured by Thermo Fisher Scientific, Inc., hereinafter referred to as "PS (+)") was used. The passage operation was performed using Trypsin-EDTA (manufactured by Thermo Fisher Scientific Inc.) and D-PBS (-) according to an ordinary method under conditions presented by the distribution source. For the cell growth assay, cells with 3 to 10 passages were used.

The cell growth assay for FGF2 was performed according to the following procedure. On Day 1, a suspension including 2.5 × 10⁴ cells/mL of NIH3T3 in DMEM of 10% CS and PS(+) was created, seeded on a CulturPlate-96 (White Opaque 96-well Microplate, manufactured by Perkin Elmer, Cat# 6005680) at 100 µL/well, and cultured in a 5% CO₂ incubator at 37°C for 16 to 24 hours. On Day 2, the medium was replaced with FGF2 (PeproTech Cat# AF-100-18B) or serum-free PS(+) DMEM including various test substances, and the cells were further cultured for 2 days. On Day 4, the 96-well plate was left at room temperature for 30 minutes, and then 100 µL of Cell Titer Glo 2.0 Reagent (manufactured by Promega Corporation) at room temperature was added to each well, followed by stirring at 500 rpm for 2 minutes using a plate mixer. The plate was further left to stand for 8 minutes, and then the luminescence in each well was measured with a Synergy HTX plate reader (manufactured by BioTek) with a sensitivity set (Gain) of 200 and a measurement time of 1 s/well.

The measurement was performed at three points at each concentration of the test substance, for the measurement at a single concentration, 100 ng/mL of FGF2 was used as a positive control and a condition of no addition was used as a negative control, and the VHH monomer was prepared with uniform 100-fold dilution, and measured. In addition, in a case of measuring EC₅₀, a 4-fold dilution series starting from 1,000 ng/mL or 100 ng/mL (FGF2) was created for 8 concentrations and subjected to the measurement. EC₅₀ measurement data were obtained according to an ordinary method using a control software attached to a plate reader. The EC₅₀ value was calculated by applying the 4-parameter fit.

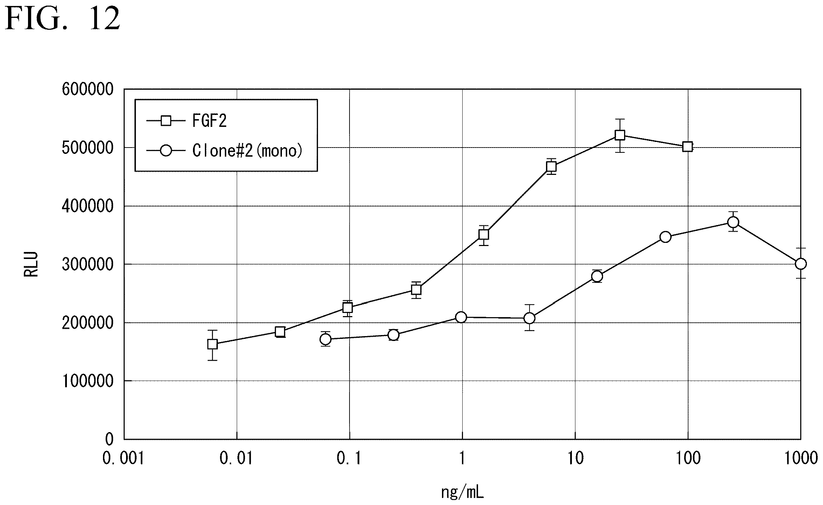

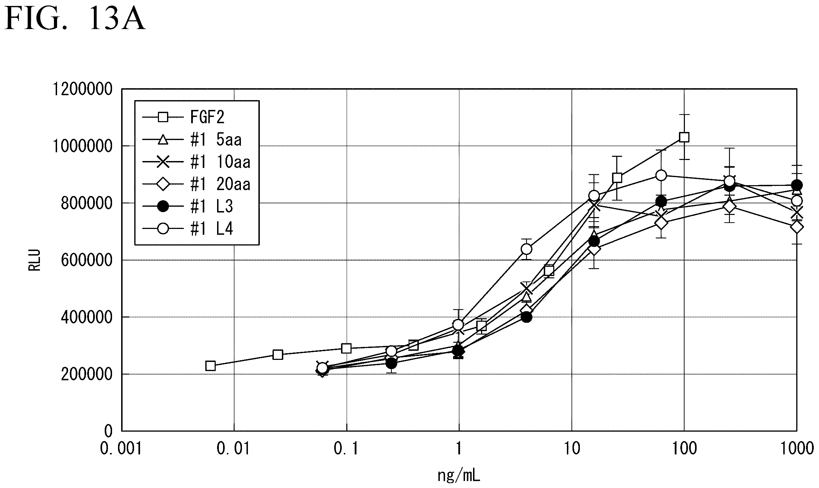

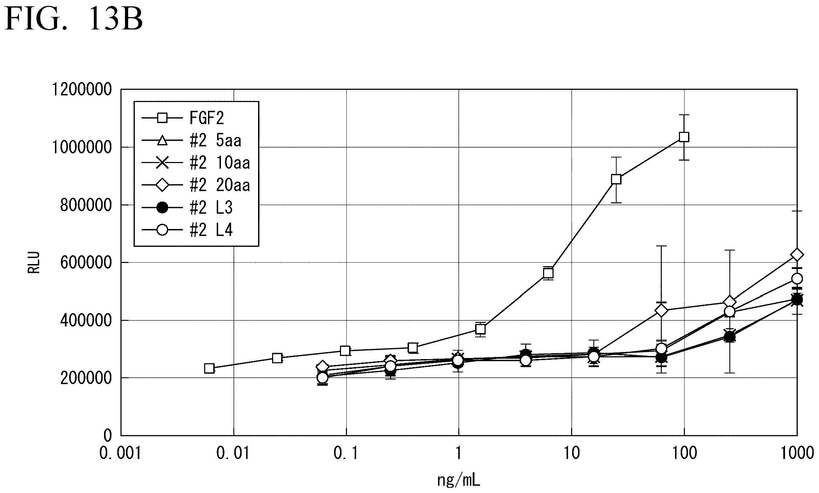

A graph showing the EC₅₀ measurement results of the VHH monomer (Clone #2) is shown in FIG. 12. RLU shown on the vertical axis of the graphs shown in FIGS. 12, 13A, and 13B all indicate the relative luminescence amount. FIG. 12 shows the concentration of the monomer of FGF2 or Clone #2. It was shown that in the monomer of Clone #2, the relative luminescence amount was low even at a high concentration, as compared with the case where FGF2 was used.

A graph showing the measurement results of EC₅₀ of the VHH homodimer (Clone #1) is shown in FIG. 13A. It was confirmed that all of the homodimers of Clone #1 used herein have an activity comparable to that of FGF2.

A graph showing the measurement results of EC₅₀ of the VHH homodimer (Clone #2) is shown in FIG. 13B. It was confirmed that all of Clone #2 homodimers used herein had a considerably lower activity than FGF2.

From the above, it was shown that the VHH homodimer (Clone #1) has EC₅₀ similar to that of FGF2.

### 2. Inhibition of Growth Factor Activity of VHH Homodimer by FGFR Antagonist

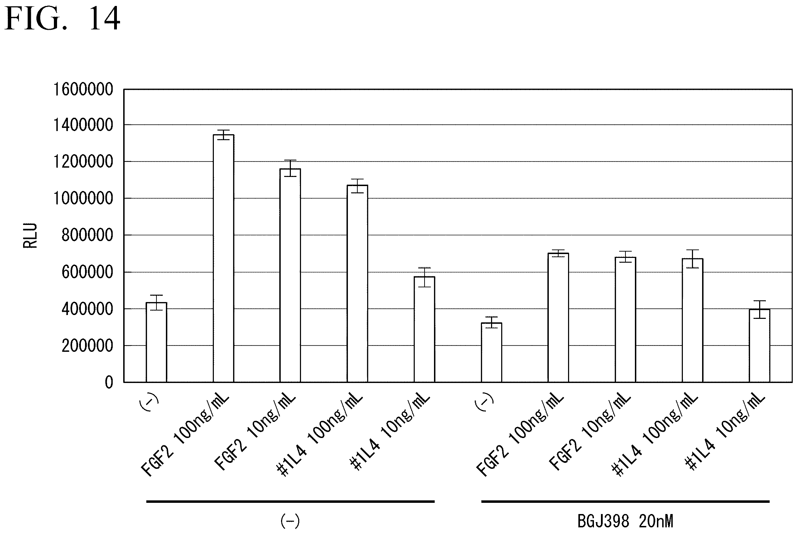

The growth suppression of the VHH homodimer by FGFR kinase inhibitor was examined, and it was checked whether or not the VHH homodimer exhibited a cell growth activity through FGFR. As the kinase inhibitor, NVP-BGJ398 (IC₅₀ value against FGFR1/2/3: 0.9/1.4/1.0 nM, manufactured by ChemScene) exhibiting a selective inhibitory activity against FGFR was used. The same operation as in the cell growth assay of FGF2 was performed separately into a group without addition of NVP-BGJ398 (represented by (-) in FIG. 14) and a group with 20 nM of NVP-BGJ398 added (represented by 20 nM of BGJ398 in FIG. 14). The addition amounts of FGF2 and #1L4 (hereinafter also referred to as "#1-L4-#1"), which is a dimer of VHH #1 with a linker L4, were each 10 or 100 ng/mL. In addition, it was separately confirmed that 20 nM of NVP-BGJ398 itself had little effect on cell growth.

The vertical axis in FIG. 14 indicates a relative luminescence amount. As shown in FIG. 14, the addition of NPV-BGJ398 inhibited the cell growth activities of FGF2 and #1L4. From this, it was confirmed that #1L4 has an FGFR-mediated cell growth activity as in the case of FGF2.

### 3. Measurement of Cell growth Activity of VHH Monomer

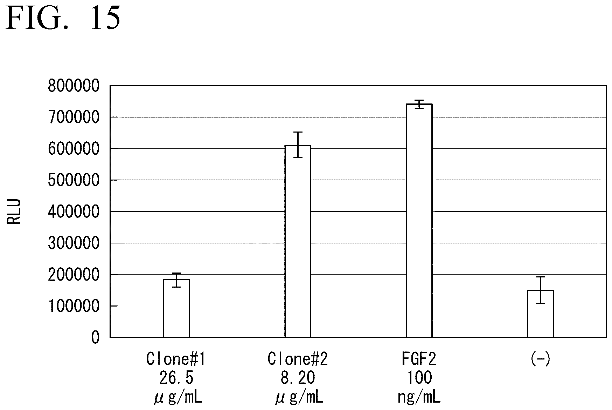

Each stock of the VHH monomer (Clone #1: 2.65 mg/mL; Clone #2: 0.82 mg/mL-PBS) was diluted 100-fold to check the presence or absence of the cell growth activity. A sample without addition of FGF2 or Clone #1 was used as a negative control, and FGF2 (100 ng/mL) was used as a positive control. The VHH monomer 1 was used at concentrations of 26.5 µg/mL and 8.20 µg/mL. The results are shown in FIG. 15. The vertical axis in FIG. 15 indicates a relative luminescence amount. The amount of luminescence was measured in the same manner as that for the product described in Example 10. As shown in FIG. 15, VHH Clone #2 significantly exhibited a cell growth activity in the monomer.

### (Example 11) Measurement of Thermal Stability of VHH (Monomer)

The thermal stability of VHH was measured by a protein thermal shift assay method.

In order to confirm the thermal stability of the VHH monomer Clone #1 and the tandem dimer #1L4, the denaturation temperature (Td) was measured by a protein thermal shift assay method. The experimental operation was carried out according to Huynh et al. (Huynh, Kathy, Partch, Carrie L., Analysis of protein stability and ligand interactions by thermal shift assay, Current Protocols in Protein Science, 2015, Vol. 79, p. 28.9.1-28.9.14). Specifically, a D-PBS solution (Fujifilm Wako Pure Chemical Corporation, Ltd.) including 0.5 mg/mL of the VHH monomer Clone #1 or the tandem dimer #1L4 and 20 µL of a 10-fold concentration of SYPRO Orange (manufactured by Thermo Fisher Scientific, Inc.) was used. In addition, in order to measure the fluorescence background, a control including no protein was also prepared at the same time, and dispensed into an 8-strip PCR tube (manufactured by Nippon Genetics Co., Ltd.). This tube was set in a real-time PCR measuring device, QuantStudio 3 (manufactured by Thermo Fisher Scientific, Inc.), and a change in fluorescence intensity in a range of 25°C to 95°C was measured under the condition of 1 °C/min using a fluorescence filter for ROX measurement. After the measurement, Td was calculated using the value obtained by subtracting the background value from the measured value.

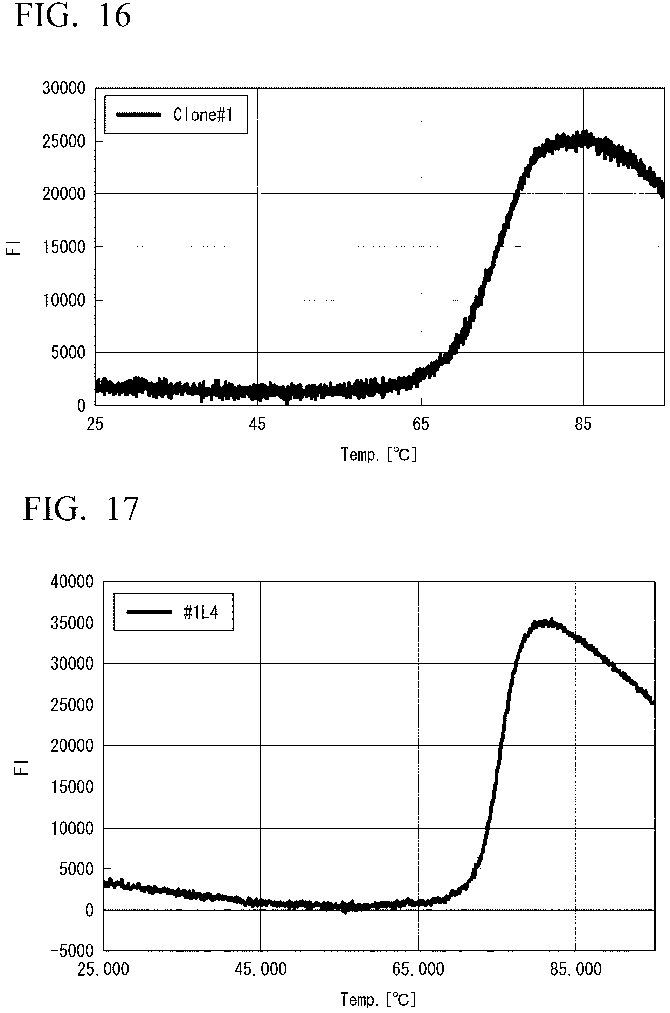

The thermal stability of the VHH monomer Clone #1 and the tandem dimer #1L4 was measured by a protein thermal shift assay. The results are shown in FIGS. 16 and 17. In FIGS. 16 and 17, the vertical axis indicates a fluorescence intensity, and the horizontal axis indicates a measured temperature range. As shown in FIGS. 16 and 17, all the VHHs exhibited high thermostability, the Td of VHH Clone #1 was 74.1°C, and the Td of #1L4 was 75.4°C.

### (Example 12) Production of Coiled Coil Body (VHH)

For VHH Clone #1 obtained in Example 4, VHH (coiled coil body) (hereinafter sometimes referred to as "VHH-C") as follows using Corynebacterium glutamicum (C. glutamicum) was expressed and purified.

### 1. Construction of Plasmid for Expressing VHH-C

First, a coiled coil sequence was added to a plasmid for expressing VHH Clone #1 as follows. A DNA of the coiled coil was obtained by artificial gene synthesis (Eurofin Genomics Co., Ltd.). FastDigest BamHI and FastDigest ApaI (both restriction enzymes manufactured by Thermo Fisher Scientific, Inc.) were added to the plasmid for expressing VHH Clone #1 and the artificial gene DNA, and the mixture was reacted at 37°C for 30 minutes. Next, the enzyme reaction solution was loaded into a 1% agarose gel including 1 × Gel green (Fujifilm Wako Pure Chemical Corporation) and subjected to electrophoresis at 100 V for 30 minutes. Thereafter, using FastGene Gel/PCR Extraction Kit (Nippon Genetics Co., Ltd.), extraction was performed from the gel according to the attached instructions, and further purification was performed.

Next, the plasmid and the coiled coil DNA were mixed at 1:3 (molar ratio), Ligation High Ver. 2 (manufactured by TOYOBO Co., Ltd.) was added thereto, and the mixture was reacted at 16°C for 30 minutes to obtain a plasmid for expressing VHH-C. Escherichia coli JM-109 (manufactured by Takara Bio Inc.) was transformed with the obtained plasmid for expressing VHH-C. The transformed Escherichia coli JM109 was seeded on an agar medium plate and cultured at 37°C overnight.

The colonies that appeared on the agar medium were picked, and cultured overnight at 37°C, and using FastGene Plasmid Mini Kit (Nippon Genetics Co., Ltd.), a plasmid for expressing VHH-C was extracted according to the attached instructions and further purified.

### 2. Expression of VHH-C Using C. glutamicum

Each of the plasmids manufactured as above was introduced into C. glutamicum by electroporation, the expression of VHH (homodimer) was confirmed, and purification was performed. The experimental procedure was the same as that in "2. Expression of VHH (Monomer) Using C. glutamicum" and "3. Purification of VHH (Monomer)" in Example 4.

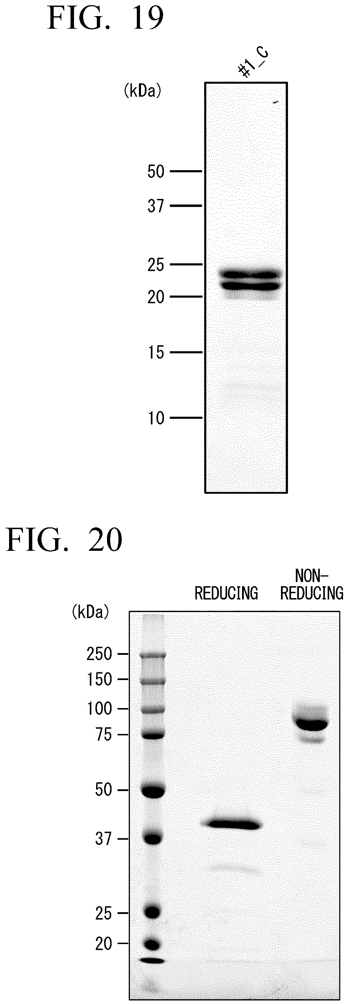

An electrophoresis image of SDS-PAGE of VHH (homodimer) is shown in FIG. 19. In FIG. 19, #1-C represents a VHH clone, and the numbers 10 to 75 represent a molecular weight and a marker.

### (Example 13) Production of VHH (Fc Body)

For VHH Clone #1 obtained in Example 4, AAVpro293T cells were used to express VHH (Fc body) (hereinafter sometimes referred to as "VHH-Fc") as described below, which was purified.

### 1. Construction of Plasmid for Expressing VHH-Fc

A restriction enzyme sequence for ligation with a pFUSE vector (manufactured by InvivoGen) was added to VHH Clone #1 of Example 4 as follows. First, VHH Clone #1 was amplified by PCR under the condition of performing 30 cycles of 10 seconds at 98°C, 5 seconds at 55°C, and 5 seconds at 72°C, using a plasmid for expressing VHH Clone #1 as a template, and the primers shown in Table 9 below, pFc-VHH#1-F (SEQ ID NO: 56), and pFc-VHH#l-R (SEQ ID NO: 57). The obtained amplification product was purified using a Gel/PCR Extraction Kit (Nippon Genetics Co., Ltd.) according to the attached instructions.

**[Table 9]**

| Base sequence of primer for PCR | | |
|---|---|---|
| Name | Base sequence (5' → 3') | SEQ ID NO: |
| pFc-VHH# 1-F | TTGCACTTGTCACGAATTCGCAGTTGCAGCTCGTGGAGTCTG | 56 |
| pFc-VHH# 1-R | TGTCAGATCTAACCATGGCCGATGAGGAGACGGTGACCTGGGTC | 57 |

Subsequently, FastDigest EcoRI and FastDigest Ncol (both restriction enzymes manufactured by Thermo Fisher Scientific, Inc.) were added to the pFUSE vector and the PCR amplification product, and the mixture was reacted at 37°C for 30 minutes. Next, the enzyme reaction solution was loaded into a 1% agarose gel including 1 × Gel green and subjected to electrophoresis at 100 V for 30 minutes. Thereafter, using FastGene Gel/PCR Extraction Kit, extraction was performed from the gel according to the attached instructions, and further purification was performed.

Next, the plasmid vector and VHH #1 DNA were mixed at 1:3 (molar ratio), Ligation High Ver. 2 was added thereto, and the mixture was reacted at 16°C for 30 minutes to obtain a plasmid for expressing VHH-Fc. Escherichia coli JM-109 was transformed with the obtained plasmid for expressing VHH-Fc. The transformed Escherichia coli JM109 was seeded on an agar medium plate and cultured at 37°C overnight.

The colonies that appeared on the agar medium were picked, and cultured overnight at 37°C, and using FastGene Plasmid Mini Kit, a plasmid for expressing VHH-C was extracted according to the attached instructions and further purified.

### 2. Expression of VHH-Fc Using AAVpro293T Cells

AAVpro293T cells (manufactured by Takara Bio Inc.) were subcultured in 10% FBS-containing D-MEM (High-glucose, Fujifilm Wako Pure Chemical Corporation). AAVpro293T cells were seeded on a culture dish for adhesive cells and cultured at 37°C in a 5% CO₂ environment. A plasmid for expressing VHH-Fc and PEI MAX (manufactured by Polysciences, Inc.) were suspended in Opti-MEM (manufactured by Gibco), and the suspension was left to stand at room temperature for 30 minutes. Thereafter, the cells were subjected to gene introduction in addition to AAVpro293T cells cultured overnight, and cultured overnight at 37°C in a 5% CO₂ environment. The medium was replaced with D-MEM containing a serum, and after culturing for 72 hours, the culture supernatant was recovered. The recovered culture supernatant was treated with a 0.22 µm filter to remove cells from the supernatant.

### 3. Purification of VHH-Fc

The culture supernatant was loaded onto a MonoSpin L ProG column (manufactured by GL Sciences Inc.). Thereafter, VHH-Fc was purified using MonoSpin ProA/G Buffer Kit (manufactured by GL Sciences Inc.) according to the attached instructions.

The purity of the eluted VHH-Fc was confirmed by SDS-PAGE (FIG. 20). SDS-PAGE was performed on a 4% concentration gel and a 10% separation gel. After applying 5 µL of a sample to each well, electrophoresis was performed under the conditions of 150 V for 1 hour. Precision Plus Protein Standard was used as a molecular weight marker. The eluate obtained as described above was dialyzed overnight at 4°C against PBS. Thereafter, the eluate was transferred to AMICON ULTRA 4, 10 kDa (manufactured by Millipore Corporation) and centrifuged at 3,500 × g for 30 minutes at 4°C to concentrate VHH-Fc. The concentration of VHH-Fc was quantified by a BCA method, using bovine serum albumin as a standard protein and using Pierce BCA Protein Assay Kit.

### (Example 14) Identification of Extracellular Domain of FGFR1 to Which VHH Clone #1 Binds

The FGFR1 extracellular domain has DII and DIII domains. Each of an FGFR1 extracellular domains (hereinafter may be abbreviated as "FGFR1-DIIDIII", "FGFR1-DII", or "FGFR1-DIII") was expressed using AAVpro293T cells as follows, these domains were purified, and an affinity measurement test with VHH Clone #1 was performed by a biolayer interference (BLI) method.

### 1. Construction of Plasmid for Expressing FGFR1 Extracellular Domain

First, each DNA was obtained by DNA fragment synthesis (Eurofin Genomics Co., Ltd.). The plasmid purified in the section 1 of Example 14, the DNA fragment, and the SLiCE reaction solution were added to an 8-strip PCR tube including an SLiCE buffer including 50 mM Tris-HCl (pH 7.5), 10 mM MgCl₂, 1 mM ATP, and 1 mM DTT, and the mixture was reacted at 37°C for 15 minutes to obtain a plasmid for expressing FGFR1-DII, FGFR1-DIII, and FGFR1-DIIDIII. Escherichia coli JM-109 was transformed with each of the obtained plasmids. The transformed Escherichia coli JM109 was seeded on an agar medium plate and cultured at 37°C overnight. The colonies that appeared on the agar medium were picked, and cultured overnight at 37°C, and using FastGene Plasmid Mini Kit, a plasmid for expressing VHH-C was extracted according to the attached instructions and further purified.

### 2. Expression of FGFR1 Extracellular Domain Using AAVpro293T Cells

The same operation as in the section 2 of Example 14 was performed to obtain a culture supernatant including FGFR1-DIIDIII, FGFR1-DII, and FGFR1-DIII.

### 3. Purification of FGFR1 Extracellular Domain

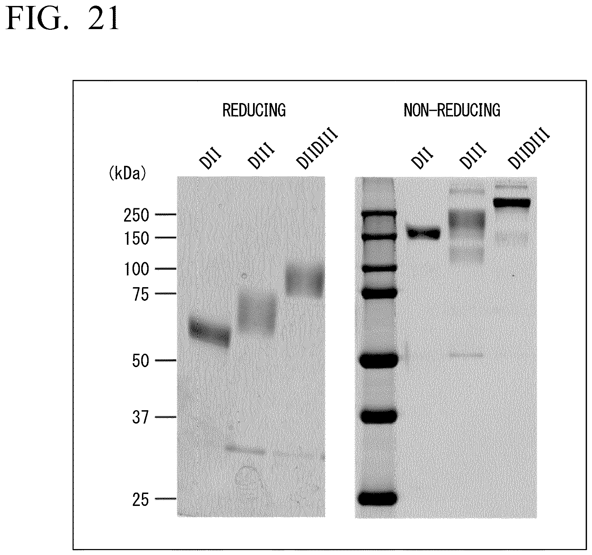

The same operation as in the section 3 of Example 14 was performed to obtain FGFR1-DIIDIII, FGFR1-DII, and FGFR1-DIII (FIG. 21).

### 4. Measurement of Binding Activity By Bilayer Interference Method

The binding activities of FGFR1-DIIDIII, FGFR1-DII, and FGFR1-DIII to VHH Clone #1 were measured by a biolayer interference method (hereinafter sometimes abbreviated as "BLI"). In BLI, using Octet 384 (manufactured by Fortebio), VHH Clone #1 diluted with a kinetics buffer (0.05% Tween20-containing PBS, pH 7.4) was immobilized on an Anti-Penta-HIS (HIS1K, manufactured by Fortebio) sensor chip, and bound to FGFR1-DIIDIII, FGFR1-DII, and FGFR1-DIII, which had been adjusted from 100 nM in 2-fold serial dilutions, and the dissociation rate was measured.

In the preparation for measurement, a tip of the sensor chip was immersed in 200 µL of the kinetics buffer for 10 minutes in order to hydrate the sensor chip. Thereafter, 70 µL of each measurement solution was added to a 384-well black plate (manufactured by Fortebio), and measurement was performed in the following steps 1) to 6).
1) Baseline step: Baseline measurement (30 seconds) in a kinetics buffer,
2) Loading step: Immobilization of a VHH antibody on a sensor (120 seconds),
3) Baseline step: Baseline measurement (30 seconds) in a kinetics buffer,
4) Association step: Association with each FGFR1 protein (120 seconds),
5) Dissociation step: Measurement in a kinetics buffer (120 seconds),
6) Regeneration step: Measurement in Glycin-HCl (pH 2.2) for 5 seconds and measurement in a kinetics buffer for 5 seconds are repeated three times.

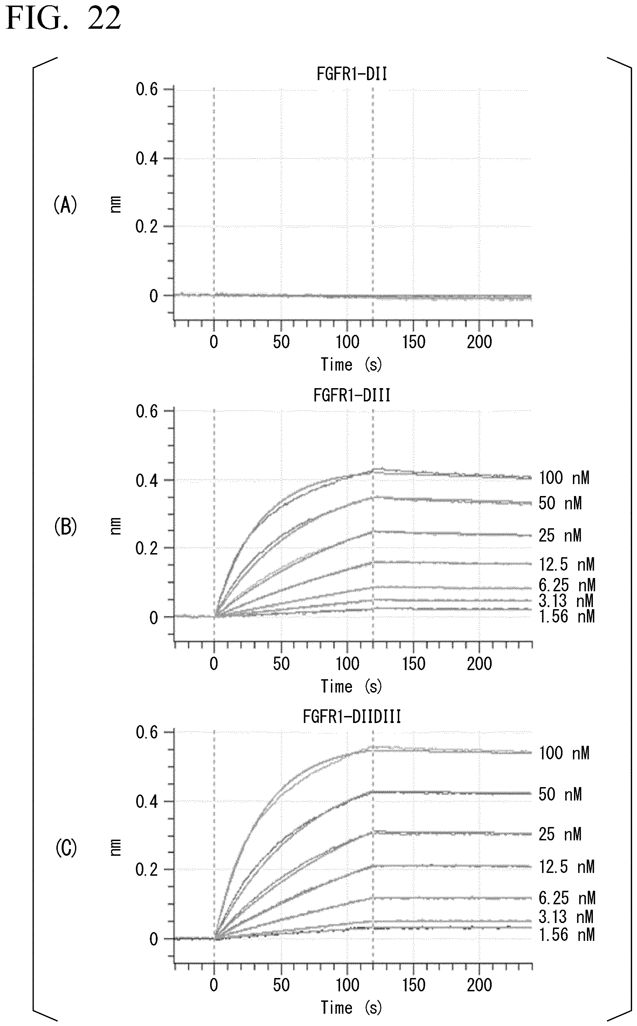

After completion of the measurements, the measured value of a Reference (only the kinetics buffer) was subtracted from the actual measurement value, and Global Fitting was performed using a 1:1 binding model using Octet software to calculate the binding activity (FIG. 22). The equilibrium dissociation constant (KD) of VHH Clone #1 for each FGFR1 protein is shown in Table 10.

**[Table 10]**

| Equilibrium dissociation constant for each FGFR1 protein of VHH Clone #1 | | | |
|---|---|---|---|
| FGFR1 Protein | KD (M) | Kon (Ms⁻¹) | Koff (s⁻¹) |
| FGFR1-DIIDIII | 4.89 × 10⁻¹⁰ | 2.89 × 10⁵ | 1.41 × 10⁻⁴ |
| FGFR1-DII | No Binding | | |
| FGFR1-DIII | 1.18 × 10⁹ | 2.98 × 10⁵ | 3.51 × 10⁻⁴ |

### (Example 15) Measurement of Agonist Activities of VHH-C and VHH-Fc

In order to verify the agonist activity for VHH-C (represented by #1_C in the drawing), #1-L4-#1, and #1_Fc, which were obtained in Examples above, the measurement was performed as described below using Western blotting for a change in expression level of Phospho-p44/42 MAPK (Erk1/2). Furthermore, a basic fibroblast growth factor (bFGF; manufactured by Fujifilm Wako Pure Chemical Corporation) was used as a positive control.

### 1. Cell Culture

As NIH3T3, those described in Example 7 were used. NIH3T3 cells were seeded on a 6-well plate for cell culture at 2 × 10⁵ cells/well, and culture was performed overnight (37°C, in a 5 % CO₂ incubator, with light shielded). After washing with a serum-free medium, bFGF (final concentration: 10 ng/mL) or VHH (final concentration: 100 nM) diluted with the serum-free medium was added thereto, and normal culture was performed for 30 minutes. Thereafter, the well was washed with ice-cooled PBS, and the cells were peeled off with a cell scraper, transferred to a tube, and centrifuged at 300 × g for 5 minutes at 4°C to recover the cells. Thereafter, the cells were solubilized with a phosphatase inhibitor-containing RIPA buffer and left to stand on ice for 30 minutes. After the solubilization reaction, centrifugation was performed at 12,000 × g for 15 minutes at 4°C to recover the supernatant. The obtained cell lysate was suspended in a sample buffer for 6 × SDS-PAGE (containing a reducing agent), and subjected to SDS-PAGE and Western blotting.

### 2. Western Blotting

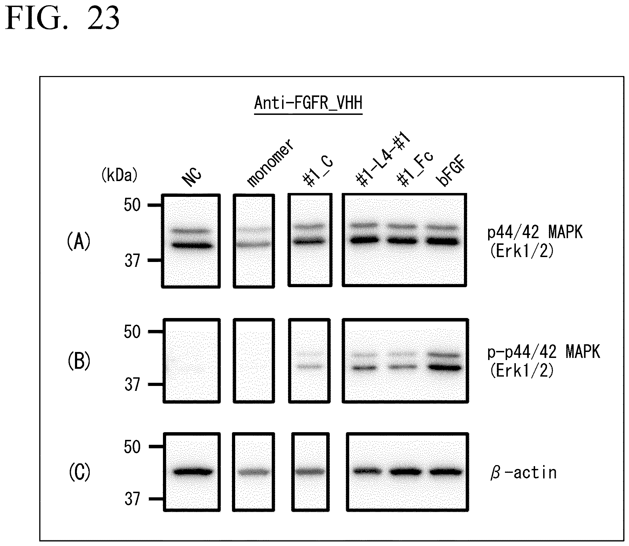

In Western blotting, anti-p44/42 MAPK (Erk1/2) (137F5) (#4695, CST), anti-Phospho-p44/42 MAPK (Erk1/2) (Thr202/Tyr204) (D13.14.4E) (#4370, CST), and anti-β-Actin (13E5) (#4970, CST) were used as a primary antibody, and an anti-rabbit IgG, HRP-linked antibody (#7074, CST) was used a secondary antibody. In FIG. 23, NC represents a negative control without an additive, monomer indicates a monomer of Clone #1, and bFGF (FGF) indicates a positive control.

FIG. 23(A) confirmed the expression of p44/42 MAPK (Erk1/2) alone, and FIG. 23(B) confirmed the expression of phosphorylated p44/42 MAPK (Erk1/2). With β-Actin in FIG. 23(C), it was confirmed whether or not a phosphorylated peptide was detected. As a result, it was confirmed that all of #1_C, #1-L4-#1, and #1_Fc exhibit an agonist activity in a case where the NIH3T3 cells were used.

### (Example 16) Measurement of Cell growth Activity of VHH-C and VHH-Fc

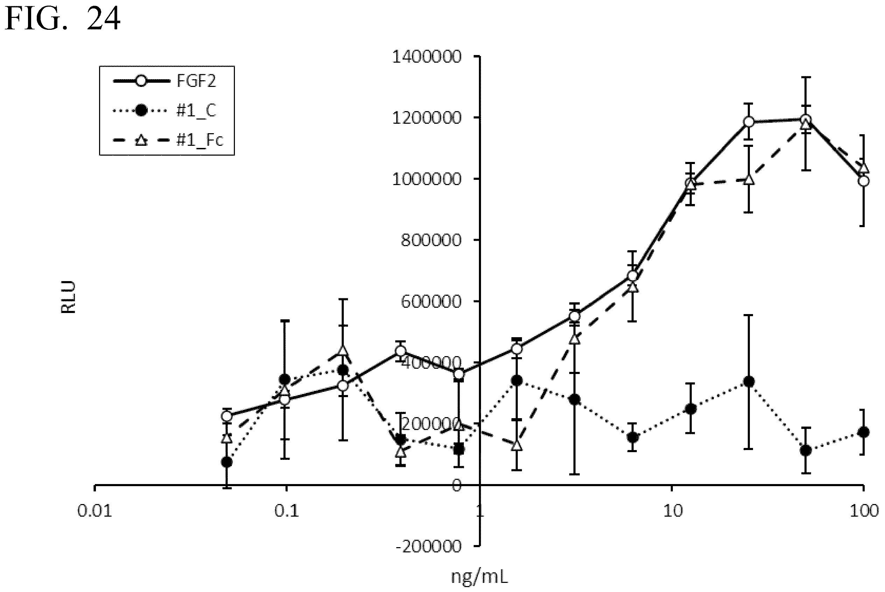

The same operation as in Example 10 was performed, and the cell growth activities of VHH-C and VHH-Fc (FIG. 24) were measured.

### (Example 17) Evaluation of iPS Cell Culturability (Growth Performance and Maintenance of Undifferentiated State)

### 1. Preparation of Evaluation

### (Recovery of cryopreserved Cells)

10 mL of a TeSR-E8 medium (Stemcell Technologies, ST05990) was added into a 15 mL PP tube and warmed in a thermostatic water tank (set at 37°C). In addition, the medium to be used was collected in advance in another tube, Y-27632 was added thereto to reach 10 µM, and the medium was warmed to room temperature. 253G1 cells (provided by: Center for iPS Cell Research and Application, Kyoto University (CiRA)) stock tube preserved in a liquid nitrogen gas phase were taken out from a liquid nitrogen container, thawed in a thermostatic water tank (set at 37°C), and then rapidly transferred into the heating tube (including 10 mL of the medium) in a clean bench (PHC, C/N: MCV-B131S-PJ) by pipetting. After mixing by pipetting, centrifugation (200 × g, 5 minutes) was performed and then the supernatant was removed with an aspirator.

An appropriate amount of complete TeSR-E8 (adjusted according to the TeSR-E8 manual; hereinafter referred to as cTeSR-E8) warmed to room temperature was added to the cells and mixed, and then the number of cells was evaluated with a cell counter (Bio-Rad Laboratories, Inc., C/N: TC-20). 253G1 cells were seeded on a 6-well plate at a seeding density of 1 to 5 × 10⁵ cells/well on a plate coated in advance with iMatrix-511 (1 µL/mm²) (Nippi, Inc., 892012), and left to stand in a 5% CO2 incubator (PHC, C/N: MCO-170AICUV-PJ) at 37°C. On the next day, medium exchange was performed using cTeSR-E8 (without addition of Y-27632). Medium exchange was carried out in the same manner as appropriate even on the next day and afterwards. After 3 to 5 days from the start of culture, cell growth could be confirmed, and passage was carried out at a stage in which the confluence reached 50% to 90%.

### (Passaging Cells)

The cells were observed under a microscope, and passage was performed in a case where the cells cultured on the 6-well plate were at 50% to 90% confluence. cTeSR-E8 to be used was collected in a PP tube in advance, Y-27632 was added thereto to obtain a concentration of 10 µM, and the mixture was warmed to room temperature. The cells were washed with D-PBS (Fujifilm Wako Pure Chemical Corporation, C/N: 045-29795), subsequently 300 µL of TrypLE Select Enzyme (Thermo Fisher Scientific, Inc., 12604013) was added thereto, and the mixture was left to stand in a 5% CO₂ incubator at 37°C.

After 5 to 10 minutes, the culture container was taken out from the CO₂ incubator. After confirming that the cell morphology had become rounder under a microscope, 1 mL of cTeSR-E8 was added to each well, and the cells were pipetted to release the cells, suspended them, and recovered in a 15 mL PP tube. The tube was centrifuged (200 × g, 5 minutes) and the supernatant was removed with an aspirator. An appropriate amount of cTeSR-E8 was added to the cell pellet and mixed by pipetting.

A part of the cell suspension was collected and the number of cells was evaluated with a cell counter. The cell suspension was appropriately diluted with cTeSR-E8 and seeded on a 6-well plate newly coated with iMatrix-511 so that the cells were at 0.1 to 5 × 10⁵ cells/well. Furthermore, one well was adjusted to 2 mL/well. The next day, cTeSR-E8 was warmed to room temperature and then used for medium exchange. During the culture period, the cells were observed with a microscope as appropriate, and images were captured and recorded as necessary. Medium exchange was performed in the same manner even on the next day and afterwards.

### 2. Evaluation

### (Purpose of Evaluation)

In order to examine whether or not VHH could be used as an alternative additive to basic FGF in the cTeSR-E7 medium, the cell growth potency and the undifferentiation performance were evaluated using iPS cells. In addition, it was evaluated whether VHH preserved at 4°C for 3 to 4 weeks maintained the functions.

### 2.1. Evaluation of Cell Growth

### (Evaluation Procedure)

Complete TeSR-E6 was adjusted according to the manual for TeSR-E6 (Stemcell Technologies, ST05946). A frozen stock of TGF-β1 (R&D Systems, 240-B-002), which has been prepared to be at 20 µg/mL with 0.1% w/v BSA and 4 mM HCl, was added so that a final concentration was 2 ng/mL, whereby cTeSR-E7 was created. cTeSR-E7 was warmed to room temperature in advance. The cells were cultured using cTeSR-E8, and then the cells with a 50% to 90% confluence state were used for passage. After washing with D-PBS, TrypLE Select was added at 300 µL/well, and the cells were released and recovered. After the centrifugation, the supernatant was removed, and an appropriate amount of cTeSR-E7 was added to the pellet, which was subsequently pipetted and suspended.

The cell suspension was collected and the number of cells was evaluated with a cell counter. A part of the cell suspension was collected in a new 15 mL PP tube, and new cTeSR-E7 was added thereto for dilution to 0.5 to 5 × 10⁴ cells/mL. A stock of basic FGF (PeproTech, AF-100-18B) (prepared with a 0.1% BSA/PBS solution to 10 µg/mL, and preserved in a freezer (-80°C); the amino acid sequence of basic FGF is an amino acid sequence of SEQ ID NO: 58; and furthermore, "basic FGF" is sometimes described as "bFGF") or VHH (homodimer (SEQ ID NO: 23, sequence name: #1-L4-#1, hereinafter also described as "VHH (homodimer)"), 10 µg/mL) was thawed and added separately to the cell suspension so that the concentration thereof was 100 ng/mL. Furthermore, at this time, a cell suspension without addition of an additive was also prepared for a negative control.

The cell suspension was seeded on a 6-well plate at a density of 0.1 to 1 × 10⁵/2 mL/well, which was left to stand in a 5% CO₂ incubator at 37°C. On the next day, cTeSR-E7 with or without addition (negative control) of an additive of each group, that is, basic FGF (positive control) or VHH (evaluation sample) was used for medium exchange in each group. During the culture period, the cells were observed with a microscope as appropriate, and images were captured and recorded as necessary. Medium exchange was carried out in the same manner as appropriate even on the next day and afterwards. Passage was carried out on Days 5 to 7. However, at the time of passage, the number of cells was individually evaluated in each well with a cell counter, then the cell suspensions in each group were mixed, and the individual values of the acquired cell numbers were averaged and used for preparing the cell suspension during the next cell seeding. The acquired individual values of cell number counts were used to calculate a cell growth straight line.

### (Results)

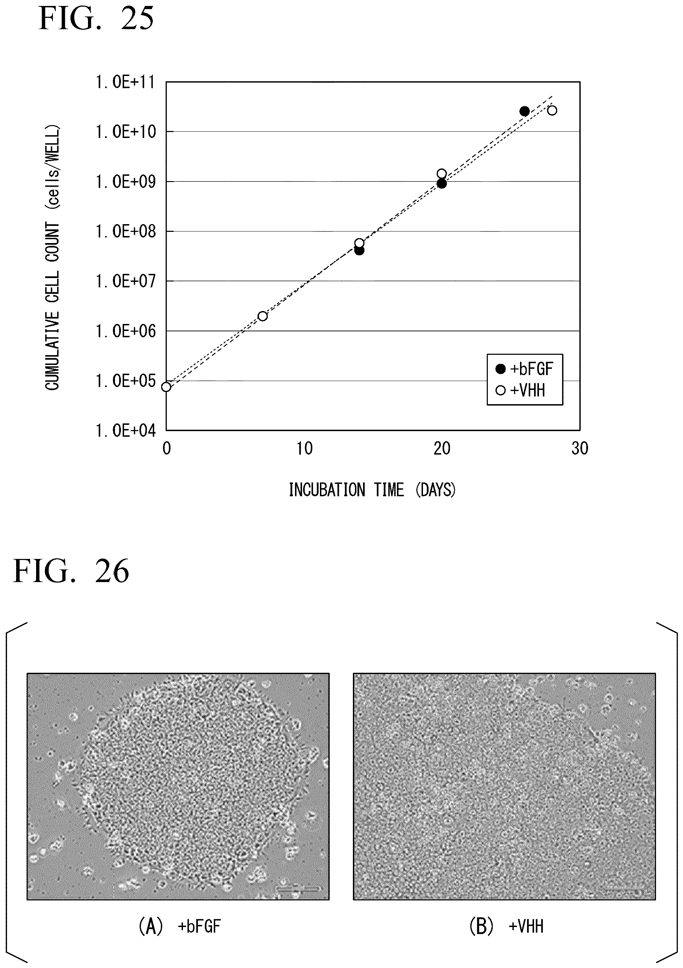

The individual values of the counting results are shown in Table 11, and the cumulative numbers (cells) of cells calculated from the counting values are shown in Table 12. FIG. 25 is a graph showing the cumulative number of cells. FIG. 26 is an image of cells (third passage, Day 5). During a culture period of 3 to 4 weeks, there was no difference in growth performance or cell morphology between the two. In addition, since the cells in the group in which no supplement had been added to cTeSR-E7 had a low growth, the cell morphology after the first passage changed, the undifferentiated state was not maintained, and thus, the culture was stopped.

**[Table 11]**

| | | T7 + bFGF | | | T7 + VHH | | | T7 | | |
|---|---|---|---|---|---|---|---|---|---|---|
| Number of passages | Number of days after seeding | Number of seedings | Count before passage | Survival rate (%) | Number of seedings | Count before passage | Survival rate (%) | Number of seedings | Count before passage | Survival rate (%) |
| 0 | Day 7 | 7.50E + 04 | 1.55E + 06 | 96.3 | 7.50E + 04 | 2.01E + 06 | 97 | 7.50E + 04 | 1.25E + 06 | 98 |
| | | 7.50E + 04 | 2.36E + 06 | 99 | 7.50E + 04 | 1.91E + 06 | 97.3 | 7.50E + 04 | 1.45E + 06 | 97.7 |
| | | Average of well | 1.96E + 06 | 97.7 | Average of well | 1.96E + 06 | 97.2 | Average of well | 1.35E + 06 | 97.6 |
| 1 | Day 7 | 7.50E + 04 | 1.67E + 06 | 99 | 7.50E + 04 | 2.07E + 06 | 99 | 7.50E + 04 | Differentiation progressed, and counting and passage were not completed. | |
| | | 7.50E + 04 | 1.5E + 06 | 97.3 | 7.50E + 04 | 2.35E + 06 | 98.3 | 7.50E + 04 | | |
| | | Average of well | 1.59E + 06 | 98.15 | Average of well | 2.21E + 06 | 98.65 | Average of well | - | - |
| 2 | Day 6 | 7.50E + 04 | 1.64E + 06 | 97.7 | 7.50E + 04 | 1.96E + 06 | 99 | | | |
| | | 7.50E + 04 | 1.68E + 06 | 98 | 7.50E + 04 | 1.80E + 06 | 98.3 | | | |
| | | Average of well | 1.66E + 06 | 97.5 | Average of well | 1.88E + 06 | 98.5 | | | |
| 3 | Day 6 | 7.50E + 04 | 1.94E + 06 | 94.3 | | | | | | |
| | | 7.50E + 04 | 1.95E + 06 | 88.3 | | | | | | |
| | | Average of well | 1.95E + 06 | 96 | | | | | | |
| | Day 8 | | | | 7.E + 05 | 1.29E + 06 | 95.3 | | | |
| | | | | | 7.E + 05 | 1.28E + 06 | 97.7 | | | |
| | | | | | Average of well | 1.29E + 06 | 96.5 | | | |

**[Table 12]**

| Day | 0 | 7 | 14 | 20 | 26 | 28 |
|---|---|---|---|---|---|---|
| T7 + bFGF | 7.5E + 04 | 2.0E + 06 | 4.2E + 07 | 9.2E + 08 | 2.6E + 10 | |
| T7 + VHH | 7.5E + 04 | 2.0E + 06 | 5.8E + 07 | 1.4E + 09 | | 2.7E + 10 |

### 2.2. Evaluation of Undifferentiated State

### (Evaluation Procedure)

The cells were cultured in the medium of each group for 3 to 5 weeks, and the following operations were performed on Days 4 to 7 of the passage. After washing the cells with D-PBS, 1 mL/well of 4% formaldehyde was added. After being left to stand for 30 minutes or longer, the solution was removed and washing was performed with D-PBS. 0.1% Triton X-100/D-PBS was added thereto and the mixture was left to stand at room temperature for 3 to 5 minutes. After washing with D-PBS, 1% of BSA/D-PBS was added thereto and the mixture was left to stand for 30 minutes.

An antibody for undifferentiation marker staining, such as OCT4 (Abcam, ab19857) or SSEA4 (Abcam, ab16287), was diluted in 1% BSA/D-PBS at an appropriate ratio, added to a well, which was left to stand for 30 to 60 minutes. After washing with D-PBS, secondary antibodies (a Goat anti-Rabbit IgG antibody (Abcam, ab150115) and a Goat anti-Mouse IgG antibody (Abcam, ab150077)) were diluted in 1% BSA/D-PBS at an appropriate ratio and added to the wells, which were left to stand with light shielded with an aluminum foil for 30 to 60 minutes. DAPI (DOJINDO LABORATORIES, 340-07971) was diluted with 1% BSA/D-PBS to a concentration of 1 to 10 µg/mL, and added to each well, which was left to stand for 5 minutes. After washing twice or three times with D-PBS, D-PBS was added to 1 mL well. The image was acquired by observing the sample under a fluorescence microscope.

### (Results)

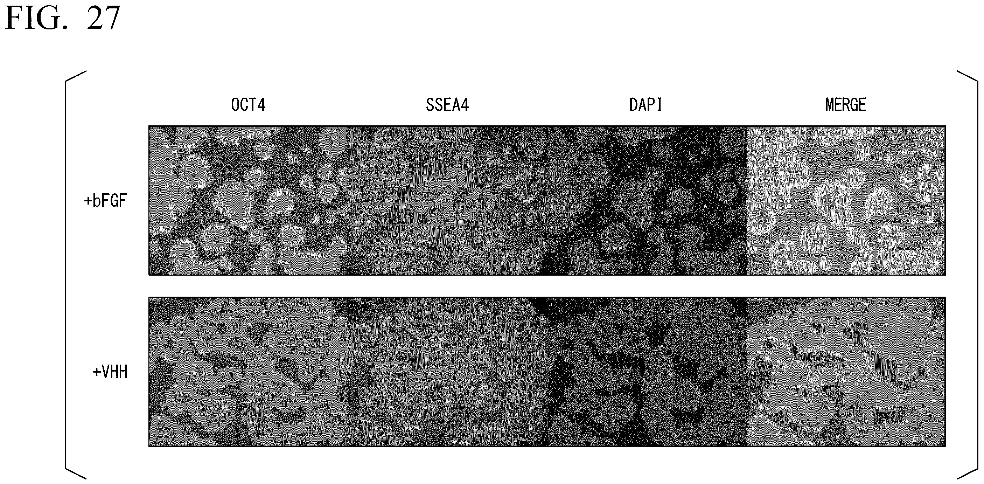

Images of undifferentiation marker staining (second passage, Day 6 (Culture day 20)) are shown in FIG. 27. Since both the basic FGF-added medium and the VHH-added medium were positive for the undifferentiation markers OCT4 and SSEA4, it was suggested that iPS cells maintained an undifferentiated state even after being cultured for 20 days.

### 2.3. Evaluation of Preservation Stability

### (Evaluation Procedure)

Basic FGF and VHH stocks were collected in a 1.5 mL microtube 3 or 4 weeks before culturing, and preserved in a refrigerator (4°C). Hereinafter, all operations were the same as those in the cell growth evaluation procedure, except that for basic FGF and VHH, a medium was prepared so that the both were preserved at 20 ng/mL using both refrigeration (4°C)- and freeze (-80°C)-preservation conditions.

### (Results)

It was found that VHH preserved at 4°C exhibited growth performance similar to that of VHH preserved at -80°C, and maintained the functions. On the other hand, with regard to basic FGF, in a case where basic FGF was preserved at 4°C, the growth performance was significantly deteriorated and the original function of basic FGF was not maintained (data not shown).

### (Example 18) Evaluation of MSC Culturability (Growth Performance and Maintenance of Undifferentiated State)

### 1. Preparation of Evaluation

### Recovery of cryopreserved Cells

10 mL of a used portion of D-MEM medium (Low Glucose, Glutamine) (Fujifilm Wako Pure Chemical Corporation, C/N: 041-29775) was added to a 15 mL PP tube, and warmed in a thermostatic water tank (set at 37°C). A human bone marrow-derived MSC (Stemcell Technologies, C/N: 70022) stock tube preserved in a liquid nitrogen gas phase was taken out from a liquid nitrogen container and thawed in a thermostatic water tank (set at 37°C). Thereafter, the cells were rapidly moved with a pipette into the tube (including 10 mL of the medium) that had been heated to 37°C in a clean bench, and mixed by pipetting. The tube was centrifuged (300 × g, 5 minutes) and the supernatant was removed with an aspirator.

An appropriate amount of D-MEM that had been warmed to room temperature was added to a cell pellet and mixed, and then the number of cells was evaluated with a cell counter. An appropriate amount of the cell suspension was taken into a 15 mL PP tube and centrifuged (300 × g, 5 minutes). The supernatant was removed, an appropriate amount of D-MEM (containing 10% or 20% of FBS (Thermo Fisher Scientific Inc., 16140071); the stock was preserved under refrigeration and used within 2 weeks) was added to a cell pellet to adjust a cell suspension (1).

The number of cells was evaluated again with a cell counter and the cells were appropriately diluted with each of the media. Next, a stock of basic FGF or VHH was added to the cell suspension (1), so that each of the final concentrations was 1 ng/mL or 20 ng/mL. Seeding was performed onto a CellBIND 6-well plate (Corning, C/N: 3335) at a density of 3-5 × 10⁴/well, and after 2 to 4 days, medium exchange was performed using D-MEM (containing 10% FBS or 20% FBS).

### (Passaging Cells)

The cells were observed under a microscope, and basically, passage was carried out after 5 days from seeding. In advance, the used D-MEM (0% FBS, 10% FBS, or 20% FBS) was warmed in advance in a thermostatic water tank (set at 37°C). After washing the cells with D-PBS, 500 µL of TrypLE Express was added thereto and the cells were left to stand in a CO₂ incubator. After 5 to 10 minutes, the culture container was removed from the CO₂ incubator, and a change in cell morphology was confirmed under a microscope. Thereafter, 1 mL of D-MEM was added to each well, and the cells were released by pipetting, suspended, and then recovered in a 15 mL PP tube.

The tube was centrifuged (300 × g, 5 minutes) and the supernatant was removed with an aspirator. An appropriate amount of a D-MEM medium (containing 10% FBS or 20% FBS) was added to the cell pellet, and mixed by pipetting. A part of the cell suspension was collected and the number of cells was evaluated with a cell counter. After counting the number of wells in each well, the cell suspensions in the same group were collected in one tube and mixed together. The average value of individual wells was used to calculate the number of cells at the time of passage.

The cell suspension was appropriately diluted with D-MEM (10% FBS or 20% FBS). The cells were seeded on a new CellBIND 6-well plate at 3 to 5 × 10⁵ cells/well. Furthermore, one well was adjusted to 2 mL/well. After 2 to 4 days, medium exchange was performed with a medium at room temperature. During the culture period, the cells were observed with a microscope as appropriate, and images were captured and recorded as necessary.

### 2. Evaluation

### (Purpose of Evaluation)

Whether or not VHH can be used as an alternative additive to basic FGF in a D-MEM medium (containing 10% FBS or 20% FBS) will be examined by using fibroblasts.

### 2.1. Evaluation of Cell Growth

### (Evaluation Procedure)

The average value of the counts of number of cells per well acquired during the passage was used to create a cell growth straight line. In addition, a value obtained by dividing the number of cells per well at the time of passage by the number of cells at the time of seeding was defined as a growth rate during that period, and the rate was used at the time of calculating a cumulative number of cells. In a case in which the number of cells per well at the time of initial seeding is defined as P(0) and the time of the N^{th} passage is defined as P(N), the cumulative number of cells at each passage is determined by the following calculation equation. Cumulative number of cells (P1) = Number of initial seedings/Well (P0) × Growth rate (P0 to P1) Cumulative number of cells (P(N)) = Cumulative number of cells (P(N-1)) × Growth rate (P(N-1) to P(N))

The growth straight line was created by plotting the cumulative number of cells determined by the equation at each passage and then drawing an approximate straight line in Excel. However, in the present evaluation, the evaluation was carried out by fixing under the conditions where the concentrations of VHH and basic FGF were 20 ng/mL.

### (Results)

The seedings and the number of cells (average value) at the time of each passage are shown in Table 13, and the cumulative number of cells (average value) is shown in Table 14.

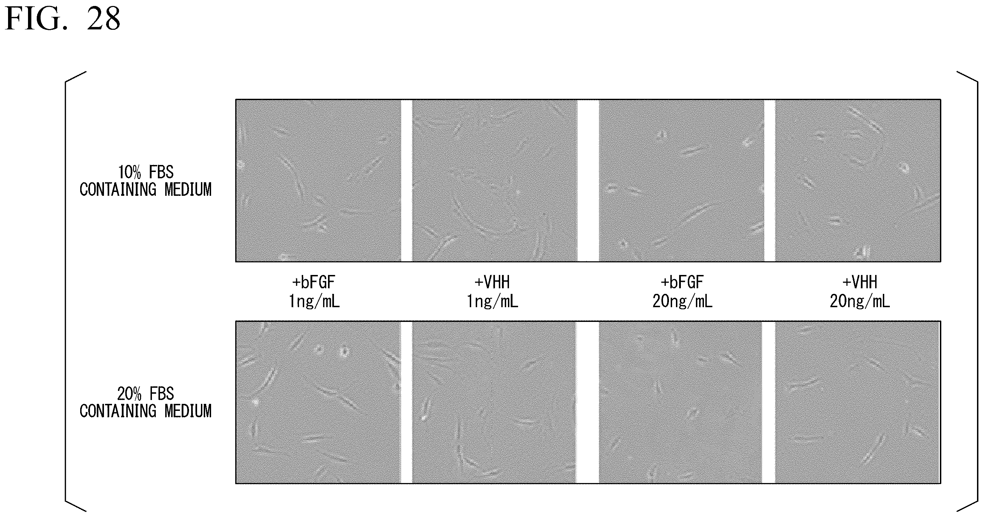

The morphology of MSC (4 passages, Day 1) is shown in FIG. 28. In a case of 10% FBS, the cells had a flattened morphology under the condition of a low concentration of VHH (1 ng/mL), and were different from the basic FGF. However, the cells exhibited a similar morphology to basic FGF under the conditions of a high-concentration VHH (20 ng/mL). On the other hand, in the case of 20% FBS, the same morphology was exhibited regardless of the addition concentration of VHH.

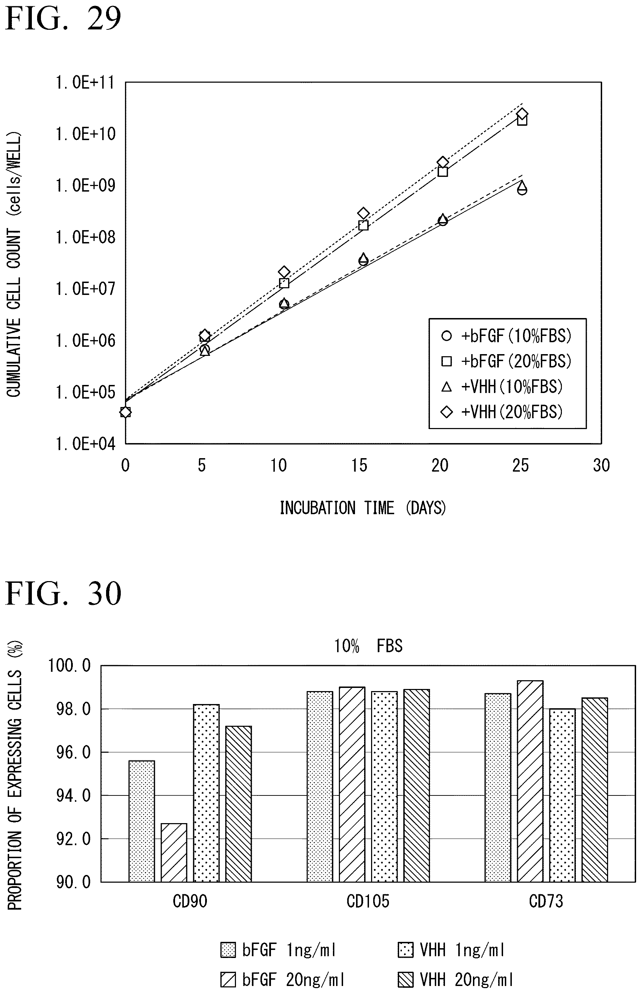

A graph of the cumulative number of cells is shown in FIG. 29. Basic FGF and VHH exhibited almost the same growth performance regardless of the FBS concentration to be added.

**[Table 13]**

| | P0 | | | P1 | | | |
|---|---|---|---|---|---|---|---|
| | | | Seeding | Count (2-well) | | Seeding (3-well) | |
| bFGF 20 ng/mL FBS 10% | | | 4.1E + 04 | 6.8E + 05 | | 5.0E + 05 | |
| bFGF 20 ng/mL FBS 20% | | | 4.1E + 04 | 1.2E + 06 | | 5.0E + 04 | |
| VHH 20 ng/mL FBS 10% | | | 4.1E + 04 | 6.3E + 05 | | 5.0E + 04 | |
| VHH 20 ng/mL FBS 20% | | | 4.1E + 04 | 1.3E + 06 | | 5.0E + 04 | |
| | | | | | | | |

| | P2 | | | P3 | | | |
|---|---|---|---|---|---|---|---|
| | Count (3-well) | Growth rate | Seeding (3-well) | Count (3-well) | Growth rate | | Seeding (3-well) |
| bFGF 20 ng/mL FBS 10% | 3.6E + 05 | 7.2 | 5.0E + 04 | 3.5E + 05 | 7.0 | | 5.0E + 04 |
| bFGF 20 ng/mL FBS 20% | 5.3E + 05 | 10.7 | 5.0E + 04 | 6.6E + 05 | 13.2 | | 5.0E + 04 |
| VHH 20 ng/mL FBS 10% | 4.3E + 05 | 8.5 | 5.0E + 04 | 3.7E + 05 | 7.5 | | 5.0E + 04 |
| VHH 20 ng/mL FBS 20% | 8.4E + 05 | 16.7 | 5.0E + 04 | 6.9E + 05 | 13.7 | | 5.0E + 04 |
| | | | | | | | |

| | P4 | | | P5 | | | |
|---|---|---|---|---|---|---|---|
| | Count (3-well) | Growth rate | Seeding (3-well) | Count (3-well) | Growth rate | | Seeding (3-well) |
| bFGF 20 ng/mL FBS 10% | 3.0E + 05 | 6.0 | 5.0E + 04 | 2.0E + 05 | 3.9 | | 5.0E + 04 |
| bFGF 20 ng/mL FBS 20% | 5.5E + 05 | 10.9 | 5.0E + 04 | 3.6E + 05 | 7.2 | | 5.0E + 04 |
| VHH 20 ng/mL FBS 10% | 2.9E + 05 | 5.8 | 5.0E + 04 | 2.2E + 05 | 4.3 | | 5.0E + 04 |
| VHH 20 ng/mL FBS 20% | 4.9E + 05 | 9.7 | 5.0E + 04 | 4.3E + 05 | 8.7 | | 5.0E + 04 |

**[Table 14]**

| | P0 | P1 | P2 | P3 | P4 | P5 |
|---|---|---|---|---|---|---|
| Number of culture days | 0 | 5 | 10 | 15 | 20 | 25 |
| bFGF 20 ng/mL FBS 10% | 4.1E + 04 | 6.8E + 05 | 4.9E + 06 | 3.4E + 07 | 2.1E + 08 | 8.1E + 08 |
| bFGF 20 ng/mL FBS 20% | 4.1E + 04 | 1.2E + 06 | 1.3E + 07 | 1.7E + 08 | 1.8E + 09 | 1.3E + 10 |
| VHH 20 ng/mL FBS 10% | 4.1E + 04 | 6.3E + 05 | 5.4E + 06 | 4.0E + 07 | 2.3E + 08 | LOE + 09 |
| VHH 20 ng/mL FBS 20% | 4.1E + 04 | 1.3E + 06 | 2.1E + 07 | 2.9E + 08 | 2.8E + 09 | 2.4E + 10 |

### 2.2. Evaluation of MSC Marker

### (Evaluation Procedure)

On Day 5 after the initial seeding, D-PBS was added to the cells and removed by suction. 500 µL of TrypLE Express was added to each well and the mixture was left to stand in a CO₂ incubator for 5 to 10 minutes. The cells were observed under a microscope, and in a case where it was possible to confirm that the cells had changed in morphology, a medium was added thereto, and the cells were recovered in a PP tube by pipetting, and centrifuged (300 × g, 5 minutes). After the centrifugation, the supernatant was removed by suction, an appropriate amount of 1% BSA/PBS was added to the pellet, and the mixture was pipetted to form a suspension.

A part of the suspension was collected and the number of cells was evaluated with a cell counter. Dilution was performed with 1% of BSA/PBS, so that the concentration was 0.6 to 1.0 × 10⁶ cells/mL. 50 µL of the mixture was dispensed into a 2 mL tube, so that the concentration was 3 to 5 × 10⁴ cells/tube. Antibodies (CD90: R & D Systems, FAB2067G; CD105: R & D Systems, FAB 10971P; CD73: R & D Systems, FAB5795P; isotype control (for CD73): R & D Systems, IC0041P; isotype control (for CD90): R & D Systems, IC003G; isotype control (for CD105): R & D Systems, IC002P) were diluted in advance with 1% BSA/PBS, so that the concentration was twice the final concentration, thereby preparing the required amount.

Equal amounts of the suspension subjected to the cell counter and the antibody solution adjusted above were mixed, and the mixture was left to stand on ice for 30 minutes or longer with light shielded. 1% of BSA/PBS was added thereto, the mixture was centrifuged (400 to 600 × g, 5 minutes), and then the supernatant was removed by suction. Then, 300 to 500 µL of 1% BSA/PBS was added thereto, and the pipetted sample was subjected to flow cytometric analysis using an Attune NxT Flow Cytometer (Thermo Fisher Scientific, Inc.).

### (Results)

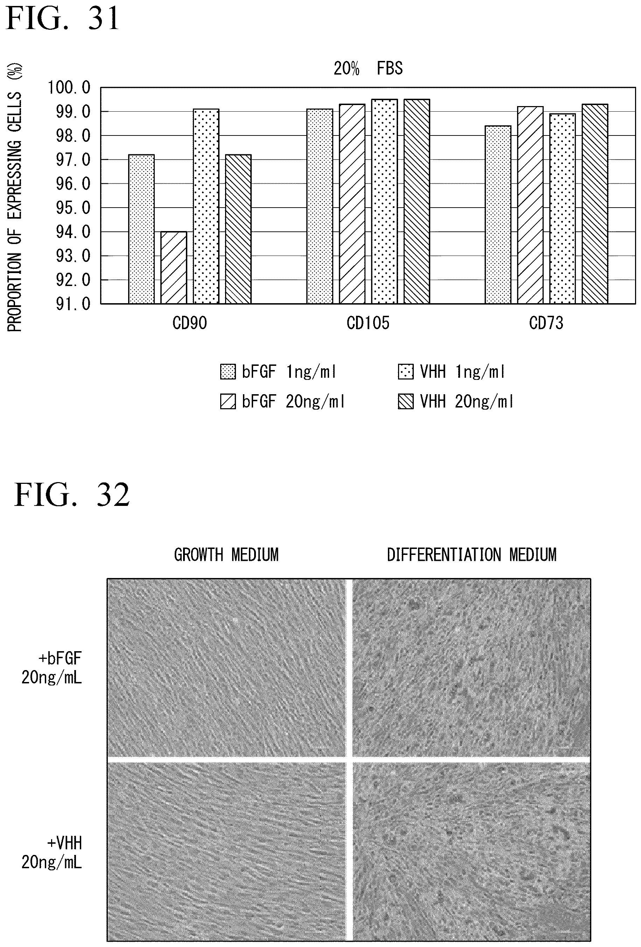

The comparison of the expressions of MSC markers (fourth passage, 10% FBS) is shown in FIG. 30, and the comparison of expressions of MSC markers (fourth passage, 20% FBS) is shown in FIG. 31. As is clear from FIGS. 30 and 31, CD105 and CD73 were expressed in 98% or more of the cells regardless of the concentrations of basic FGF and VHH. On the other hand, with regard to CD90, the number of expressing cells decreased in inverse proportion to the concentration in the basic FGF. In particular, at 20 ng/mL, CD90-expressing cells were less than 95%, resulting in not satisfying the minimum standards for a human MSC proposed by the International Society for Cellular therapy (ISCT). However, for VHH, 97% or more of the cells were positive cells at any concentration, suggesting clear superiority. These were the same results regardless of whether the concentration of FBS added was 10% or 20%.

Furthermore, the International Society for Cellular Cell therapy proposed, as a criteria for defining a human MSC, to satisfy minimum requirements of (1) having adhesiveness to plastics under standard culture conditions, (2) being positive for CD73, CD90, and CD105 of cell surface markers, and negative for CD 11b or CD14, CD 19 or CD79α, CD34, CD45, and HLA-DR, and (3) having a differentiation potency into osteoblasts, cartilage cells, and fat cells.

As described above, VHH showed superiority in maintaining cell quality over recombinant bFGFs in the related art.

### 2.3. Evaluation of Fat Differentiation Induction

### (Evaluation Procedure)

A third passage was performed in a CellBIND^{(R)} 24-well transparent multi-well plate (Corning, C/N: 3337), and then at a time of reaching 80% to 90% confluence, medium exchange was performed in MesenCult Stem Cell Adipogenic Differentiation Medium (PromoCell, C/N: C-28016) which is a medium for differentiation induction. Furthermore, for the cells as a negative control, D-MEM (10% FBS or 20% FBS) was used for the medium exchange. The medium exchange was carried out every 2 to 4 days.

After 14 days from seeding, a 4% paraformaldehyde/phosphate buffer solution was added thereto at 500 µL/well, and the cells were left to stand for 2 hours or longer. After the immobilization, purified water was added thereto at 500 µL/well. An Oil Red stock liquid (Cosmo Bio Co., Ltd., C/N: AK09F) and purified water were mixed at a ratio of 6:4, and the mixture was left to stand at room temperature for 10 to 15 minutes. Thereafter, the filtrate was filtered through a membrane filter having a pore size of 0.5 to 1.0 µm, and the filtrate was used as an Oil Red O liquid. 500 µL of the Oil Red O liquid was added to each well and the mixture was left to stand at room temperature for 15 minutes. Thereafter, the Oil Red O liquid was removed, and washing was performed three times or more with purified water until the washing water became transparent. The water was completely removed, and the coating film was dried, and observed with a microscope to acquire an image. An extraction liquid, Isopanol (Cosmo Bio Co., Ltd., C/N: AK09F), was added into the dried wells to elute the dye. 100 µL of the eluate was transferred to a 96-well plate and the absorbance at 520 nm was measured. However, the evaluation was carried out at a fixed concentration of 20 ng/mL for basic FGF and VHH.

### (Results)

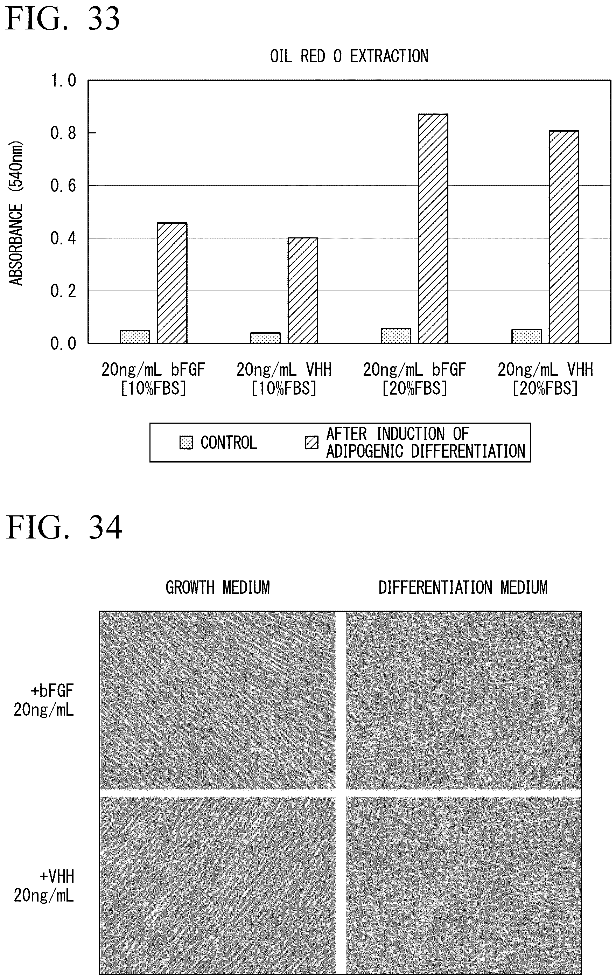

Images of fat differentiation induction (after 3-passage subculture) are shown in FIG. 32. In addition, a graph of the quantitative evaluation of the Oil Red O extracted after the fat differentiation is shown in FIG. 33.

Differentiation was similarly recognized in a medium supplemented with both basic FGF and VHH, and it was suggested that MSC maintained a differentiation potency into the fat cell line.

### 2.4. Evaluation of Bone Differentiation Induction

### (Evaluation Procedure)

A third passage was performed in a CellBIND^{(R)} 24-well transparent multi-well plate, and then at a time of reaching 80% to 90% confluence, medium exchange was performed in MesenCult Stem Cell Osteogenic Differentiation Medium (PromoCell, C/N: C-28013) which is a medium for differentiation induction. Furthermore, for the cells as a negative control, D-MEM (10% FBS or 20% FBS) which is a culture medium was used for the medium exchange. The medium exchange was carried out every 2 to 4 days.

After 14 days from seeding, a 4% paraformaldehyde/phosphate buffer solution was added thereto at 500 µL/well, and the cells were left to stand for 2 hours or longer. After the immobilization, purified water was added thereto at 500 µL/well. 500 µL of an Alizarin Red solution (PG Research Co., Ltd., ARD-SET) was added to each well, which was left to stand at room temperature for 30 minutes. The Alizarin Red solution was removed, and washing was performed three times or more with purified water until the washing water became transparent. The water was completely removed, and the coating film was dried, and observed with a microscope to acquire an image. 500 µL of a calcifide nodule dissolution solution (PG Research Co., Ltd., ARD-SET) was added into the well and distributed for 10 minutes to elute the dye. 100 µL of the eluate was transferred to a 96-well plate and the absorbance at 450 nm was measured. However, the evaluation was carried out at a fixed concentration of 20 ng/mL for basic FGF and VHH.

### (Results)

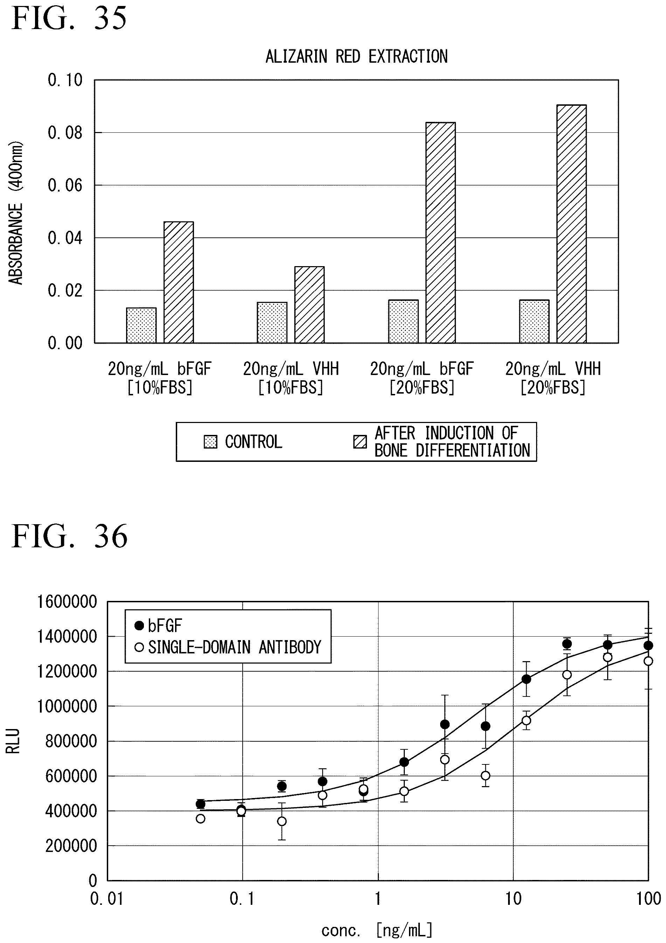

Images of bone differentiation induction (after 3 passages of subculture) are shown in FIG. 34. In addition, a graph of the quantitative evaluation of the Alizarin Red extracted after the bone differentiation is shown in FIG. 35. Differentiation was similarly recognized in a medium supplemented with both basic FGF and VHH, and it was suggested that MSC maintained the differentiation potency.

As described above, it was found that the single-domain antibody provided by the present invention can substitute for human bFGF in the maintenance culture for the undifferentiated state and the differentiation potency of iPS cells and MSC. From the viewpoint that the single-domain antibody has extremely high heat resistance with a Td value of 70°C or higher, it can be said that the single-domain antibody is a protein having excellent structural stability, and in actual use, it is excellent in terms of storage, transportation, and the like of a solution, as compared with human bFGF.

The method for culturing a cell of the present invention can maintain a high undifferentiated state, a high differentiation potency, and a high viability of a stem cell. Therefore, the method is an extremely useful technique in the field of regenerative medicine.

### (Example 19) Evaluation of Fibroblast Culturability

### 1. Promotion of Fibroblast Growth by VHH (Homodimer)

A mouse fibroblast cell line NIH/3T3 was used as a fibroblast. A mouse fibroblast cell line NIH/3T3 was obtained from ECACC through a domestic agent, subjected to extended culture, then cryopreserved at -80°C using CellBanker1 plus (manufactured by ZENOAQ), and used. The maintenance culture was performed with D-MEM (high glucose, manufactured by Fujifilm Wako Pure Chemical Corporation, hereinafter referred to as "DMEM"), 10% Bovine Serum (manufactured by New Zealand origin: Thermo Fisher Scientific, Inc., hereinafter referred to as "CS"), and 100 U Pen-Strep (manufactured by Thermo Fisher Scientific, Inc., hereinafter referred to as "PS (+)"), and a passage operation was performed according to an ordinary method under conditions presented by distribution sources using Trypsin-EDTA (manufactured by Thermo Fisher Scientific Inc.) and D-PBS (-). For the cell growth assay, cells with 3 to 10 passages were used.

The cell growth assay for bFGF was performed according to the following procedure. On Day 1, a suspension including 2.5 × 10⁴ cells/mL of cells in a medium of DMEM, 10% CS, and PS (+) was created, seeded on a CulturPlate-96 (White Opaque 96-well Microplate, manufactured by Perkin Elmer, Cat# 6005680) at 100 µL/well, and cultured in 5% CO₂ at 37°C for 16 to 24 hours. On Day 2, the medium was replaced with serum-free DMEM PS (+) including bFGF (PeproTech Cat No. AF-100-18B) or various test substances (antibodies or bFGF), and the cells were further cultured for 2 days. On Day 4, the 96-well plate was left at room temperature for 30 minutes, and then 100 µL of Cell Titer Glo 2.0 Reagent (manufactured by Promega Corporation) at room temperature was added to each well, followed by stirring at 500 rpm for 2 minutes with a plate mixer. The plate was further left to stand for 8 minutes, and then the luminescence in each well was measured with a Synergy HTX plate reader (manufactured by BioTek) with a sensitivity set (Gain) of 200 and a measurement time of 1 s/well. For an antibody, a homodimer of VHH (SEQ ID NO: 23, sequence name: #1-L4-#1, hereinafter also referred to as a "single-domain antibody") was used.

The measurement was performed at three points at each concentration of the test substance, and the conditions were set such that for the measurement at a single concentration, 100 ng/mL of bFGF was set as a positive control and a condition of no addition was set as a negative control. In addition, in a case of measuring EC₅₀, a 2-fold dilution series starting from 100 ng/mL (bFGF) was created for 12 concentrations and subjected to the measurement. The EC₅₀ measurement data were fitted to 4-parameter fit according to an ordinary method by control software attached to a plate reader, and an EC₅₀ value was calculated.

As shown in FIG. 36, the EC₅₀ of the single-domain antibody (VHH homodimer) was 11.6 nM and the EC₅₀ of bFGF was 5.1 nM. It was found that the both had substantially the same EC₅₀.

### 2. Inhibition of Growth Factor Activity of VHH Homodimer by FGFR Inhibitor

In order to confirm whether or not the VHH homodimer exhibits a cell growth activity via FGFR, the growth suppression by an FGFR kinase inhibitor was examined. As a kinase inhibitor, NVP-BGJ398 (IC₅₀ value for FGFR1/FGFR2/FGFR3: 0.9 nM/1.4 nM/1.0 nM) exhibiting a selective inhibitory activity against FGFR was used. The same operation as in the cell growth assay of bFGF was performed for no addition (-), 10 ng/mL and 100 ng/mL of bFGFs, 10 ng/mL and 100 ng/mL VHH homodimers (SEQ ID NO: 23, sequence name: #1-L4-#1, sometimes referred to as a single-domain antibody), 20 nM of NVP-BGJ398 (manufactured by ChemScene) were added at the same time as the above additions, which was compared with the one with no addition under the same conditions as in "1. Promotion of Fibroblast Growth by VHH (Homodimer)" described above.

In addition, it was separately confirmed that 20 nM of NVP-BGJ398 itself has little effect on cell growth. It was examined whether the cell growth activities of bFGF and the single-domain antibody were inhibited by NPV-BGJ398 which is an FGFR kinase inhibitor.

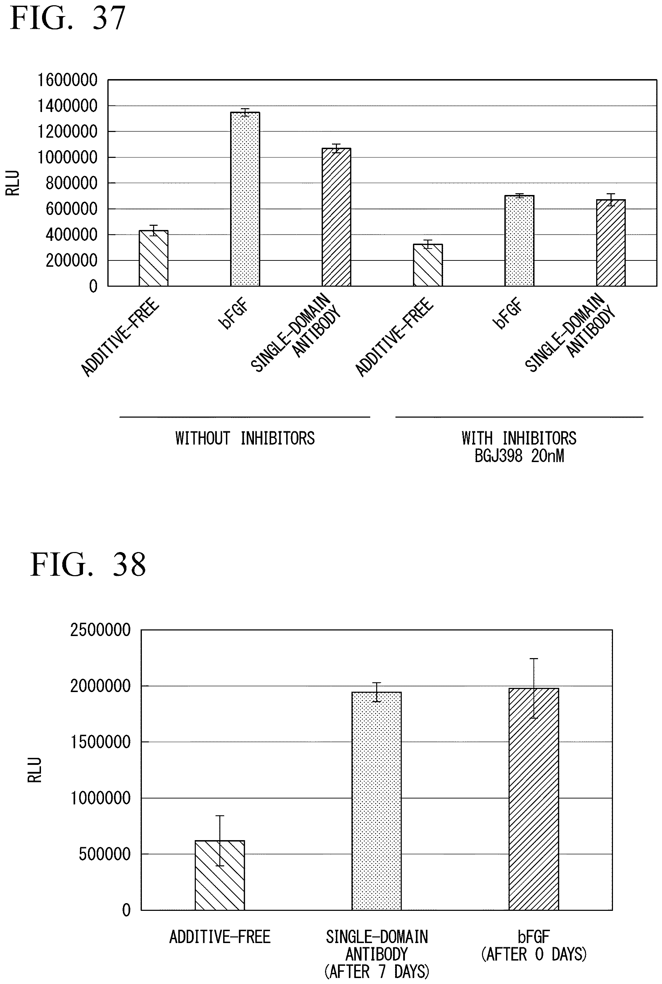

As shown in FIG. 37, the addition of NPV-BGJ398, which is an FGFR kinase inhibitor, suppressed cell growth due to bFGF and the single-domain antibody. From this, it was confirmed that the single-domain antibody has an FGFR-mediated cell growth activity as in the case of bFGF.

### 3. Comparison of Solution Preservation Stability between bFGF and VHH Homodimer

bFGF and the VHH homodimer (SEQ ID NO: 23, sequence name: #1-L4-#1, sometimes referred to as a single-domain antibody) was mixed with a D-PBS solution (Fujifilm Wako Pure Chemical Corporation) so that the concentration was 0.5 mg/mL, and incubation was performed at 37°C for 1 week. Then, the growth promoting abilities for fibroblast growth of four test subjects, which are an incubated VHH homodimer, a pre-incubated bFGF, a VHH homodimer after thawing from freeze-preservation, and bFGF immediately after solutionizing with D-PBS from a freeze-dried product, were evaluated. As a negative control, culture without any of the bFGF and the VHH homodimer was also performed at the same time. The culture conditions were the same as those in Item 1 and Item 2 of the present Example, and the cell growth was evaluated by the number of cells on the second day after the start of culture.

As shown in FIG. 38, the single-domain antibody (the column in the middle of FIG. 38) incubated at 37°C for 1 week exhibited the same cell growth activity as that of bFGF (the column at the right end of FIG. 38) immediately after solutionizing. In addition, the single-domain antibody after incubation exhibited a similar growth activity to that of the single-domain antibody after freeze-preservation and thawing (data not shown). On the other hand, in a case where bFGF was incubated at 37°C for one week, the culture promoting ability thereof was the same as that in the case of no addition (column at the left end of FIG. 38) (data not shown).

From the above, it has been revealed that the preservation stability of a single-domain antibody (VHH homodimer) in a solution is remarkably higher than that of bFGF, and can be more effectively used than bFGF in cell culture of fibroblasts, stem cells, and the like.

Since the receptor tyrosine kinase agonist of the present invention is excellent in stability, it can be used as a substitute for a human fibroblast growth factor 2 (FGF2) during cell culture of human iPS cells. Moreover, since only a small amount is required, the cost of iPS cells can be reduced.

### [Sequence Listing]

The base sequences and the amino acid sequences are shown below.

**[Table 15]**

| Name of sequence | Base sequence (5' → 3') | SEQ ID NO: |
|---|---|---|
| VHH-specific primer | CAT TTT ACA TTC TAC AAC TAC AAG CCA CCA TGG CTG AGT TGC AGC TCG TGG | 1 |
| | GCC GCC GTG ATG ATG ATG ATG ATG GCT ACC ACC TCC CGA TTG TGG TTT TGG TGT CTT GGG TTC TGA GGA G | 2 |
| | GCC GCC GTG ATG ATG ATG ATG ATG GCT ACC ACC TCC CGA TTG TGG TTT TGG TGT CTT GGG | 3 |
| Primer for overlap PCR | GAT CCC GCG AAA TTA ATA CGA CTC ACT ATA GGG GA AGTA TTT TTA CAA CAA TTA CCA ACA ACA ACA ACA AAC AAC AAC AAC ATT ACA TTT TAC ATT CTA CAA CTA CAA GCC ACC ATG | 4 |
| | TTT CCA CGC CGC CCC CCG TCC TGC TTC CGC CGT GAT GAT GAT GAT GAT GGC TGC CTC CCC C | 5 |

**[Table 16]**

| Name of sequence | Base sequence (5' → 3') | SEQ ID NO: |
|---|---|---|
| ^{cnv}K NewYtag for poly A | TTT CCA CGC CGC CCC CCG TCC T | 6 |
| T7 omeganew | GAT CCC GCG AAA TTA ATA CGA CTC ACT ATA GGG GAA GTΛ TTT TTΛ CΛΛ CΛΛ TTΛ CCΛ ΛCΛ | 7 |

**[Table 17]**

| Name of sequence | Base sequence (5' → 3') | SEQ ID NO: |
|---|---|---|
| NGS Fw 1st PCR primer | TCG TCG GCA GCG TCA GAT GTG TAT AAG AGA CAG NNN NAT GGC TGA GGT GCA GCT CGT G | 8 |
| NGS Rv 1st PCR primer | GTC TCG TGG GCT CGG AGA TGT GTA TAA GAG ACA GNN NNT GAT GAT GAT GGC TAC CAC CTC CCG | 9 |

**[Table 18]**

| Name of sequence | Base sequence (5' → 3') | SEQ ID NO: |
|---|---|---|
| Alp-to-pPK4-Nco-Sfi-VHH-F | CCG GCC ATG GCC ACT GCG GCC SAG KTG CAG CTC GTG GAG TC | 10 |
| Alp-GGGS-HisTag-to-pPK4-R | GCC TGC GGC CGC GTG ATG ATG ATG ATG ATG GGA TCC TCC CCC CG | 11 |

**[Table 19]**

| Name of sequence | Amino acid sequence (N-terminal → C-terminal) | SEQ ID NO: |
|---|---|---|
| 5aa | GSGGG | 12 |
| 10aa | GSGGGGSGGG | 13 |
| 20aa | GSGGGGSGGGGSGGGGSGGG | 14 |
| L3 | GSAGSAAGSGEF | 15 |
| L4 | GSEGKSSGSGSESKST | 16 |

The amino acid sequences of VHH monomer Clone #1 (VHH Clone #1) and VHH monomer Clone #2 (VHH Clone #2) are shown in Table 20. In addition, the amino acid sequences of the tandem dimers are shown in Table 10+X and Table 11+X.

**[Table 20]**

| Name of sequence | Amino acid sequence (N-terminal → C-terminal) | SEQ ID NO: |
|---|---|---|
| VHH Clone #1 | QLQLVESGGGSVQAGGSLRLSCAASRSLSRFNVWTWYRQAPVKQRELV ASITKSGDTNYAESVKGRFIISRDDDKNTVYLQMNNLKSEDTGIYYCAGS YYSGSYLYPSPGGFSVWGQGTQVTVSS | 17 |
| VHH Clone #2 | QVQLVESGGGLVQAGGSLRLSCAVPGSIFRFNTMGWYRQPPGKQRELVA QMSRGGITKYGDSVKGRFTITRDNAKNTVHLQMNSLKPEDTAVYYCAGS YYSGSYYYPSPQGFSVWGQGTQVTVSP | 18 |

**[Table 21]**

| Name of sequence | Amino acid sequence (N-terminal → C-terminal) | SEQ ID NO: |
|---|---|---|
| #1-5aa-#1 | QLQLVESGGGSVQAGGSLRLSCAASRSLSRFNVWTWYRQAPVK QRELVASITKSGDTNYAESVKGRFIISRDDDKNTVYLQMNNLKSE DTGIYYCAGSYYSGSYLYPSPGGFSVWGQGTQVTVSSGSGGGQL QLVESGGGSVQAGGSLRLSCAASRSLSRFNVWTWYRQAPVKQR ELVASITKSGDTNYAESVKGRFIISRDDDKNTVYLQMNNLKSEDT GIYYCAGSYYSGSYLYPSPGGFSVWGQGTQVTVSS | 19 |
| #1-10aa-#1 | QLQLVESGGGSVQAGGSLRLSCAASRSLSRFNVWTWYRQAPVK QRELVASITKSGDTNYAESVKGRFIISRDDDKNTVYLQMNNLKSE DTGIYYCAGSYYSGSYLYPSPGGFSVWGQGTQVTVSSGSGGGGS GGGQLQLVESGGGSVQAGGSLRLSCAASRSLSRFNVWTWYRQA PVKQRELVASITKSGDTNYAESVKGRFIISRDDDKNTVYLQMNNL KSEDTGIYYCAGSYYSGSYLYPSPGGFSVWGQGTQVTVSS | 20 |
| #1-20aa-#1 | QLQLVESGGGSVQAGGSLRLSCAASRSLSRFNVWTWYRQAPVK QRELVASITKSGDTNYAESVKGRFIISRDDDKNTVYLQMNNLKSE DTGIYYCAGSYYSGSYLYPSPGGFSVWGQGTQVTVSSGSGGGGS GGGGSGGGGSGGGQLQLVESGGGSVQAGGSLRLSCAASRSLSRF NVWTWYRQAPVKQRELVASITKSGDTNYAESVKGRFIISRDDDK NTVYLQMNNLKSEDTGIYYCAGSYYSGSYLYPSPGGFSVWGQGT QVTVSS | 21 |
| #1-L3-#1 | QLQLVESGGGSVQAGGSLRLSCAASRSLSRFNVWTWYRQAPVK QRELVASITKSGDTNYAESVKGRFIISRDDDKNTVYLQMNNLKSE DTGIYYCAGSYYSGSYLYPSPGGFSVWGQGTQVTVSSGSAGSAA GSGEFQLQLVESGGGSVQAGGSLRLSCAASRSLSRFNVWTWYRQ APVKQRELVASITKSGDTNYAESVKGRFIISRDDDKNTVYLQMNN LKSEDTGIYYCAGSYYSGSYLYPSPGGFSVWGQGTQVTVSS | 22 |
| #1-L4-#1 | QLQLVESGGGSVQAGGSLRLSCAASRSLSRFNVWTWYRQAPVK QRELVASITKSGDTNYAESVKGRFIISRDDDKNTVYLQMNNLKSE DTGIYYCAGSYYSGSYLYPSPGGFSVWGQGTQVTVSSGSEGKSSG SGSESKSTQLQLVESGGGSVQAGGSLRLSCAASRSLSRFNVWTW YRQAPVKQRELVASITKSGDTNYAESVKGRFIISRDDDKNTVYLQ MNNLKSEDTGIYYCAGSYYSGSYLYPSPGGFSVWGQGTQVTVSS | 23 |

**[Table 22]**

| Name of sequence | Amino acid sequence (N-terminal → C-terminal) | SEQ ID NO: |
|---|---|---|
| #2-5aa-#2 | QVQLVESGGGLVQAGGSLRLSCAVPGSIFRFNTMGWYRQPPGKQR ELVAQMSRGGITKYGDSVKGRFTITRDNAKNTVHLQMNSLKPEDT AVYYCAGSYYSGSYYYPSPQGFSVWGQGTQVTVSPGSGGGQVQL VESGGGLVQAGGSLRLSCAVPGSIFRFNTMGWYRQPPGKQRELVA QMSRGGITKYGDSVKGRFTITRDNAKNTVHLQMNSLKPEDTAVY YCAGSYYSGSYYYPSPQGFSVWGQGTQVTVSP | 24 |
| #2-10aa-#2 | QVQLVESGGGLVQAGGSLRLSCAVPGSIFRFNTMGWYRQPPGKQR ELVAQMSRGGITKYGDSVKGRFTITRDNAKNTVHLQMNSLKPEDT AVYYCAGSYYSGSYYYPSPQGFSVWGQGTQVTVSPGSGGGGSGG GQVQLVESGGGLVQAGGSLRLSCAVPGSIFRFNTMGWYRQPPGK QRELVAQMSRGGITKYGDSVKGRFTITRDNAKNTVHLQMNSLKPE DTAVYYCAGSYYSGSYYYPSPQGFSVWGQGTQVTVSP | 25 |
| #2-20aa-#2 | QVQLVESGGGLVQAGGSLRLSCAVPGSIFRFNTMGWYRQPPGKQR ELVAQMSRGGITKYGDSVKGRFTITRDNAKNTVHLQMNSLKPEDT AVYYCAGSYYSGSYYYPSPQGFSVWGQGTQVTVSPGSGGGGSGG GGSGGGGSGGGQVQLVESGGGLVQAGGSLRLSCAVPGSIFRFNTM GWYRQPPGKQRELVAQMSRGGITKYGDSVKGRFTITRDNAKNTV HLQMNSLKPEDTAVYYCAGSYYSGSYYYPSPQGFSVWGQGTQVT VSP | 26 |
| #2-L3-#2 | QVQLVESGGGLVQAGGSLRLSCAVPGSIFRFNTMGWYRQPPGKQR ELVAQMSRGGITKYGDSVKGRFTITRDNAKNTVHLQMNSLKPEDT AVYYCAGSYYSGSYYYPSPQGFSVWGQGTQVTVSPGSAGSAAGS GEFQVQLVESGGGLVQAGGSLRLSCAVPGSIFRFNTMGWYRQPPG KQRELVAQMSRGGITKYGDSVKGRFTITRDNAKNTVHLQMNSLK PEDTAVYYCAGSYYSGSYYYPSPQGFSVWGQGTQVTVSP | 27 |
| #2-L4-#2 | QVQLVESGGGLVQAGGSLRLSCAVPGSIFRFNTMGWYRQPPGKQR ELVAQMSRGGITKYGDSVKGRFTITRDNAKNTVHLQMNSLKPEDT AVYYCAGSYYSGSYYYPSPQGFSVWGQGTQVTVSPGSEGKSSGS GSESKSTQVQLVESGGGLVQAGGSLRLSCAVPGSIFRFNTMGWYR QPPGKQRELVAQMSRGGITKYGDSVKGRFTITRDNAKNTVHLQM NSLKPEDTAVYYCAGSYYSGSYYYPSPQGFSVWGQGTQVTVSP | 28 |

The amino acid sequences of the CDR3 region (#1-CDR) of VHH Clone #1 and the CDR3 region (#2-CDR) of VHH Clone #2 are shown in the following table.

**[Table 23]**

| Name of sequence | Amino acid sequence (N-terminal → C-terminal) | SEQ ID NO: |
|---|---|---|
| #1-CDR | SYYSGSYLYPSPGGFSV | 29 |
| #2-CDR | SYYSGSYYYPSPQGFSV | 30 |

The framework sequences 1 to 4 of humanized VHH, human VH reference, universal VHH, CAMDR, LAMGL, and VICPA are shown in the table below. The amino acid numbers are assigned according to "Kabat numbering" (Dondelinger, Mathieu and 6 others, Understanding the Significance and Implications of Antibody Numbering and Antigen-Binding Surface/Residue Definition, Frontiers in Immunology, 2018, Vol. 9, p. 2278).

**[Table 24]**

| Name of sequence | Amino acid sequence (N-terminal → C-terminal) | SEQ ID NO: |
|---|---|---|
| humanized VHH (1) FR1 | QVQLVESGGGLVQPGGSLRLSCAAS | 31 |
| humanized VHH (35b) FR2 | LGWFRQAPGQGLEAVAA | 32 |
| humanized VHH (58) FR3 | YYADSVKGRFTISRDNSKNTLYLQMNSLRAEDTAVYYCA | 33 |
| humanized VHH (103) FR4 | WGQGTLVTVSS | 34 |
| human VH reference (1) FR1 | QVQLVQSGGGLVQPGGSLRLSCAAS | 35 |
| human VH reference (36) FR2 | WVRQAPGKGLEWVSP | 36 |
| human VH reference (58) FR3 | YYADSVKGRFTISRDNSKNTLYLQMNTLRAEDTAVYYCA | 37 |
| human VH reference (103) FR4 | WGQGTMVTVSS | 38 |
| universal VHH (1) FR 1 | QVQLVESGGGSVQPGGSLRLSCTAS | 39 |
| universal VHH (35b) FR2 | LGWFRQAPGQEREAVAA | 40 |
| universal VHH (58) FR3 | YYADSVKGRFTISRDNAKNTVTLQMNNLKPEDTAIYYCA | 41 |
| universal VHH (103) FR4 | WGQGTQVTVSS | 42 |

**[Table 25]**

| Name of sequence | Amino acid sequence (N-terminal → C-terminal) | SEQ ID NO: |
|---|---|---|
| CAMDR (1) FR1 | EVQLVESGGGSVQAGGSLRLSCAAS | 43 |
| CAMDR (36) FR2 | WYRQAPGKEREFVS | 44 |
| CAMDR (58) FR3 | YADSVKGRFTISQDNAKNTVYLQMNSLIPEDTAMYYCKT | 45 |
| CAMDR (103) FR4 | WGQGTMVTVSS | 46 |
| LAMGL (1)FR1 | QVQLQESGGGLVQPGGSLRLSCTAS | 47 |
| LAMGL (36) FR2 | WYRQAPGSKREFVA | 48 |
| LAMGL (58) FR3 | FADSVKGRFTISRDNAKTTVDLQMNSLKPEDTAVYYCAA | 49 |
| LAMGL (103) FR4 | WGKGTQVTVSS | 50 |
| VICPA (1) FR1 | QVQLVETGGGLVQTGGSLRLSCKAS | 51 |
| VICPA (36) FR2 | WFRQAPGKERDFVA | 52 |
| VICPA (58) FR3 | TYADSVKGRFTISRDNASNMVYLLMNNLKPEDTAVYYCA A | 53 |
| VICPA (103) FR4 | WGQGTQVTVSS | 54 |

**[Table 26]**

| Name of sequence | Base sequence (5' → 3') | SEQ ID NO: |
|---|---|---|
| Main chain of ^{cnv}K rG Linker Biotin fragment | AAgAATTTCCAKGCCGCCCCCCGVCCT | 55 |

| | | |
|---|---|---|
| g, guanosine; V, Amino C6-dT; K, 3-cyanovinylcarbazole. | | |

**[Table 27]**

| Name of sequence | Base sequence (5' → 3') | SEQ ID NO: |
|---|---|---|
| pFc-VHH#1-F | TTGCACTTGTCACGAATTCGCAGTTGCAGCTCGTGGAGTCT G | 56 |
| pFc-VHH#1-R | TGTCAGATCTAACCATGGCCGATGAGGAGACGGTGACCTG GGTC | 57 |

**[Table 28]**

| Name of sequence | Amino acid sequence (N-terminal → C-terminal) | SEQ ID NO: |
|---|---|---|
| bFGF | | 58 |

### [Industrial Applicability]

The receptor tyrosine kinase agonist of the present invention is useful in the technical field of cell culture and the technical field of stem cell culture.

## Claims

1. A receptor tyrosine kinase agonist comprising a single-domain antibody having a cell growth activity,
wherein the receptor tyrosine kinase agonist is
at least one receptor tyrosine kinase agonist selected from the group consisting of a human fibroblast growth factor receptor 1 (FGFR1), a human fibroblast growth factor receptor 2 (FGFR2), a human fibroblast growth factor receptor 3 (FGFR3), and a human fibroblast growth factor receptor 4 (FGFR4).

2. The receptor tyrosine kinase agonist according to Claim 1,
wherein an EC₅₀ value of the cell growth activity is 10 µg/mL or less.

3. The receptor tyrosine kinase agonist according to Claim 1 or 2,
wherein the receptor tyrosine kinase agonist is a dimer in which the single-domain antibodies having the same sequence are linked by a linker.

4. The receptor tyrosine kinase agonist according to Claim 3,
wherein the linker is an oligopeptide linker or a chemical linker.

5. The receptor tyrosine kinase agonist according to Claim 1,
wherein a denaturation temperature (Td) is 65°C or higher.

6. The receptor tyrosine kinase agonist according to Claim 1,
wherein a value of an equilibrium dissociation constant (KD) is 5 × 10⁻⁹ M or less.

7. The receptor tyrosine kinase agonist according to Claim 1,
wherein an amino acid sequence of a complementarity determining region (CDR) of the single-domain antibody includes an amino acid sequence represented by SEQ ID NO: 29 or an amino acid sequence represented by SEQ ID NO: 30.

8. The receptor tyrosine kinase agonist according to Claim 1 or 2,
wherein the single-domain antibody includes an amino acid having characteristics of any one of the following (1) to (5) at a position defined (specified) according to Kabat numbering:
(1) a 37^{th} amino acid is any amino acid selected from the group consisting of tyrosine (Y), phenylalanine (F), and valine (V),
(2) a 41^{st} amino acid is proline (P),
(3) a 44^{th} amino acid is any amino acid selected from the group consisting of glutamine (Q), glycine (G), glutamic acid (E), and lysine (K),
(4) a 45^{th} amino acid is arginine (R) or leucine (L), and
(5) a 47^{th} amino acid is any amino acid selected from the group consisting of leucine (L), alanine (A), tryptophan (W), glycine (G), and phenylalanine (F).

9. A medium composition for cell culture, containing 0.1 ng/mL to 10 µg/mL of a receptor tyrosine kinase agonist,
wherein the receptor tyrosine kinase agonist includes a single-domain antibody having a cell growth activity, and
is at least one selected from the group consisting of a human fibroblast growth factor receptor 1 (FGFR1), a human fibroblast growth factor receptor 2 (FGFR2), a human fibroblast growth factor receptor 3 (FGFR3), and a human fibroblast growth factor receptor 4 (FGFR4).

10. The medium composition for cell culture according to Claim 9,
wherein a cell grown by the cell growth activity is at least one cell selected from the group consisting of a fibroblast, a mesenchymal stem cell, and an iPS cell.

11. A composition for maintaining an undifferentiated state of a stem cell, the composition comprising the receptor tyrosine kinase agonist according to Claim 1 as an active ingredient.

12. The composition for maintaining an undifferentiated state according to Claim 11,
wherein the stem cell is a mesenchymal stem cell or an iPS cell.
